(19) Europäisches Patentamt
European Patent Office
Office européen des brevets

(11) **EP 3 680 343 A2**

(12) **EUROPEAN PATENT APPLICATION**
published in accordance with Art. 153(4) EPC

(43) Date of publication:
**15.07.2020 Bulletin 2020/29**

(21) Application number: **18853447.3**

(22) Date of filing: **07.09.2018**

(51) Int Cl.:
***C12P 21/00*** *(2006.01)* ***C12N 9/24*** *(2006.01)*
***C07K 14/415*** *(2006.01)*

(86) International application number:
**PCT/KR2018/010466**

(87) International publication number:
**WO 2019/050318 (14.03.2019 Gazette 2019/11)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **07.09.2017 KR 20170114813**
**19.03.2018 KR 20180031579**

(71) Applicant: **Korea Advanced Institute of Science and Technology**
**Daejeon 34141 (KR)**

(72) Inventors:
- **AHN, Jung Hoon**
  **Busan 47163 (KR)**
- **BYUN, Hyunjong**
  **Daegu 41425 (KR)**
- **PARK, Jiyeon**
  **Busan 47391 (KR)**

(74) Representative: **Agasse, Stéphane et al**
**Cabinet GERMAIN & MAUREAU**
**B.P. 6153**
**69466 Lyon Cedex 06 (FR)**

(54) **METHOD FOR INCREASING SECRETION OF RECOMBINANT PROTEIN**

(57) The present invention provides a method for effective extracellular secretion of a target protein, by preparing a fusion protein connecting LARD3 to the target protein and having pI lowered by adjusting the overall charge of target protein, and by using ABC transporter of a bacterial type 1 secretion system (T1SS). The method can allows a protein be produced at a large amount simply and effectively without a separate purification process.

【Fig. 9】

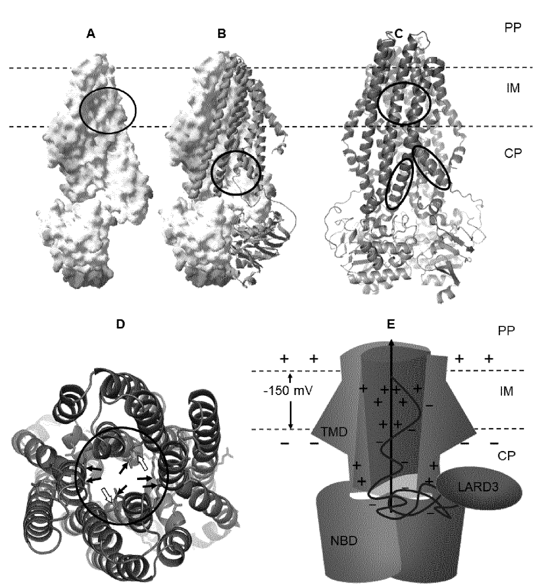


EP 3 680 343 A2

<110> KOREA ADVANCED INSTITUTE OF SCIENCE AND TECHNOLOGY

<120> Method of increasing signal sequence-mediated secretion of recombinant proteins

<130> OPP20183085KR

<150> KR 10/2018/0031579
<151> 2018-03-19

<150> KR 10/2017/0114813
<151> 2017-09-07

<160> 35

<170> KoPatentIn 3.0

<210> 1
<211> 476
<212> PRT
<213> Artificial Sequence

<220>
<223> Amino acid sequence of TliA wild type

<400> 1

```
Met Gly Val Phe Asp Tyr Lys Asn Leu Gly Thr Glu Ala Ser Lys Thr
  1               5                  10                  15

Leu Phe Ala Asp Ala Thr Ala Ile Thr Leu Tyr Thr Tyr His Asn Leu
             20                  25                  30

Asp Asn Gly Phe Ala Val Gly Tyr Gln Gln His Gly Leu Gly Leu Gly
         35                  40                  45

Leu Pro Ala Thr Leu Val Gly Ala Leu Leu Gly Ser Thr Asp Ser Gln
     50                  55                  60

Gly Val Ile Pro Gly Ile Pro Trp Asn Pro Asp Ser Glu Lys Ala Ala
 65                  70                  75                  80

Leu Asp Ala Val His Ala Ala Gly Trp Thr Pro Ile Ser Ala Ser Ala
                 85                  90                  95

Leu Gly Tyr Gly Gly Lys Val Asp Ala Arg Gly Thr Phe Phe Gly Glu
            100                 105                 110
```

Lys Ala Gly Tyr Thr Thr Ala Gln Ala Glu Val Leu Gly Lys Tyr Asp
115 120 125

Asp Ala Gly Lys Leu Leu Glu Ile Gly Ile Gly Phe Arg Gly Thr Ser
130 135 140

Gly Pro Arg Glu Ser Leu Ile Thr Asp Ser Ile Gly Asp Leu Val Ser
145 150 155 160

Asp Leu Leu Ala Ala Leu Gly Pro Lys Asp Tyr Ala Lys Asn Tyr Ala
165 170 175

Gly Glu Ala Phe Gly Gly Leu Leu Lys Thr Val Ala Asp Tyr Ala Gly
180 185 190

Ala His Gly Leu Ser Gly Lys Asp Val Leu Val Ser Gly His Ser Leu
195 200 205

Gly Gly Leu Ala Val Asn Ser Met Ala Asp Leu Ser Thr Ser Lys Trp
210 215 220

Ala Gly Phe Tyr Lys Asp Ala Asn Tyr Leu Ala Tyr Ala Ser Pro Thr
225 230 235 240

Gln Ser Ala Gly Asp Lys Val Leu Asn Ile Gly Tyr Glu Asn Asp Pro
245 250 255

Val Phe Arg Ala Leu Asp Gly Ser Thr Phe Asn Leu Ser Ser Leu Gly
260 265 270

Val His Asp Lys Ala His Glu Ser Thr Thr Asp Asn Ile Val Ser Phe
275 280 285

Asn Asp His Tyr Ala Ser Thr Leu Trp Asn Val Leu Pro Phe Ser Ile
290 295 300

Ala Asn Leu Ser Thr Trp Val Ser His Leu Pro Ser Ala Tyr Gly Asp
305 310 315 320

Gly Met Thr Arg Val Leu Glu Ser Gly Phe Tyr Glu Gln Met Thr Arg
325 330 335

Asp Ser Thr Ile Ile Val Ala Asn Leu Ser Asp Pro Ala Arg Ala Asn
340 345 350

Thr Trp Val Gln Asp Leu Asn Arg Asn Ala Glu Pro His Thr Gly Asn

355 360 365

Thr Phe Ile Ile Gly Ser Asp Gly Asn Asp Leu Ile Gln Gly Gly Lys
370 375 380

Gly Ala Asp Phe Ile Glu Gly Gly Lys Gly Asn Asp Thr Ile Arg Asp
385 390 395 400

Asn Ser Gly His Asn Thr Phe Leu Phe Ser Gly His Phe Gly Gln Asp
405 410 415

Arg Ile Ile Gly Tyr Gln Pro Thr Asp Arg Leu Val Phe Gln Gly Ala
420 425 430

Asp Gly Ser Thr Asp Leu Arg Asp His Ala Lys Ala Val Gly Ala Asp
435 440 445

Thr Val Leu Ser Phe Gly Ala Asp Ser Val Thr Leu Val Gly Val Gly
450 455 460

Leu Gly Gly Leu Trp Ser Glu Gly Val Leu Ile Ser
465 470 475

<210> 2
<211> 589
<212> PRT
<213> Artificial Sequence

<220>
<223> Amino acid sequence of TliA expressed in pDART plasmid

<400> 2
Met Ser Arg Met Gly Val Phe Asp Tyr Lys Asn Leu Gly Thr Glu Ala
1 5 10 15

Ser Lys Thr Leu Phe Ala Asp Ala Thr Ala Ile Thr Leu Tyr Thr Tyr
20 25 30

His Asn Leu Asp Asn Gly Phe Ala Val Gly Tyr Gln Gln His Gly Leu
35 40 45

Gly Leu Gly Leu Pro Ala Thr Leu Val Gly Ala Leu Leu Gly Ser Thr
50 55 60

Asp Ser Gln Gly Val Ile Pro Gly Ile Pro Trp Asn Pro Asp Ser Glu

65 70 75 80

Lys Ala Ala Leu Asp Ala Val His Ala Ala Gly Trp Thr Pro Ile Ser
85 90 95

Ala Ser Ala Leu Gly Tyr Gly Gly Lys Val Asp Ala Arg Gly Thr Phe
100 105 110

Phe Gly Glu Lys Ala Gly Tyr Thr Thr Ala Gln Ala Glu Val Leu Gly
115 120 125

Lys Tyr Asp Asp Ala Gly Lys Leu Leu Glu Ile Gly Ile Gly Phe Arg
130 135 140

Gly Thr Ser Gly Pro Arg Glu Ser Leu Ile Thr Asp Ser Ile Gly Asp
145 150 155 160

Leu Val Ser Asp Leu Leu Ala Ala Leu Gly Pro Lys Asp Tyr Ala Lys
165 170 175

Asn Tyr Ala Gly Glu Ala Phe Gly Gly Leu Leu Lys Thr Val Ala Asp
180 185 190

Tyr Ala Gly Ala His Gly Leu Ser Gly Lys Asp Val Leu Val Ser Gly
195 200 205

His Ser Leu Gly Gly Leu Ala Val Asn Ser Met Ala Asp Leu Ser Thr
210 215 220

Ser Lys Trp Ala Gly Phe Tyr Lys Asp Ala Asn Tyr Leu Ala Tyr Ala
225 230 235 240

Ser Pro Thr Gln Ser Ala Gly Asp Lys Val Leu Asn Ile Gly Tyr Glu
245 250 255

Asn Asp Pro Val Phe Arg Ala Leu Asp Gly Ser Thr Phe Asn Leu Ser
260 265 270

Ser Leu Gly Val His Asp Lys Ala His Glu Ser Thr Thr Asp Asn Ile
275 280 285

Val Ser Phe Asn Asp His Tyr Ala Ser Thr Leu Trp Asn Val Leu Pro
290 295 300

Phe Ser Ile Ala Asn Leu Ser Thr Trp Val Ser His Leu Pro Ser Ala
305 310 315 320

Tyr Gly Asp Gly Met Thr Arg Val Leu Glu Ser Gly Phe Tyr Glu Gln
325 330 335

Met Thr Arg Asp Ser Thr Ile Ile Val Ala Asn Leu Ser Asp Pro Ala
340 345 350

Arg Ala Asn Thr Trp Val Gln Asp Leu Asn Arg Asn Ala Glu Pro His
355 360 365

Thr Gly Asn Thr Phe Ile Ile Gly Ser Asp Gly Asn Asp Leu Ile Gln
370 375 380

Gly Gly Lys Gly Ala Asp Phe Ile Glu Gly Gly Lys Gly Asn Asp Thr
385 390 395 400

Ile Arg Asp Asn Ser Gly His Asn Thr Phe Leu Phe Ser Gly His Phe
405 410 415

Gly Gln Asp Arg Ile Ile Gly Tyr Gln Pro Thr Asp Arg Leu Val Phe
420 425 430

Gln Gly Ala Asp Gly Ser Thr Asp Leu Arg Asp His Ala Lys Ala Val
435 440 445

Gly Ala Asp Thr Val Leu Ser Phe Gly Ala Asp Ser Val Thr Leu Val
450 455 460

Gly Val Gly Leu Gly Gly Leu Trp Ser Glu Gly Val Leu Ile Ser Glu
465 470 475 480

Leu Ile Glu Gly Arg Gly Ser Asp Gly Asn Asp Leu Ile Gln Gly Gly
485 490 495

Lys Gly Ala Asp Phe Ile Glu Gly Gly Lys Gly Asn Asp Thr Ile Arg
500 505 510

Asp Asn Ser Gly His Asn Thr Phe Leu Phe Ser Gly His Phe Gly Gln
515 520 525

Asp Arg Ile Ile Gly Tyr Gln Pro Thr Asp Arg Leu Val Phe Gln Gly
530 535 540

Ala Asp Gly Ser Thr Asp Leu Arg Asp His Ala Lys Ala Val Gly Ala
545 550 555 560

Asp Thr Val Leu Ser Phe Gly Ala Asp Ser Val Thr Leu Val Gly Val
565 570 575

Gly Leu Gly Gly Leu Trp Ser Glu Gly Val Leu Ile Ser
580 585

<210> 3
<211> 614
<212> PRT
<213> Artificial Sequence

<220>
<223> Amino acid sequence of NKC-TliA

<400> 3
Met Ser Arg His Met Gly Thr Ala Pro Lys Ala Met Lys Leu Leu Lys
1 5 10 15

Lys Leu Leu Lys Leu Gln Lys Lys Gly Ile Gly Ser Met Gly Val Phe
20 25 30

Asp Tyr Lys Asn Leu Gly Thr Glu Ala Ser Lys Thr Leu Phe Ala Asp
35 40 45

Ala Thr Ala Ile Thr Leu Tyr Thr Tyr His Asn Leu Asp Asn Gly Phe
50 55 60

Ala Val Gly Tyr Gln Gln His Gly Leu Gly Leu Gly Leu Pro Ala Thr
65 70 75 80

Leu Val Gly Ala Leu Leu Gly Ser Thr Asp Ser Gln Gly Val Ile Pro
85 90 95

Gly Ile Pro Trp Asn Pro Asp Ser Glu Lys Ala Ala Leu Asp Ala Val
100 105 110

His Ala Ala Gly Trp Thr Pro Ile Ser Ala Ser Ala Leu Gly Tyr Gly
115 120 125

Gly Lys Val Asp Ala Arg Gly Thr Phe Phe Gly Glu Lys Ala Gly Tyr
130 135 140

Thr Thr Ala Gln Ala Glu Val Leu Gly Lys Tyr Asp Asp Ala Gly Lys
145 150 155 160

Leu Leu Glu Ile Gly Ile Gly Phe Arg Gly Thr Ser Gly Pro Arg Glu
165 170 175

Ser Leu Ile Thr Asp Ser Ile Gly Asp Leu Val Ser Asp Leu Leu Ala
180 185 190

Ala Leu Gly Pro Lys Asp Tyr Ala Lys Asn Tyr Ala Gly Glu Ala Phe
195 200 205

Gly Gly Leu Leu Lys Thr Val Ala Asp Tyr Ala Gly Ala His Gly Leu
210 215 220

Ser Gly Lys Asp Val Leu Val Ser Gly His Ser Leu Gly Gly Leu Ala
225 230 235 240

Val Asn Ser Met Ala Asp Leu Ser Thr Ser Lys Trp Ala Gly Phe Tyr
245 250 255

Lys Asp Ala Asn Tyr Leu Ala Tyr Ala Ser Pro Thr Gln Ser Ala Gly
260 265 270

Asp Lys Val Leu Asn Ile Gly Tyr Glu Asn Asp Pro Val Phe Arg Ala
275 280 285

Leu Asp Gly Ser Thr Phe Asn Leu Ser Ser Leu Gly Val His Asp Lys
290 295 300

Ala His Glu Ser Thr Thr Asp Asn Ile Val Ser Phe Asn Asp His Tyr
305 310 315 320

Ala Ser Thr Leu Trp Asn Val Leu Pro Phe Ser Ile Ala Asn Leu Ser
325 330 335

Thr Trp Val Ser His Leu Pro Ser Ala Tyr Gly Asp Gly Met Thr Arg
340 345 350

Val Leu Glu Ser Gly Phe Tyr Glu Gln Met Thr Arg Asp Ser Thr Ile
355 360 365

Ile Val Ala Asn Leu Ser Asp Pro Ala Arg Ala Asn Thr Trp Val Gln
370 375 380

Asp Leu Asn Arg Asn Ala Glu Pro His Thr Gly Asn Thr Phe Ile Ile
385 390 395 400

Gly Ser Asp Gly Asn Asp Leu Ile Gln Gly Gly Lys Gly Ala Asp Phe
405 410 415

Ile Glu Gly Gly Lys Gly Asn Asp Thr Ile Arg Asp Asn Ser Gly His

420 425 430

Asn Thr Phe Leu Phe Ser Gly His Phe Gly Gln Asp Arg Ile Ile Gly
435 440 445

Tyr Gln Pro Thr Asp Arg Leu Val Phe Gln Gly Ala Asp Gly Ser Thr
450 455 460

Asp Leu Arg Asp His Ala Lys Ala Val Gly Ala Asp Thr Val Leu Ser
465 470 475 480

Phe Gly Ala Asp Ser Val Thr Leu Val Gly Val Gly Leu Gly Gly Leu
485 490 495

Trp Ser Glu Gly Val Leu Ile Ser Glu Leu Ile Glu Gly Arg Gly Ser
500 505 510

Asp Gly Asn Asp Leu Ile Gln Gly Gly Lys Gly Ala Asp Phe Ile Glu
515 520 525

Gly Gly Lys Gly Asn Asp Thr Ile Arg Asp Asn Ser Gly His Asn Thr
530 535 540

Phe Leu Phe Ser Gly His Phe Gly Gln Asp Arg Ile Ile Gly Tyr Gln
545 550 555 560

Pro Thr Asp Arg Leu Val Phe Gln Gly Ala Asp Gly Ser Thr Asp Leu
565 570 575

Arg Asp His Ala Lys Ala Val Gly Ala Asp Thr Val Leu Ser Phe Gly
580 585 590

Ala Asp Ser Val Thr Leu Val Gly Val Gly Leu Gly Gly Leu Trp Ser
595 600 605

Glu Gly Val Leu Ile Ser
610

<210> 4
<211> 644
<212> PRT
<213> Artificial Sequence

<220>
<223> Amino acid sequence of CTP-TliA

<400> 4

Met Ser Arg Met Arg Gly Ser His His His His His His Gly Met Ala
1 5 10 15

Ser Met Thr Gly Gly Gln Gln Met Gly Arg Asp Leu Tyr Asp Asp Asp
20 25 30

Asp Lys Asp Arg Trp Gly Ser Met Tyr Gly Arg Arg Ala Arg Arg Arg
35 40 45

Arg Arg Arg Ser Met Ala Gly Thr Gly Gly Met Gly Val Phe Asp Tyr
50 55 60

Lys Asn Leu Gly Thr Glu Ala Ser Lys Thr Leu Phe Ala Asp Ala Thr
65 70 75 80

Ala Ile Thr Leu Tyr Thr Tyr His Asn Leu Asp Asn Gly Phe Ala Val
85 90 95

Gly Tyr Gln Gln His Gly Leu Gly Leu Gly Leu Pro Ala Thr Leu Val
100 105 110

Gly Ala Leu Leu Gly Ser Thr Asp Ser Gln Gly Val Ile Pro Gly Ile
115 120 125

Pro Trp Asn Pro Asp Ser Glu Lys Ala Ala Leu Asp Ala Val His Ala
130 135 140

Ala Gly Trp Thr Pro Ile Ser Ala Ser Ala Leu Gly Tyr Gly Gly Lys
145 150 155 160

Val Asp Ala Arg Gly Thr Phe Phe Gly Glu Lys Ala Gly Tyr Thr Thr
165 170 175

Ala Gln Ala Glu Val Leu Gly Lys Tyr Asp Asp Ala Gly Lys Leu Leu
180 185 190

Glu Ile Gly Ile Gly Phe Arg Gly Thr Ser Gly Pro Arg Glu Ser Leu
195 200 205

Ile Thr Asp Ser Ile Gly Asp Leu Val Ser Asp Leu Leu Ala Ala Leu
210 215 220

Gly Pro Lys Asp Tyr Ala Lys Asn Tyr Ala Gly Glu Ala Phe Gly Gly
225 230 235 240

Leu Leu Lys Thr Val Ala Asp Tyr Ala Gly Ala His Gly Leu Ser Gly
245 250 255

Lys Asp Val Leu Val Ser Gly His Ser Leu Gly Gly Leu Ala Val Asn
260 265 270

Ser Met Ala Asp Leu Ser Thr Ser Lys Trp Ala Gly Phe Tyr Lys Asp
275 280 285

Ala Asn Tyr Leu Ala Tyr Ala Ser Pro Thr Gln Ser Ala Gly Asp Lys
290 295 300

Val Leu Asn Ile Gly Tyr Glu Asn Asp Pro Val Phe Arg Ala Leu Asp
305 310 315 320

Gly Ser Thr Phe Asn Leu Ser Ser Leu Gly Val His Asp Lys Ala His
325 330 335

Glu Ser Thr Thr Asp Asn Ile Val Ser Phe Asn Asp His Tyr Ala Ser
340 345 350

Thr Leu Trp Asn Val Leu Pro Phe Ser Ile Ala Asn Leu Ser Thr Trp
355 360 365

Val Ser His Leu Pro Ser Ala Tyr Gly Asp Gly Met Thr Arg Val Leu
370 375 380

Glu Ser Gly Phe Tyr Glu Gln Met Thr Arg Asp Ser Thr Ile Ile Val
385 390 395 400

Ala Asn Leu Ser Asp Pro Ala Arg Ala Asn Thr Trp Val Gln Asp Leu
405 410 415

Asn Arg Asn Ala Glu Pro His Thr Gly Asn Thr Phe Ile Ile Gly Ser
420 425 430

Asp Gly Asn Asp Leu Ile Gln Gly Gly Lys Gly Ala Asp Phe Ile Glu
435 440 445

Gly Gly Lys Gly Asn Asp Thr Ile Arg Asp Asn Ser Gly His Asn Thr
450 455 460

Phe Leu Phe Ser Gly His Phe Gly Gln Asp Arg Ile Ile Gly Tyr Gln
465 470 475 480

Pro Thr Asp Arg Leu Val Phe Gln Gly Ala Asp Gly Ser Thr Asp Leu
485 490 495

Arg Asp His Ala Lys Ala Val Gly Ala Asp Thr Val Leu Ser Phe Gly
500 505 510

Ala Asp Ser Val Thr Leu Val Gly Val Gly Leu Gly Gly Leu Trp Ser
515 520 525

Glu Gly Val Leu Ile Ser Glu Leu Ile Glu Gly Arg Gly Ser Asp Gly
530 535 540

Asn Asp Leu Ile Gln Gly Gly Lys Gly Ala Asp Phe Ile Glu Gly Gly
545 550 555 560

Lys Gly Asn Asp Thr Ile Arg Asp Asn Ser Gly His Asn Thr Phe Leu
565 570 575

Phe Ser Gly His Phe Gly Gln Asp Arg Ile Ile Gly Tyr Gln Pro Thr
580 585 590

Asp Arg Leu Val Phe Gln Gly Ala Asp Gly Ser Thr Asp Leu Arg Asp
595 600 605

His Ala Lys Ala Val Gly Ala Asp Thr Val Leu Ser Phe Gly Ala Asp
610 615 620

Ser Val Thr Leu Val Gly Val Gly Leu Gly Gly Leu Trp Ser Glu Gly
625 630 635 640

Val Leu Ile Ser

<210> 5
<211> 454
<212> PRT
<213> Artificial Sequence

<220>
<223> Amino acid sequence of Mannanase

<400> 5
Met Ser Arg His His His His His His Thr Val Ser Pro Val Asn Pro
1 5 10 15

Asn Ala Gln Gln Thr Thr Lys Ala Val Met Asn Trp Leu Ala His Leu
20 25 30

Pro Asn Arg Thr Glu Asn Arg Val Leu Ser Gly Ala Phe Gly Gly Tyr
35 40 45

Ser His Asp Thr Phe Ser Met Ala Glu Ala Asp Arg Ile Arg Ser Ala
50 55 60

Thr Gly Gln Ser Pro Ala Ile Tyr Gly Cys Asp Tyr Ala Arg Gly Trp
65 70 75 80

Leu Glu Thr Ala Asn Ile Glu Asp Ser Ile Asp Val Ser Cys Asn Ser
85 90 95

Asp Leu Met Ser Tyr Trp Lys Asn Asp Gly Ile Pro Gln Ile Ser Leu
100 105 110

His Leu Ala Asn Pro Ala Phe Gln Ser Gly His Phe Lys Thr Pro Ile
115 120 125

Thr Asn Asp Gln Tyr Lys Lys Ile Leu Asp Ser Ser Thr Ala Glu Gly
130 135 140

Lys Arg Leu Asn Thr Met Leu Ser Lys Ile Ala Asp Gly Leu Gln Glu
145 150 155 160

Leu Glu Asn Gln Gly Val Pro Val Leu Phe Arg Pro Leu His Glu Met
165 170 175

Asn Gly Glu Arg Phe Trp Trp Gly Leu Thr Ser Tyr Asn Gln Lys Asp
180 185 190

Asn Glu Arg Ile Ser Leu Tyr Lys Gln Leu Tyr Lys Lys Ile Tyr His
195 200 205

Tyr Met Thr Asp Thr Arg Gly Leu Asp His Leu Ile Trp Val Tyr Ser
210 215 220

Pro Asp Ala Asn Arg Asp Phe Lys Thr Asp Phe Tyr Pro Gly Ala Ser
225 230 235 240

Tyr Val Asp Ile Val Gly Leu Asp Ala Tyr Phe Gln Asp Ala Tyr Ser
245 250 255

Ile Asn Gly Tyr Asp Gln Leu Thr Ala Leu Asn Lys Pro Phe Ala Phe
260 265 270

Thr Glu Val Gly Pro Gln Thr Ala Asn Gly Ser Phe Asp Tyr Ser Leu

275 280 285

Phe Ile Asn Ala Ile Lys His Arg Tyr Pro Lys Thr Ile Tyr Phe Leu
290 295 300

Ala Trp Asn Asp Glu Trp Ser Pro Ala Val Asn Lys Gly Ala Ser Ala
305 310 315 320

Leu Tyr His Asp Ser Trp Thr Leu Asn Lys Gly Glu Ile Trp Asn Gly
325 330 335

Asp Ser Leu Thr Pro Ile Val Glu Glu Leu Ile Glu Gly Arg Gly Ser
340 345 350

Asp Gly Asn Asp Leu Ile Gln Gly Gly Lys Gly Ala Asp Phe Ile Glu
355 360 365

Gly Gly Lys Gly Asn Asp Thr Ile Arg Asp Asn Ser Gly His Asn Thr
370 375 380

Phe Leu Phe Ser Gly His Phe Gly Gln Asp Arg Ile Ile Gly Tyr Gln
385 390 395 400

Pro Thr Asp Arg Leu Val Phe Gln Gly Ala Asp Gly Ser Thr Asp Leu
405 410 415

Arg Asp His Ala Lys Ala Val Gly Ala Asp Thr Val Leu Ser Phe Gly
420 425 430

Ala Asp Ser Val Thr Leu Val Gly Val Gly Leu Gly Gly Leu Trp Ser
435 440 445

Glu Gly Val Leu Ile Ser
450

<210> 6
<211> 266
<212> PRT
<213> Artificial Sequence

<220>
<223> Amino acid sequence of Mussel adhesion protein

<400> 6
Met Ser Ser Met Arg Gly Ser His His His His His His Gly Ser Ala

1 5 10 15

Ser Ala Lys Pro Ser Tyr Pro Pro Thr Tyr Lys Ala Lys Pro Ser Tyr
20 25 30

Pro Pro Thr Tyr Lys Ala Lys Pro Ser Tyr Pro Pro Thr Tyr Lys Gly
35 40 45

Cys Ser Ser Glu Glu Tyr Lys Gly Gly Tyr Tyr Pro Gly Asn Ser Asn
50 55 60

His Tyr His Ser Gly Gly Ser Tyr His Gly Ser Gly Tyr His Gly Gly
65 70 75 80

Tyr Lys Gly Lys Tyr Tyr Gly Lys Ala Lys Lys Tyr Tyr Tyr Lys Tyr
85 90 95

Lys Asn Ser Gly Lys Tyr Lys Tyr Leu Lys Lys Ala Arg Lys Tyr His
100 105 110

Arg Lys Gly Tyr Lys Lys Tyr Tyr Gly Gly Ser Ser Glu Phe Ala Lys
115 120 125

Pro Ser Tyr Pro Pro Thr Tyr Lys Ala Lys Pro Ser Tyr Pro Pro Thr
130 135 140

Tyr Lys Ala Lys Pro Ser Tyr Pro Pro Thr Tyr Lys Glu Leu Ile Glu
145 150 155 160

Gly Arg Gly Ser Asp Gly Asn Asp Leu Ile Gln Gly Gly Lys Gly Ala
165 170 175

Asp Phe Ile Glu Gly Gly Lys Gly Asn Asp Thr Ile Arg Asp Asn Ser
180 185 190

Gly His Asn Thr Phe Leu Phe Ser Gly His Phe Gly Gln Asp Arg Ile
195 200 205

Ile Gly Tyr Gln Pro Thr Asp Arg Leu Val Phe Gln Gly Ala Asp Gly
210 215 220

Ser Thr Asp Leu Arg Asp His Ala Lys Ala Val Gly Ala Asp Thr Val
225 230 235 240

Leu Ser Phe Gly Ala Asp Ser Val Thr Leu Val Gly Val Gly Leu Gly
245 250 255

Gly Leu Trp Ser Glu Gly Val Leu Ile Ser
260 265

<210> 7
<211> 483
<212> PRT
<213> Artificial Sequence

<220>
<223> Amino acid sequence of Maltose binding protein

<400> 7
Met Ser Arg Lys Ile Glu Glu Gly Lys Leu Val Ile Trp Ile Asn Gly
1 5 10 15

Asp Lys Gly Tyr Asn Gly Leu Ala Glu Val Gly Lys Lys Phe Glu Lys
20 25 30

Asp Thr Gly Ile Lys Val Thr Val Glu His Pro Asp Lys Leu Glu Glu
35 40 45

Lys Phe Pro Gln Val Ala Ala Thr Gly Asp Gly Pro Asp Ile Ile Phe
50 55 60

Trp Ala His Asp Arg Phe Gly Gly Tyr Ala Gln Ser Gly Leu Leu Ala
65 70 75 80

Glu Ile Thr Pro Asp Lys Ala Phe Gln Asp Lys Leu Tyr Pro Phe Thr
85 90 95

Trp Asp Ala Val Arg Tyr Asn Gly Lys Leu Ile Ala Tyr Pro Ile Ala
100 105 110

Val Glu Ala Leu Ser Leu Ile Tyr Asn Lys Asp Leu Leu Pro Asn Pro
115 120 125

Pro Lys Thr Trp Glu Glu Ile Pro Ala Leu Asp Lys Glu Leu Lys Ala
130 135 140

Lys Gly Lys Ser Ala Leu Met Phe Asn Leu Gln Glu Pro Tyr Phe Thr
145 150 155 160

Trp Pro Leu Ile Ala Ala Asp Gly Gly Tyr Ala Phe Lys Tyr Glu Asn
165 170 175

Gly Lys Tyr Asp Ile Lys Asp Val Gly Val Asp Asn Ala Gly Ala Lys
180 185 190

Ala Gly Leu Thr Phe Leu Val Asp Leu Ile Lys Asn Lys His Met Asn
195 200 205

Ala Asp Thr Asp Tyr Ser Ile Ala Glu Ala Ala Phe Asn Lys Gly Glu
210 215 220

Thr Ala Met Thr Ile Asn Gly Pro Trp Ala Trp Ser Asn Ile Asp Thr
225 230 235 240

Ser Lys Val Asn Tyr Gly Val Thr Val Leu Pro Thr Phe Lys Gly Gln
245 250 255

Pro Ser Lys Pro Phe Val Gly Val Leu Ser Ala Gly Ile Asn Ala Ala
260 265 270

Ser Pro Asn Lys Glu Leu Ala Lys Glu Phe Leu Glu Asn Tyr Leu Leu
275 280 285

Thr Asp Glu Gly Leu Glu Ala Val Asn Lys Asp Lys Pro Leu Gly Ala
290 295 300

Val Ala Leu Lys Ser Tyr Glu Glu Glu Leu Ala Lys Asp Pro Arg Ile
305 310 315 320

Ala Ala Thr Met Glu Asn Ala Gln Lys Gly Glu Ile Met Pro Asn Ile
325 330 335

Pro Gln Met Ser Ala Phe Trp Tyr Ala Val Arg Thr Ala Val Ile Asn
340 345 350

Ala Ala Ser Gly Arg Gln Thr Val Asp Glu Ala Leu Lys Asp Ala Gln
355 360 365

Thr Arg Ile Thr Lys Glu Leu Ile Glu Gly Arg Gly Ser Asp Gly Asn
370 375 380

Asp Leu Ile Gln Gly Gly Lys Gly Ala Asp Phe Ile Glu Gly Gly Lys
385 390 395 400

Gly Asn Asp Thr Ile Arg Asp Asn Ser Gly His Asn Thr Phe Leu Phe
405 410 415

Ser Gly His Phe Gly Gln Asp Arg Ile Ile Gly Tyr Gln Pro Thr Asp
420 425 430

Arg Leu Val Phe Gln Gly Ala Asp Gly Ser Thr Asp Leu Arg Asp His
435 440 445

Ala Lys Ala Val Gly Ala Asp Thr Val Leu Ser Phe Gly Ala Asp Ser
450 455 460

Val Thr Leu Val Gly Val Gly Leu Gly Gly Leu Trp Ser Glu Gly Val
465 470 475 480

Leu Ile Ser

<210> 8
<211> 240
<212> PRT
<213> Artificial Sequence

<220>
<223> Amino acid sequence of Thioredoxin

<400> 8
Met Ser Arg Met Leu His Gln Gln Arg Asn Gln His Ala Arg Leu Ile
1 5 10 15

Pro Val Glu Leu Tyr Met Ser Asp Lys Ile Ile His Leu Thr Asp Asp
20 25 30

Ser Phe Asp Thr Asp Val Leu Lys Ala Asp Gly Ala Ile Leu Val Asp
35 40 45

Phe Trp Ala Glu Trp Cys Gly Pro Cys Lys Met Ile Ala Pro Ile Leu
50 55 60

Asp Glu Ile Ala Asp Glu Tyr Gln Gly Lys Leu Thr Val Ala Lys Leu
65 70 75 80

Asn Ile Asp Gln Asn Pro Gly Thr Ala Pro Lys Tyr Gly Ile Arg Gly
85 90 95

Ile Pro Thr Leu Leu Leu Phe Lys Asn Gly Glu Val Ala Ala Thr Lys
100 105 110

Val Gly Ala Leu Ser Lys Gly Gln Leu Lys Glu Phe Leu Asp Ala Asn
115 120 125

Leu Ala Glu Leu Ile Glu Gly Arg Gly Ser Asp Gly Asn Asp Leu Ile
130 135 140

Gln Gly Gly Lys Gly Ala Asp Phe Ile Glu Gly Gly Lys Gly Asn Asp
145 150 155 160

Thr Ile Arg Asp Asn Ser Gly His Asn Thr Phe Leu Phe Ser Gly His
165 170 175

Phe Gly Gln Asp Arg Ile Ile Gly Tyr Gln Pro Thr Asp Arg Leu Val
180 185 190

Phe Gln Gly Ala Asp Gly Ser Thr Asp Leu Arg Asp His Ala Lys Ala
195 200 205

Val Gly Ala Asp Thr Val Leu Ser Phe Gly Ala Asp Ser Val Thr Leu
210 215 220

Val Gly Val Gly Leu Gly Gly Leu Trp Ser Glu Gly Val Leu Ile Ser
225 230 235 240

<210> 9
<211> 335
<212> PRT
<213> Artificial Sequence

<220>
<223> Amino acid sequence of Cutinase

<400> 9
Met Ser Arg His His His His His His Ala Pro Thr Ser Asn Pro Ala
1 5 10 15

Gln Glu Leu Glu Ala Arg Gln Leu Gly Arg Thr Thr Arg Asp Asp Leu
20 25 30

Ile Asn Gly Asn Ser Ala Ser Cys Ala Asp Val Ile Phe Ile Tyr Ala
35 40 45

Arg Gly Ser Thr Glu Thr Gly Asn Leu Gly Thr Leu Gly Pro Ser Ile
50 55 60

Ala Ser Asn Leu Glu Ser Ala Phe Gly Lys Asp Gly Val Trp Ile Gln
65 70 75 80

Gly Val Gly Gly Ala Tyr Arg Ala Thr Leu Gly Asp Asn Ala Leu Pro
85 90 95

Arg Gly Thr Ser Ser Ala Ala Ile Arg Glu Met Leu Gly Leu Phe Gln
100 105 110

Gln Ala Asn Thr Lys Cys Pro Asp Ala Thr Leu Ile Ala Gly Gly Tyr
115 120 125

Ser Gln Gly Ala Ala Leu Ala Ala Ala Ser Ile Glu Asp Leu Asp Ser
130 135 140

Ala Ile Arg Asp Lys Ile Ala Gly Thr Val Leu Phe Gly Tyr Thr Lys
145 150 155 160

Asn Leu Gln Asn Arg Gly Arg Ile Pro Asn Tyr Pro Ala Asp Arg Thr
165 170 175

Lys Val Phe Cys Asn Thr Gly Asp Leu Val Cys Thr Gly Ser Leu Ile
180 185 190

Val Ala Ala Pro His Leu Ala Tyr Gly Pro Asp Ala Arg Gly Pro Ala
195 200 205

Pro Glu Phe Leu Ile Glu Lys Val Arg Ala Val Arg Gly Ser Ala Leu
210 215 220

Glu Glu Leu Ile Glu Gly Arg Gly Ser Asp Gly Asn Asp Leu Ile Gln
225 230 235 240

Gly Gly Lys Gly Ala Asp Phe Ile Glu Gly Gly Lys Gly Asn Asp Thr
245 250 255

Ile Arg Asp Asn Ser Gly His Asn Thr Phe Leu Phe Ser Gly His Phe
260 265 270

Gly Gln Asp Arg Ile Ile Gly Tyr Gln Pro Thr Asp Arg Leu Val Phe
275 280 285

Gln Gly Ala Asp Gly Ser Thr Asp Leu Arg Asp His Ala Lys Ala Val
290 295 300

Gly Ala Asp Thr Val Leu Ser Phe Gly Ala Asp Ser Val Thr Leu Val
305 310 315 320

Gly Val Gly Leu Gly Gly Leu Trp Ser Glu Gly Val Leu Ile Ser

325 330 335

<210> 10
<211> 762
<212> PRT
<213> Artificial Sequence

<220>
<223> Amino acid sequence of Chitinase

<400> 10
Met Ser Arg His His His His His His Ala Asn Ser Pro Lys Gln Ser
1 5 10 15

Gln Lys Ile Val Gly Tyr Phe Pro Ser Trp Gly Val Tyr Gly Arg Asn
20 25 30

Tyr Gln Val Ala Asp Ile Asp Ala Ser Lys Leu Thr His Leu Asn Tyr
35 40 45

Ala Phe Ala Asp Ile Cys Trp Asn Gly Lys His Gly Asn Pro Ser Thr
50 55 60

His Pro Asp Asn Pro Asn Lys Gln Thr Trp Asn Cys Lys Glu Ser Gly
65 70 75 80

Val Pro Leu Gln Asn Lys Glu Val Pro Asn Gly Thr Leu Val Leu Gly
85 90 95

Glu Pro Trp Ala Asp Val Thr Lys Ser Tyr Pro Gly Ser Gly Thr Thr
100 105 110

Trp Glu Asp Cys Asp Lys Tyr Ala Arg Cys Gly Asn Phe Gly Glu Leu
115 120 125

Lys Arg Leu Lys Ala Lys Tyr Pro His Leu Lys Thr Ile Ile Ser Val
130 135 140

Gly Gly Trp Thr Trp Ser Asn Arg Phe Ser Asp Met Ala Ala Asp Glu
145 150 155 160

Lys Thr Arg Lys Val Phe Ala Glu Ser Thr Val Ala Phe Leu Arg Ala
165 170 175

Tyr Gly Phe Asp Gly Val Asp Leu Asp Trp Glu Tyr Pro Gly Val Glu

180 185 190

Thr Ile Pro Gly Gly Ser Tyr Arg Pro Glu Asp Lys Gln Asn Phe Thr
195 200 205

Leu Leu Leu Gln Asp Val Arg Asn Ala Leu Asn Lys Ala Gly Ala Glu
210 215 220

Asp Gly Lys Gln Tyr Leu Leu Thr Ile Ala Ser Gly Ala Ser Arg Arg
225 230 235 240

Tyr Ala Asp His Thr Glu Leu Lys Lys Ile Ser Gln Ile Leu Asp Trp
245 250 255

Ile Asn Ile Met Thr Tyr Asp Phe His Gly Gly Trp Glu Ala Thr Ser
260 265 270

Asn His Asn Ala Ala Leu Tyr Lys Asp Pro Asn Asp Pro Ala Ala Asn
275 280 285

Thr Asn Phe Tyr Val Asp Gly Ala Ile Asn Val Tyr Thr Asn Glu Gly
290 295 300

Val Pro Val Asp Lys Leu Val Leu Gly Val Pro Phe Tyr Gly Arg Gly
305 310 315 320

Trp Lys Ser Cys Gly Lys Glu Asn Asn Gly Gln Tyr Gln Pro Cys Lys
325 330 335

Pro Gly Ser Asp Gly Lys Leu Ala Ser Lys Gly Thr Trp Asp Asp Tyr
340 345 350

Ser Thr Gly Asp Thr Gly Val Tyr Asp Tyr Gly Asp Leu Ala Ala Asn
355 360 365

Tyr Val Asn Lys Asn Gly Phe Val Arg Tyr Trp Asn Asp Thr Ala Lys
370 375 380

Val Pro Tyr Leu Tyr Asn Ala Thr Thr Gly Thr Phe Ile Ser Tyr Asp
385 390 395 400

Asp Asn Glu Ser Met Lys Tyr Lys Thr Asp Ser Ile Lys Thr Lys Gly
405 410 415

Leu Ser Gly Ala Met Phe Trp Glu Leu Ser Gly Asp Cys Arg Thr Ser
420 425 430

Pro Lys Tyr Ser Cys Ser Gly Pro Lys Leu Leu Asp Thr Leu Val Lys
435 440 445

Glu Leu Leu Gly Gly Pro Ile Asn Gln Lys Asp Thr Glu Pro Pro Thr
450 455 460

Asn Val Lys Asn Ile Val Val Thr Asn Lys Asn Ser Asn Ser Val Gln
465 470 475 480

Leu Asn Trp Thr Ala Ser Thr Asp Asn Val Gly Val Thr Glu Tyr Glu
485 490 495

Ile Thr Ala Gly Glu Glu Lys Trp Ser Thr Thr Thr Asn Ser Ile Thr
500 505 510

Ile Lys Asn Leu Lys Pro Asn Thr Glu Tyr Lys Phe Ser Ile Ile Ala
515 520 525

Lys Asp Ala Ala Gly Asn Lys Ser Gln Pro Thr Ala Leu Thr Val Lys
530 535 540

Thr Asp Glu Ala Asn Met Thr Pro Pro Asp Gly Asn Gly Thr Ala Thr
545 550 555 560

Phe Ser Val Thr Ser Asn Trp Gly Ser Gly Tyr Asn Phe Ser Ile Ile
565 570 575

Ile Lys Asn Asn Gly Thr Asn Pro Ile Lys Asn Trp Lys Leu Glu Phe
580 585 590

Asp Tyr Ser Gly Asn Leu Thr Gln Val Trp Asp Ser Lys Ile Ser Ser
595 600 605

Lys Thr Asn Asn His Tyr Val Ile Thr Asn Ala Gly Trp Asn Gly Glu
610 615 620

Ile Pro Pro Gly Gly Ser Ile Thr Ile Gly Gly Ala Gly Thr Gly Asn
625 630 635 640

Pro Ala Glu Leu Leu Asn Ala Val Ile Ser Glu Asn Glu Leu Ile Glu
645 650 655

Gly Arg Gly Ser Asp Gly Asn Asp Leu Ile Gln Gly Gly Lys Gly Ala
660 665 670

Asp Phe Ile Glu Gly Gly Lys Gly Asn Asp Thr Ile Arg Asp Asn Ser
675 680 685

Gly His Asn Thr Phe Leu Phe Ser Gly His Phe Gly Gln Asp Arg Ile
690 695 700

Ile Gly Tyr Gln Pro Thr Asp Arg Leu Val Phe Gln Gly Ala Asp Gly
705 710 715 720

Ser Thr Asp Leu Arg Asp His Ala Lys Ala Val Gly Ala Asp Thr Val
725 730 735

Leu Ser Phe Gly Ala Asp Ser Val Thr Leu Val Gly Val Gly Leu Gly
740 745 750

Gly Leu Trp Ser Glu Gly Val Leu Ile Ser
755 760

<210> 11
<211> 466
<212> PRT
<213> Artificial Sequence

<220>
<223> Amino acid sequence of M37 lipase

<400> 11
Met Ser Arg His Met Ser Tyr Thr Lys Glu Gln Leu Met Leu Ala Phe
1 5 10 15

Ser Tyr Met Ser Tyr Tyr Gly Ile Thr His Thr Gly Ser Ala Lys Lys
20 25 30

Asn Ala Glu Leu Ile Leu Lys Lys Met Lys Glu Ala Leu Lys Thr Trp
35 40 45

Lys Pro Phe Gln Glu Asp Asp Trp Glu Val Val Trp Gly Pro Ala Val
50 55 60

Tyr Thr Met Pro Phe Thr Ile Phe Asn Asp Ala Met Met Tyr Val Ile
65 70 75 80

Gln Lys Lys Gly Ala Glu Gly Glu Tyr Val Ile Ala Ile Arg Gly Thr
85 90 95

Asn Pro Val Ser Ile Ser Asp Trp Leu Phe Asn Asp Phe Met Val Ser
100 105 110

Ala Met Lys Lys Trp Pro Tyr Ala Ser Val Glu Gly Arg Ile Leu Lys
115 120 125

Ile Ser Glu Ser Thr Ser Tyr Gly Leu Lys Thr Leu Gln Lys Leu Lys
130 135 140

Pro Lys Ser His Ile Pro Gly Glu Asn Lys Thr Ile Leu Gln Phe Leu
145 150 155 160

Asn Glu Lys Ile Gly Pro Glu Gly Lys Ala Lys Ile Cys Val Thr Gly
165 170 175

His Ser Lys Gly Gly Ala Leu Ser Ser Thr Leu Ala Leu Trp Leu Lys
180 185 190

Asp Ile Gln Gly Val Lys Leu Ser Gln Asn Ile Asp Ile Ser Thr Ile
195 200 205

Pro Phe Ala Gly Pro Thr Ala Gly Asn Ala Asp Phe Ala Asp Tyr Phe
210 215 220

Asp Asp Cys Leu Gly Asp Gln Cys Thr Arg Ile Ala Asn Ser Leu Asp
225 230 235 240

Ile Val Pro Tyr Ala Trp Asn Thr Asn Ser Leu Lys Lys Leu Lys Ser
245 250 255

Ile Tyr Ile Ser Glu Gln Ala Ser Val Lys Pro Leu Leu Tyr Gln Arg
260 265 270

Ala Leu Ile Arg Ala Met Ile Ala Glu Thr Lys Gly Lys Lys Tyr Lys
275 280 285

Gln Ile Lys Ala Glu Thr Pro Pro Leu Glu Gly Asn Ile Asn Pro Ile
290 295 300

Leu Ile Glu Tyr Leu Val Gln Ala Ala Tyr Gln His Val Val Gly Tyr
305 310 315 320

Pro Glu Leu Met Gly Met Met Asp Asp Ile Pro Leu Thr Asp Ile Phe
325 330 335

Glu Asp Ala Ile Ala Gly Leu Leu Leu Glu His His His His His His
340 345 350

Gly Thr Ala Ser Glu Leu Ile Glu Gly Arg Gly Ser Asp Gly Asn Asp

355 360 365

Leu Ile Gln Gly Gly Lys Gly Ala Asp Phe Ile Glu Gly Gly Lys Gly
370 375 380

Asn Asp Thr Ile Arg Asp Asn Ser Gly His Asn Thr Phe Leu Phe Ser
385 390 395 400

Gly His Phe Gly Gln Asp Arg Ile Ile Gly Tyr Gln Pro Thr Asp Arg
405 410 415

Leu Val Phe Gln Gly Ala Asp Gly Ser Thr Asp Leu Arg Asp His Ala
420 425 430

Lys Ala Val Gly Ala Asp Thr Val Leu Ser Phe Gly Ala Asp Ser Val
435 440 445

Thr Leu Val Gly Val Gly Leu Gly Gly Leu Trp Ser Glu Gly Val Leu
450 455 460

Ile Ser
465

<210> 12
<211> 505
<212> PRT
<213> Artificial Sequence

<220>
<223> Amino acid sequence of Capsid

<400> 12
Met Ser Arg Met Ala Arg Lys Lys Ser Thr Pro Arg Arg Arg Lys Ala
1 5 10 15

Val Lys Arg Arg Arg Thr Val Arg Arg Arg Gln Ser Pro Lys Ala Arg
20 25 30

Val Arg Ser Thr Thr Thr Lys Ala Lys Arg Arg Ile Ser Pro Ser Gly
35 40 45

Ser Gly Ser Gln His Leu Thr Val Arg Lys Gln Pro Phe Ser Asn Ala
50 55 60

Thr Ser Gln Pro Lys Ile Leu Asp Gly Ala Leu Thr Ser Ser Leu Ser

65 70 75 80

Arg Arg Leu Gln Asn Val Ile Gly Leu Thr Asn Gly Asn Gly Gly Leu
85 90 95

Gly Thr Glu Ile Met His Ile Phe Phe Ala Pro Thr Leu Gly Ile Pro
100 105 110

Leu Ile Ala Met Asn Ser Ala Glu Gly Val Ala Leu Arg Pro Ser Ser
115 120 125

Ser Ala Asp Pro Phe Phe Ile Gly Phe Pro Gly Gln Thr Ile Lys Phe
130 135 140

Asp Tyr Val Ser Ser Gly Thr Thr Pro Pro Ala Thr Gly Asn Leu Val
145 150 155 160

Thr Phe Ser Asn Glu Cys Gly Phe Ser Lys Trp Arg Ile Val Ser Gln
165 170 175

Gly Leu Arg Met Glu Leu Ala Asn Ser Asp Glu Glu Asn Asp Gly Trp
180 185 190

Phe Glu Ala Val Arg Phe Asn Trp Arg Asn Asn Pro Ala Asp Ile Cys
195 200 205

Phe Thr Pro Ile Asp Gly Thr Leu Gly Gly Ala Lys Thr Thr Asp Phe
210 215 220

Ala Val Ala Pro Ser Pro Val Gly Met Tyr Ala Leu Lys Asp Met Ala
225 230 235 240

Met Val Glu Gln Pro Gly Tyr Thr Thr Gly Leu Leu Lys Asp Leu Lys
245 250 255

Asn His Glu Phe Met Leu His Pro Gln Ser Thr Thr His Asp Pro Ile
260 265 270

Ile Leu Glu Gln Ser Tyr Glu Gly Thr Ile Gly Thr Thr Ala Ala Asp
275 280 285

Asp Met Tyr Tyr Ser Val Thr Ser Glu Val Phe Glu Leu Gly Asn Thr
290 295 300

Val Arg Gly Asn Thr Met Lys Asn Ser Leu Val Asp Asn Asn Met Asp
305 310 315 320

Trp Ile Tyr Leu Arg Leu His Cys Arg Thr Asn Asn Gly Thr Thr Ser
325 330 335

Asn Gly Ser Lys Leu Ile Val Asn Ala Ile Gln Asn Leu Glu Val Ser
340 345 350

Phe Asn Pro Ser Ser Asp Phe Ala Ala Phe Gln Thr Ile Asn Lys Met
355 360 365

His Pro Gln Gln Lys Lys Val Asp Asp Gln Leu Asn Asn Ser Ala Glu
370 375 380

Ala Ser Asn Lys Arg Gln Lys Thr Gly Gly Gly Glu Leu Ile Glu Gly
385 390 395 400

Arg Gly Ser Asp Gly Asn Asp Leu Ile Gln Gly Gly Lys Gly Ala Asp
405 410 415

Phe Ile Glu Gly Gly Lys Gly Asn Asp Thr Ile Arg Asp Asn Ser Gly
420 425 430

His Asn Thr Phe Leu Phe Ser Gly His Phe Gly Gln Asp Arg Ile Ile
435 440 445

Gly Tyr Gln Pro Thr Asp Arg Leu Val Phe Gln Gly Ala Asp Gly Ser
450 455 460

Thr Asp Leu Arg Asp His Ala Lys Ala Val Gly Ala Asp Thr Val Leu
465 470 475 480

Ser Phe Gly Ala Asp Ser Val Thr Leu Val Gly Val Gly Leu Gly Gly
485 490 495

Leu Trp Ser Glu Gly Val Leu Ile Ser
500 505

<210> 13
<211> 493
<212> PRT
<213> Artificial Sequence

<220>
<223> Amino acid sequence of DnaJ

<400> 13

Met Ser Arg Met Ala Lys Gln Asp Tyr Tyr Glu Ile Leu Gly Val Ser
1 5 10 15

Lys Thr Ala Glu Glu Arg Glu Ile Arg Lys Ala Tyr Lys Arg Leu Ala
20 25 30

Met Lys Tyr His Pro Asp Arg Asn Gln Gly Asp Lys Glu Ala Glu Ala
35 40 45

Lys Phe Lys Glu Ile Lys Glu Ala Tyr Glu Val Leu Thr Asp Ser Gln
50 55 60

Lys Arg Ala Ala Tyr Asp Gln Tyr Gly His Ala Ala Phe Glu Gln Gly
65 70 75 80

Gly Met Gly Gly Gly Gly Phe Gly Gly Gly Ala Asp Phe Ser Asp Ile
85 90 95

Phe Gly Asp Val Phe Gly Asp Ile Phe Gly Gly Gly Arg Gly Arg Gln
100 105 110

Arg Ala Ala Arg Gly Ala Asp Leu Arg Tyr Asn Met Glu Leu Thr Leu
115 120 125

Glu Glu Ala Val Arg Gly Val Thr Lys Glu Ile Arg Ile Pro Thr Leu
130 135 140

Glu Glu Cys Asp Val Cys His Gly Ser Gly Ala Lys Pro Gly Thr Gln
145 150 155 160

Pro Gln Thr Cys Pro Thr Cys His Gly Ser Gly Gln Val Gln Met Arg
165 170 175

Gln Gly Phe Phe Ala Val Gln Gln Thr Cys Pro His Cys Gln Gly Arg
180 185 190

Gly Thr Leu Ile Lys Asp Pro Cys Asn Lys Cys His Gly His Gly Arg
195 200 205

Val Glu Arg Ser Lys Thr Leu Ser Val Lys Ile Pro Ala Gly Val Asp
210 215 220

Thr Gly Asp Arg Ile Arg Leu Ala Gly Glu Gly Glu Ala Gly Glu His
225 230 235 240

Gly Ala Pro Ala Gly Asp Leu Tyr Val Gln Val Gln Val Lys Gln His
245 250 255

Pro Ile Phe Glu Arg Glu Gly Asn Asn Leu Tyr Cys Glu Val Pro Ile
260 265 270

Asn Phe Ala Met Ala Ala Leu Gly Gly Glu Ile Glu Val Pro Thr Leu
275 280 285

Asp Gly Arg Val Lys Leu Lys Val Pro Gly Glu Thr Gln Thr Gly Lys
290 295 300

Leu Phe Arg Met Arg Gly Lys Gly Val Lys Ser Val Arg Gly Gly Ala
305 310 315 320

Gln Gly Asp Leu Leu Cys Arg Val Val Val Glu Thr Pro Val Gly Leu
325 330 335

Asn Glu Arg Gln Lys Gln Leu Leu Gln Glu Leu Gln Glu Ser Phe Gly
340 345 350

Gly Pro Thr Gly Glu His Asn Ser Pro Arg Ser Lys Ser Phe Phe Asp
355 360 365

Gly Val Lys Lys Phe Phe Asp Asp Leu Thr Arg Gly Thr Ala Ser Glu
370 375 380

Leu Ile Glu Gly Arg Gly Ser Asp Gly Asn Asp Leu Ile Gln Gly Gly
385 390 395 400

Lys Gly Ala Asp Phe Ile Glu Gly Gly Lys Gly Asn Asp Thr Ile Arg
405 410 415

Asp Asn Ser Gly His Asn Thr Phe Leu Phe Ser Gly His Phe Gly Gln
420 425 430

Asp Arg Ile Ile Gly Tyr Gln Pro Thr Asp Arg Leu Val Phe Gln Gly
435 440 445

Ala Asp Gly Ser Thr Asp Leu Arg Asp His Ala Lys Ala Val Gly Ala
450 455 460

Asp Thr Val Leu Ser Phe Gly Ala Asp Ser Val Thr Leu Val Gly Val
465 470 475 480

Gly Leu Gly Gly Leu Trp Ser Glu Gly Val Leu Ile Ser
485 490

<210> 14
<211> 348
<212> PRT
<213> Artificial Sequence

<220>
<223> Amino acid sequence of Eg15

<400> 14
Met Ser Arg His His His His His His Tyr Lys Ala Thr Thr Thr Arg
1 5 10 15

Tyr Tyr Asp Gly Gln Glu Gly Ala Cys Gly Cys Gly Ser Ser Ser Gly
20 25 30

Ala Phe Pro Trp Gln Leu Gly Ile Gly Asn Gly Val Tyr Thr Ala Ala
35 40 45

Gly Ser Gln Ala Leu Phe Asp Thr Ala Gly Ala Ser Trp Cys Gly Ala
50 55 60

Gly Cys Gly Lys Cys Tyr Gln Leu Thr Ser Thr Gly Gln Ala Pro Cys
65 70 75 80

Ser Ser Cys Gly Thr Gly Gly Ala Ala Gly Gln Ser Ile Ile Val Met
85 90 95

Val Thr Asn Leu Cys Pro Asn Asn Gly Asn Ala Gln Trp Cys Pro Val
100 105 110

Val Gly Gly Thr Asn Gln Tyr Gly Tyr Ser Tyr His Phe Asp Ile Met
115 120 125

Ala Gln Asn Glu Ile Phe Gly Asp Asn Val Val Val Asp Phe Glu Pro
130 135 140

Ile Ala Cys Pro Gly Gln Ala Ala Ser Asp Trp Gly Thr Cys Leu Cys
145 150 155 160

Val Gly Gln Gln Glu Thr Asp Pro Thr Pro Val Leu Gly Asn Asp Thr
165 170 175

Gly Ser Thr Pro Pro Gly Ser Ser Pro Pro Ala Thr Ser Ser Ser Pro
180 185 190

Pro Ser Gly Gly Gly Gln Gln Thr Leu Tyr Gly Gln Cys Gly Gly Ala

195 200 205

Gly Trp Thr Gly Pro Thr Thr Cys Gln Ala Pro Gly Thr Cys Lys Val
210 215 220

Gln Asn Gln Trp Tyr Ser Gln Cys Leu Pro Gly Thr Ala Ser Glu Leu
225 230 235 240

Ile Glu Gly Arg Gly Ser Asp Gly Asn Asp Leu Ile Gln Gly Gly Lys
245 250 255

Gly Ala Asp Phe Ile Glu Gly Gly Lys Gly Asn Asp Thr Ile Arg Asp
260 265 270

Asn Ser Gly His Asn Thr Phe Leu Phe Ser Gly His Phe Gly Gln Asp
275 280 285

Arg Ile Ile Gly Tyr Gln Pro Thr Asp Arg Leu Val Phe Gln Gly Ala
290 295 300

Asp Gly Ser Thr Asp Leu Arg Asp His Ala Lys Ala Val Gly Ala Asp
305 310 315 320

Thr Val Leu Ser Phe Gly Ala Asp Ser Val Thr Leu Val Gly Val Gly
325 330 335

Leu Gly Gly Leu Trp Ser Glu Gly Val Leu Ile Ser
340 345

<210> 15
<211> 347
<212> PRT
<213> Artificial Sequence

<220>
<223> Amino acid sequence of GFP

<400> 15
Met Ser Arg Met Ser Lys Gly Glu Glu Leu Phe Thr Gly Val Val Pro
1 5 10 15

Ile Leu Val Glu Leu Asp Gly Asp Val Asn Gly His Lys Phe Ser Val
20 25 30

Ser Gly Glu Gly Glu Gly Asp Ala Thr Tyr Gly Lys Leu Thr Leu Lys

35 40 45

Phe Ile Cys Thr Thr Gly Lys Leu Pro Val Pro Trp Pro Thr Leu Val
50 55 60

Thr Thr Phe Ser Tyr Gly Val Gln Cys Phe Ser Arg Tyr Pro Asp His
65 70 75 80

Met Lys Arg His Asp Phe Phe Lys Ser Ala Met Pro Glu Gly Tyr Val
85 90 95

Gln Glu Arg Thr Ile Ser Phe Lys Asp Asp Gly Asn Tyr Lys Thr Arg
100 105 110

Ala Glu Val Lys Phe Glu Gly Asp Thr Leu Val Asn Arg Ile Glu Leu
115 120 125

Lys Gly Ile Asp Phe Lys Glu Asp Gly Asn Ile Leu Gly His Lys Leu
130 135 140

Glu Tyr Asn Tyr Asn Ser His Asn Val Tyr Ile Thr Ala Asp Lys Gln
145 150 155 160

Lys Asn Gly Ile Lys Ala Asn Phe Lys Ile Arg His Asn Ile Glu Asp
165 170 175

Gly Ser Val Gln Leu Ala Asp His Tyr Gln Gln Asn Thr Pro Ile Gly
180 185 190

Asp Gly Pro Val Leu Leu Pro Asp Asn His Tyr Leu Ser Thr Gln Ser
195 200 205

Ala Leu Ser Lys Asp Pro Asn Glu Lys Arg Asp His Met Val Leu Leu
210 215 220

Glu Phe Val Thr Ala Ala Gly Ile Thr His Gly Met Asp Glu Leu Ile
225 230 235 240

Glu Gly Arg Gly Ser Asp Gly Asn Asp Leu Ile Gln Gly Gly Lys Gly
245 250 255

Ala Asp Phe Ile Glu Gly Gly Lys Gly Asn Asp Thr Ile Arg Asp Asn
260 265 270

Ser Gly His Asn Thr Phe Leu Phe Ser Gly His Phe Gly Gln Asp Arg
275 280 285

Ile Ile Gly Tyr Gln Pro Thr Asp Arg Leu Val Phe Gln Gly Ala Asp
290 295 300

Gly Ser Thr Asp Leu Arg Asp His Ala Lys Ala Val Gly Ala Asp Thr
305 310 315 320

Val Leu Ser Phe Gly Ala Asp Ser Val Thr Leu Val Gly Val Gly Leu
325 330 335

Gly Gly Leu Trp Ser Glu Gly Val Leu Ile Ser
340 345

<210> 16
<211> 361
<212> PRT
<213> Artificial Sequence

<220>
<223> Amino acid sequence of -30 Negatively supercharged GFP

<400> 16
Met Ser Arg Met Gly His His His His His His Gly Gly Ala Ser Lys
1 5 10 15

Gly Glu Glu Leu Phe Asp Gly Val Val Pro Ile Leu Val Glu Leu Asp
20 25 30

Gly Asp Val Asn Gly His Glu Phe Ser Val Arg Gly Glu Gly Glu Gly
35 40 45

Asp Ala Thr Glu Gly Glu Leu Thr Leu Lys Phe Ile Cys Thr Thr Gly
50 55 60

Glu Leu Pro Val Pro Trp Pro Thr Leu Val Thr Thr Leu Thr Tyr Gly
65 70 75 80

Val Gln Cys Phe Ser Asp Tyr Pro Asp His Met Asp Gln His Asp Phe
85 90 95

Phe Lys Ser Ala Met Pro Glu Gly Tyr Val Gln Glu Arg Thr Ile Ser
100 105 110

Phe Lys Asp Asp Gly Thr Tyr Lys Thr Arg Ala Glu Val Lys Phe Glu
115 120 125

Gly Asp Thr Leu Val Asn Arg Ile Glu Leu Lys Gly Ile Asp Phe Lys
130 135 140

Glu Asp Gly Asn Ile Leu Gly His Lys Leu Glu Tyr Asn Phe Asn Ser
145 150 155 160

His Asp Val Tyr Ile Thr Ala Asp Lys Gln Glu Asn Gly Ile Lys Ala
165 170 175

Glu Phe Glu Ile Arg His Asn Val Glu Asp Gly Ser Val Gln Leu Ala
180 185 190

Asp His Tyr Gln Gln Asn Thr Pro Ile Gly Asp Gly Pro Val Leu Leu
195 200 205

Pro Asp Asp His Tyr Leu Ser Thr Glu Ser Ala Leu Ser Lys Asp Pro
210 215 220

Asn Glu Asp Arg Asp His Met Val Leu Leu Glu Phe Val Thr Ala Ala
225 230 235 240

Gly Ile Asp His Gly Met Asp Glu Leu Tyr Lys Glu Leu Ile Glu Gly
245 250 255

Arg Gly Ser Asp Gly Asn Asp Leu Ile Gln Gly Gly Lys Gly Ala Asp
260 265 270

Phe Ile Glu Gly Gly Lys Gly Asn Asp Thr Ile Arg Asp Asn Ser Gly
275 280 285

His Asn Thr Phe Leu Phe Ser Gly His Phe Gly Gln Asp Arg Ile Ile
290 295 300

Gly Tyr Gln Pro Thr Asp Arg Leu Val Phe Gln Gly Ala Asp Gly Ser
305 310 315 320

Thr Asp Leu Arg Asp His Ala Lys Ala Val Gly Ala Asp Thr Val Leu
325 330 335

Ser Phe Gly Ala Asp Ser Val Thr Leu Val Gly Val Gly Leu Gly Gly
340 345 350

Leu Trp Ser Glu Gly Val Leu Ile Ser
355 360

<210> 17

<211> 361
<212> PRT
<213> Artificial Sequence

<220>
<223> Amino acid sequence of +36 Positively supercharged GFP

<400> 17
Met Ser Arg Met Gly His His His His His His Gly Gly Ala Ser Lys
1 5 10 15

Gly Glu Arg Leu Phe Arg Gly Lys Val Pro Ile Leu Val Glu Leu Lys
20 25 30

Gly Asp Val Asn Gly His Lys Phe Ser Val Arg Gly Lys Gly Lys Gly
35 40 45

Asp Ala Thr Arg Gly Lys Leu Thr Leu Lys Phe Ile Cys Thr Thr Gly
50 55 60

Lys Leu Pro Val Pro Trp Pro Thr Leu Val Thr Thr Leu Thr Tyr Gly
65 70 75 80

Val Gln Cys Phe Ser Arg Tyr Pro Lys His Met Lys Arg His Asp Phe
85 90 95

Phe Lys Ser Ala Met Pro Lys Gly Tyr Val Gln Glu Arg Thr Ile Ser
100 105 110

Phe Lys Lys Asp Gly Lys Tyr Lys Thr Arg Ala Glu Val Lys Phe Glu
115 120 125

Gly Arg Thr Leu Val Asn Arg Ile Lys Leu Lys Gly Arg Asp Phe Lys
130 135 140

Glu Lys Gly Asn Ile Leu Gly His Lys Leu Arg Tyr Asn Phe Asn Ser
145 150 155 160

His Lys Val Tyr Ile Thr Ala Asp Lys Arg Lys Asn Gly Ile Lys Ala
165 170 175

Lys Phe Lys Ile Arg His Asn Val Lys Asp Gly Ser Val Gln Leu Ala
180 185 190

Asp His Tyr Gln Gln Asn Thr Pro Ile Gly Arg Gly Pro Val Leu Leu
195 200 205

Pro Arg Asn His Tyr Leu Ser Thr Arg Ser Lys Leu Ser Lys Asp Pro
210 215 220

Lys Glu Lys Arg Asp His Met Val Leu Leu Glu Phe Val Thr Ala Ala
225 230 235 240

Gly Ile Lys His Gly Arg Asp Glu Arg Tyr Lys Glu Leu Ile Glu Gly
245 250 255

Arg Gly Ser Asp Gly Asn Asp Leu Ile Gln Gly Gly Lys Gly Ala Asp
260 265 270

Phe Ile Glu Gly Gly Lys Gly Asn Asp Thr Ile Arg Asp Asn Ser Gly
275 280 285

His Asn Thr Phe Leu Phe Ser Gly His Phe Gly Gln Asp Arg Ile Ile
290 295 300

Gly Tyr Gln Pro Thr Asp Arg Leu Val Phe Gln Gly Ala Asp Gly Ser
305 310 315 320

Thr Asp Leu Arg Asp His Ala Lys Ala Val Gly Ala Asp Thr Val Leu
325 330 335

Ser Phe Gly Ala Asp Ser Val Thr Leu Val Gly Val Gly Leu Gly Gly
340 345 350

Leu Trp Ser Glu Gly Val Leu Ile Ser
355 360

<210> 18
<211> 164
<212> PRT
<213> Artificial Sequence

<220>
<223> Amino acid sequence of epidermal growth factor

<400> 18
Met Asn Ser Asp Ser Glu Cys Pro Leu Ser His Asp Gly Tyr Cys Leu
1 5 10 15

His Asp Gly Val Cys Met Tyr Ile Glu Ala Leu Asp Lys Tyr Ala Cys
20 25 30

Asn Cys Val Val Gly Tyr Ile Gly Glu Arg Cys Gln Tyr Arg Asp Leu
35 40 45

Lys Trp Trp Glu Leu Arg Ser Arg Ile Glu Gly Arg Gly Ser Asp Gly
50 55 60

Asn Asp Leu Ile Gln Gly Gly Lys Gly Ala Asp Phe Ile Glu Gly Gly
65 70 75 80

Lys Gly Asn Asp Thr Ile Arg Asp Asn Ser Gly His Asn Thr Phe Leu
85 90 95

Phe Ser Gly His Phe Gly Gln Asp Arg Ile Ile Gly Tyr Gln Pro Thr
100 105 110

Asp Arg Leu Val Phe Gln Gly Ala Asp Gly Ser Thr Asp Leu Arg Asp
115 120 125

His Ala Lys Ala Val Gly Ala Asp Thr Val Leu Ser Phe Gly Ala Asp
130 135 140

Ser Val Thr Leu Val Gly Val Gly Leu Gly Gly Leu Trp Ser Glu Gly
145 150 155 160

Val Leu Ile Ser

<210> 19
<211> 559
<212> PRT
<213> Artificial Sequence

<220>
<223> Amino acid sequence of Alkaline phosphatase

<400> 19
Met Ser Ser Met Pro Val Leu Glu Asn Arg Ala Ala Gln Gly Asp Ile
1 5 10 15

Thr Ala Pro Gly Gly Ala Arg Arg Leu Thr Gly Asp Gln Thr Ala Ala
20 25 30

Leu Arg Asp Ser Leu Ser Asp Lys Pro Ala Lys Asn Ile Ile Leu Leu
35 40 45

Ile Gly Asp Gly Met Gly Asp Ser Glu Ile Thr Ala Ala Arg Asn Tyr
50 55 60

Ala Glu Gly Ala Gly Gly Phe Phe Lys Gly Ile Asp Ala Leu Pro Leu
65 70 75 80

Thr Gly Gln Tyr Thr His Tyr Ala Leu Asn Lys Lys Thr Gly Lys Pro
85 90 95

Asp Tyr Val Thr Asp Ser Ala Ala Ser Ala Thr Ala Trp Ser Thr Gly
100 105 110

Val Lys Thr Tyr Asn Gly Ala Leu Gly Val Asp Ile His Glu Lys Asp
115 120 125

His Pro Thr Ile Leu Glu Met Ala Lys Ala Ala Gly Leu Ala Thr Gly
130 135 140

Asn Val Ser Thr Ala Glu Leu Gln Asp Ala Thr Pro Ala Ala Leu Val
145 150 155 160

Ala His Val Thr Ser Arg Lys Cys Tyr Gly Pro Ser Ala Thr Ser Glu
165 170 175

Lys Cys Pro Gly Asn Ala Leu Glu Lys Gly Gly Lys Gly Ser Ile Thr
180 185 190

Glu Gln Leu Leu Asn Ala Arg Ala Asp Val Thr Leu Gly Gly Gly Ala
195 200 205

Lys Thr Phe Ala Glu Thr Ala Thr Ala Gly Glu Trp Gln Gly Lys Thr
210 215 220

Leu Arg Glu Gln Ala Gln Ala Arg Gly Tyr Gln Leu Val Ser Asp Ala
225 230 235 240

Ala Ser Leu Asn Ser Val Thr Glu Ala Asn Gln Gln Lys Pro Leu Leu
245 250 255

Gly Leu Phe Ala Asp Gly Asn Met Pro Val Arg Trp Leu Gly Pro Lys
260 265 270

Ala Thr Tyr His Gly Asn Ile Asp Lys Pro Ala Val Thr Cys Thr Pro
275 280 285

Asn Pro Gln Arg Asn Asp Ser Val Pro Thr Leu Ala Gln Met Thr Asp

290 295 300

Lys Ala Ile Glu Leu Leu Ser Lys Asn Glu Lys Gly Phe Phe Leu Gln
305 310 315 320

Val Glu Gly Ala Ser Ile Asp Lys Gln Asp His Ala Ala Asn Pro Cys
325 330 335

Gly Gln Ile Gly Glu Thr Val Asp Leu Asp Glu Ala Val Gln Arg Ala
340 345 350

Leu Glu Phe Ala Lys Lys Glu Gly Asn Thr Leu Val Ile Val Thr Ala
355 360 365

Asp His Ala His Ala Ser Gln Ile Val Ala Pro Asp Thr Lys Ala Pro
370 375 380

Gly Leu Thr Gln Ala Leu Asn Thr Lys Asp Gly Ala Val Met Val Met
385 390 395 400

Ser Tyr Gly Asn Ser Glu Glu Asp Ser Gln Glu His Thr Gly Ser Gln
405 410 415

Leu Arg Ile Ala Ala Tyr Gly Pro His Ala Ala Asn Val Val Gly Leu
420 425 430

Thr Asp Gln Thr Asp Leu Phe Tyr Thr Met Lys Ala Ala Leu Gly Leu
435 440 445

Lys Glu Leu Ile Glu Gly Arg Gly Ser Asp Gly Asn Asp Leu Ile Gln
450 455 460

Gly Gly Lys Gly Ala Asp Phe Ile Glu Gly Gly Lys Gly Asn Asp Thr
465 470 475 480

Ile Arg Asp Asn Ser Gly His Asn Thr Phe Leu Phe Ser Gly His Phe
485 490 495

Gly Gln Asp Arg Ile Ile Gly Tyr Gln Pro Thr Asp Arg Leu Val Phe
500 505 510

Gln Gly Ala Asp Gly Ser Thr Asp Leu Arg Asp His Ala Lys Ala Val
515 520 525

Gly Ala Asp Thr Val Leu Ser Phe Gly Ala Asp Ser Val Thr Leu Val
530 535 540

Gly Val Gly Leu Gly Gly Leu Trp Ser Glu Gly Val Leu Ile Ser
545 550 555

<210> 20
<211> 440
<212> PRT
<213> Artificial Sequence

<220>
<223> Amino acid sequence of Phospholipase A1

<400> 20
Met Ser Met Ser Leu Ser Phe Thr Ser Ala Ile Ala Pro Ala Ala Ile
1 5 10 15

Gln Pro Pro Met Val Arg Thr Gln Pro Glu Pro Leu Ser Ser Ser Gln
20 25 30

Pro Val Glu Ala Ser Ala Thr Lys Ala Pro Val Ala Thr Leu Ser Gln
35 40 45

Asn Ser Leu Asn Ala Gln Ser Leu Leu Asn Thr Leu Val Ser Glu Ile
50 55 60

Ser Ala Ala Ala Pro Ala Ala Ala Asn Gln Gly Val Thr Arg Gly Gln
65 70 75 80

Gln Pro Gln Lys Gly Asp Tyr Thr Leu Ala Leu Leu Ala Lys Asp Val
85 90 95

Tyr Ser Thr Gly Ser Gln Gly Val Glu Gly Phe Asn Arg Leu Ser Ala
100 105 110

Asp Ala Leu Leu Gly Ala Gly Ile Asp Pro Ala Ser Leu Gln Asp Ala
115 120 125

Ala Ser Gly Phe Gln Ala Gly Ile Tyr Thr Asp Asn Gln Gln Tyr Val
130 135 140

Leu Ala Phe Ala Gly Thr Asn Asp Met Arg Asp Trp Leu Ser Asn Val
145 150 155 160

Arg Gln Ala Thr Gly Tyr Asp Asp Val Gln Tyr Asn Gln Ala Val Ser
165 170 175

Leu Ala Lys Ser Ala Lys Ala Ala Phe Gly Asp Ala Leu Val Ile Ala
180 185 190

Gly His Ser Leu Gly Gly Gly Leu Ala Ala Thr Ala Ala Leu Ala Thr
195 200 205

Gly Thr Val Ala Val Thr Phe Asn Ala Ala Gly Val Ser Asp Tyr Thr
210 215 220

Leu Asn Arg Met Gly Ile Asp Pro Ala Ala Ala Lys Gln Asp Ala Gln
225 230 235 240

Ala Gly Gly Ile Arg Arg Tyr Ser Glu Gln Tyr Asp Met Leu Thr Gly
245 250 255

Thr Gln Glu Ser Thr Ser Leu Ile Pro Asp Ala Ile Gly His Lys Ile
260 265 270

Thr Leu Ala Asn Asn Asp Thr Leu Ser Gly Ile Asp Asp Trp Arg Pro
275 280 285

Ser Lys His Leu Asp Arg Ser Leu Thr Ala His Gly Ile Asp Lys Val
290 295 300

Ile Ser Ser Met Ala Glu Gln Lys Pro Trp Glu Ala Met Ala Asn Ala
305 310 315 320

His His His His His His Gly Thr Ala Ser Glu Leu Ile Glu Gly Arg
325 330 335

Gly Ser Asp Gly Asn Asp Leu Ile Gln Gly Gly Lys Gly Ala Asp Phe
340 345 350

Ile Glu Gly Gly Lys Gly Asn Asp Thr Ile Arg Asp Asn Ser Gly His
355 360 365

Asn Thr Phe Leu Phe Ser Gly His Phe Gly Gln Asp Arg Ile Ile Gly
370 375 380

Tyr Gln Pro Thr Asp Arg Leu Val Phe Gln Gly Ala Asp Gly Ser Thr
385 390 395 400

Asp Leu Arg Asp His Ala Lys Ala Val Gly Ala Asp Thr Val Leu Ser
405 410 415

Phe Gly Ala Asp Ser Val Thr Leu Val Gly Val Gly Leu Gly Gly Leu
420 425 430

Trp Ser Glu Gly Val Leu Ile Ser
435 440

<210> 21
<211> 4
<212> PRT
<213> Artificial Sequence

<220>
<223> Amino acid sequence of Factor Xa

<400> 21
Ile Glu Gly Arg
1

<210> 22
<211> 104
<212> PRT
<213> Artificial Sequence

<220>
<223> Amino acid sequence of LARD3 signal peptide

<400> 22
Gly Ser Asp Gly Asn Asp Leu Ile Gln Gly Gly Lys Gly Ala Asp Phe
1 5 10 15

Ile Glu Gly Gly Lys Gly Asn Asp Thr Ile Arg Asp Asn Ser Gly His
20 25 30

Asn Thr Phe Leu Phe Ser Gly His Phe Gly Gln Asp Arg Ile Ile Gly
35 40 45

Tyr Gln Pro Thr Asp Arg Leu Val Phe Gln Gly Ala Asp Gly Ser Thr
50 55 60

Asp Leu Arg Asp His Ala Lys Ala Val Gly Ala Asp Thr Val Leu Ser
65 70 75 80

Phe Gly Ala Asp Ser Val Thr Leu Val Gly Val Gly Leu Gly Gly Leu
85 90 95

Trp Ser Glu Gly Val Leu Ile Ser
100

<210> 23
<211> 119
<212> PRT
<213> Artificial Sequence

<220>
<223> Amino acid sequence of pDART Translation Structure

<400> 23
Met Ser Arg His Met Gly Thr Ala Ser Glu Leu Ile Glu Gly Arg Gly
1 5 10 15

Ser Asp Gly Asn Asp Leu Ile Gln Gly Gly Lys Gly Ala Asp Phe Ile
20 25 30

Glu Gly Gly Lys Gly Asn Asp Thr Ile Arg Asp Asn Ser Gly His Asn
35 40 45

Thr Phe Leu Phe Ser Gly His Phe Gly Gln Asp Arg Ile Ile Gly Tyr
50 55 60

Gln Pro Thr Asp Arg Leu Val Phe Gln Gly Ala Asp Gly Ser Thr Asp
65 70 75 80

Leu Arg Asp His Ala Lys Ala Val Gly Ala Asp Thr Val Leu Ser Phe
85 90 95

Gly Ala Asp Ser Val Thr Leu Val Gly Val Gly Leu Gly Gly Leu Trp
100 105 110

Ser Glu Gly Val Leu Ile Ser
115

<210> 24
<211> 131
<212> PRT
<213> Artificial Sequence

<220>
<223> Amino acid sequence of pFD10 Translation Structure

<400> 24

Met Ser Ser Asp Asp Asp Asp Asp Asp Asp Asp Asp Asp Ser Arg His
1 5 10 15

Met Gly Thr Ala Ser Glu Leu Ile Glu Gly Arg Gly Ser Asp Gly Asn
20 25 30

Asp Leu Ile Gln Gly Gly Lys Gly Ala Asp Phe Ile Glu Gly Gly Lys
35 40 45

Gly Asn Asp Thr Ile Arg Asp Asn Ser Gly His Asn Thr Phe Leu Phe
50 55 60

Ser Gly His Phe Gly Gln Asp Arg Ile Ile Gly Tyr Gln Pro Thr Asp
65 70 75 80

Arg Leu Val Phe Gln Gly Ala Asp Gly Ser Thr Asp Leu Arg Asp His
85 90 95

Ala Lys Ala Val Gly Ala Asp Thr Val Leu Ser Phe Gly Ala Asp Ser
100 105 110

Val Thr Leu Val Gly Val Gly Leu Gly Gly Leu Trp Ser Glu Gly Val
115 120 125

Leu Ile Ser
130

<210> 25
<211> 130
<212> PRT
<213> Artificial Sequence

<220>
<223> Amino acid sequence of pBD10 Translation Structure

<400> 25

Met Ser Arg His Met Gly Thr Ala Ser Glu Leu Asp Asp Asp Asp Asp
1 5 10 15

Asp Asp Asp Asp Asp Asp Ile Glu Gly Arg Gly Ser Asp Gly Asn Asp
20 25 30

Leu Ile Gln Gly Gly Lys Gly Ala Asp Phe Ile Glu Gly Gly Lys Gly

35 40 45

Asn Asp Thr Ile Arg Asp Asn Ser Gly His Asn Thr Phe Leu Phe Ser
50 55 60

Gly His Phe Gly Gln Asp Arg Ile Ile Gly Tyr Gln Pro Thr Asp Arg
65 70 75 80

Leu Val Phe Gln Gly Ala Asp Gly Ser Thr Asp Leu Arg Asp His Ala
85 90 95

Lys Ala Val Gly Ala Asp Thr Val Leu Ser Phe Gly Ala Asp Ser Val
100 105 110

Thr Leu Val Gly Val Gly Leu Gly Gly Leu Trp Ser Glu Gly Val Leu
115 120 125

Ile Ser
130

<210> 26
<211> 130
<212> PRT
<213> Artificial Sequence

<220>
<223> Amino acid sequence of pBE10 Translation Structure

<400> 26
Met Ser Arg His Met Gly Thr Ala Ser Glu Leu Glu Glu Glu Glu Glu
1 5 10 15

Glu Glu Glu Glu Glu Gly Ile Glu Gly Arg Gly Ser Asp Gly Asn Asp
20 25 30

Leu Ile Gln Gly Gly Lys Gly Ala Asp Phe Ile Glu Gly Gly Lys Gly
35 40 45

Asn Asp Thr Ile Arg Asp Asn Ser Gly His Asn Thr Phe Leu Phe Ser
50 55 60

Gly His Phe Gly Gln Asp Arg Ile Ile Gly Tyr Gln Pro Thr Asp Arg
65 70 75 80

Leu Val Phe Gln Gly Ala Asp Gly Ser Thr Asp Leu Arg Asp His Ala

85 90 95

Lys Ala Val Gly Ala Asp Thr Val Leu Ser Phe Gly Ala Asp Ser Val
100 105 110

Thr Leu Val Gly Val Gly Leu Gly Gly Leu Trp Ser Glu Gly Val Leu
115 120 125

Ile Ser
130

<210> 27
<211> 130
<212> PRT
<213> Artificial Sequence

<220>
<223> Amino acid sequence of pBH10 Translation Structure

<400> 27
Met Ser Arg His Met Gly Thr Ala Ser Glu Leu His His His His His
1 5 10 15

His His His His His Gly Ile Glu Gly Arg Gly Ser Asp Gly Asn Asp
20 25 30

Leu Ile Gln Gly Gly Lys Gly Ala Asp Phe Ile Glu Gly Gly Lys Gly
35 40 45

Asn Asp Thr Ile Arg Asp Asn Ser Gly His Asn Thr Phe Leu Phe Ser
50 55 60

Gly His Phe Gly Gln Asp Arg Ile Ile Gly Tyr Gln Pro Thr Asp Arg
65 70 75 80

Leu Val Phe Gln Gly Ala Asp Gly Ser Thr Asp Leu Arg Asp His Ala
85 90 95

Lys Ala Val Gly Ala Asp Thr Val Leu Ser Phe Gly Ala Asp Ser Val
100 105 110

Thr Leu Val Gly Val Gly Leu Gly Gly Leu Trp Ser Glu Gly Val Leu
115 120 125

Ile Ser

130

<210> 28
<211> 130
<212> PRT
<213> Artificial Sequence

<220>
<223> Amino acid sequence of pBR10 Translation Structure

<400> 28
Met Ser Arg His Met Gly Thr Ala Ser Glu Leu Arg Arg Arg Arg Arg
1 5 10 15

Arg Arg Arg Arg Arg Gly Ile Glu Gly Arg Gly Ser Asp Gly Asn Asp
20 25 30

Leu Ile Gln Gly Gly Lys Gly Ala Asp Phe Ile Glu Gly Gly Lys Gly
35 40 45

Asn Asp Thr Ile Arg Asp Asn Ser Gly His Asn Thr Phe Leu Phe Ser
50 55 60

Gly His Phe Gly Gln Asp Arg Ile Ile Gly Tyr Gln Pro Thr Asp Arg
65 70 75 80

Leu Val Phe Gln Gly Ala Asp Gly Ser Thr Asp Leu Arg Asp His Ala
85 90 95

Lys Ala Val Gly Ala Asp Thr Val Leu Ser Phe Gly Ala Asp Ser Val
100 105 110

Thr Leu Val Gly Val Gly Leu Gly Gly Leu Trp Ser Glu Gly Val Leu
115 120 125

Ile Ser
130

<210> 29
<211> 16
<212> PRT
<213> Artificial Sequence

<220>

<223> Amino acid sequence of LARD purification

<400> 29
Val Leu Ser Phe Gly Ala Asp Ser Val Thr Leu Val Gly Val Gly Leu
1 5 10 15

<210> 30
<211> 44
<212> PRT
<213> Artificial Sequence

<220>
<223> Amino acid sequence of LARD secretion signal peptide

<400> 30
Gly Ser Asp Gly Asn Asp Leu Ile Gln Gly Gly Lys Gly Ala Asp Phe
1 5 10 15

Ile Glu Gly Gly Lys Gly Asn Asp Thr Ile Arg Asp Asn Ser Gly His
20 25 30

Asn Thr Phe Leu Phe Ser Gly His Phe Gly Gln Asp
35 40

<210> 31
<211> 174
<212> PRT
<213> Artificial Sequence

<220>
<223> Amino acid sequence of LARD1

<400> 31
Ser Ile Ala Asn Leu Ser Thr Trp Val Ser His Leu Pro Ser Ala Tyr
1 5 10 15

Gly Asp Gly Met Thr Arg Val Leu Glu Ser Gly Phe Tyr Glu Gln Met
20 25 30

Thr Arg Asp Ser Thr Ile Ile Val Ala Asn Leu Ser Asp Pro Ala Arg

35 40 45

Ala Asn Thr Trp Val Gln Asp Leu Asn Arg Asn Ala Glu Pro His Thr
50 55 60

Gly Asn Thr Phe Ile Ile Gly Ser Asp Gly Asn Asp Leu Ile Gln Gly
65 70 75 80

Gly Lys Gly Ala Asp Phe Ile Glu Gly Gly Lys Gly Asn Asp Thr Ile
85 90 95

Arg Asp Asn Ser Gly His Asn Thr Phe Leu Phe Ser Gly His Phe Gly
100 105 110

Gln Asp Arg Ile Ile Gly Tyr Gln Pro Thr Asp Arg Leu Val Phe Gln
115 120 125

Gly Ala Asp Gly Ser Thr Asp Leu Arg Asp His Ala Lys Ala Val Gly
130 135 140

Ala Asp Thr Val Leu Ser Phe Gly Ala Asp Ser Val Thr Leu Val Gly
145 150 155 160

Val Gly Leu Gly Gly Leu Trp Ser Glu Gly Val Leu Ile Ser
165 170

<210> 32
<211> 140
<212> PRT
<213> Artificial Sequence

<220>
<223> Amino acid sequence of LARD2

<400> 32
Asp Ser Thr Ile Ile Val Ala Asn Leu Ser Asp Pro Ala Arg Ala Asn
1 5 10 15

Thr Trp Val Gln Asp Leu Asn Arg Asn Ala Glu Pro His Thr Gly Asn
20 25 30

Thr Phe Ile Ile Gly Ser Asp Gly Asn Asp Leu Ile Gln Gly Gly Lys
35 40 45

Gly Ala Asp Phe Ile Glu Gly Gly Lys Gly Asn Asp Thr Ile Arg Asp

50 55 60

Asn Ser Gly His Asn Thr Phe Leu Phe Ser Gly His Phe Gly Gln Asp
65 70 75 80

Arg Ile Ile Gly Tyr Gln Pro Thr Asp Arg Leu Val Phe Gln Gly Ala
85 90 95

Asp Gly Ser Thr Asp Leu Arg Asp His Ala Lys Ala Val Gly Ala Asp
100 105 110

Thr Val Leu Ser Phe Gly Ala Asp Ser Val Thr Leu Val Gly Val Gly
115 120 125

Leu Gly Gly Leu Trp Ser Glu Gly Val Leu Ile Ser
130 135 140

<210> 33
<211> 10
<212> PRT
<213> Artificial Sequence

<220>
<223> 10 Asparatic acid

<400> 33
Asp Asp Asp Asp Asp Asp Asp Asp Asp Asp
1 5 10

<210> 34
<211> 32
<212> DNA
<213> Artificial Sequence

<220>
<223> hlyBD-s primer

<400> 34
ggggagctcg gattcttgtc ataaaattga tt 32

<210> 35
<211> 31

<212> DNA
<213> Artificial Sequence

<220>
<223> hlyBD-a primer

<400> 35
gggggatcct taacgctcat gtaaactttc t 31

## Description

[TECHNICAL FIELD]

The present invention relates to a method of increasing secretion of a target protein linked to lipase ABC transporter recognition domain (LARD3), by using bacterial Type 1 Secretion system (T1SS) and lowering a pI, isoelectric point of the target protein in extracellular secretion of the target protein, and a method of producing a target protein efficiently.

[BACKGROUND ART]

Mass production of recombinant proteins is an important issue in various industries. A general method for mass production of recombinant proteins is to synthesize recombinant proteins in prokaryotic cells such as *Escherichia coli* and then, lyse the cells and purify the cell extracts obtained by biochemical methods to produce recombinant proteins in a large scale.

Compared with the general method, a protein production system capable of simultaneously expressing and secreting recombinant protein in a cell is much more efficient and economical method since the need of expensive extraction and purification is reduced.

As the demand for protein products increases in clinical, industrial and academic fields, methods for efficient mass production of proteins from microorganisms have been developed. Some of the methods for mass production of proteins require that microorganisms produce target proteins in a culture medium and secret them extracellularly, in order to avoid the need for refolding proteins produced by manipulating microorganisms and purifying proteins intensively to isolate target proteins from proteins extracted from cells.

The desired proteins can be obtained without disruption of microorganisms, by engineering microorganisms to secret target proteins into a culture medium. Compared to current commercially available protein production systems using genetically engineered microorganisms, this method can minimize the contamination of protein products by intrinsic proteins of microorganisms due to no disruption of microorganism, thereby significantly reducing the cost of purification process.

[DISCLOSURE]

[TECHNICAL PROBLEM]

The present invention provides a method of improving extracellular secretion of a target protein by regulating pI of a target protein recombined with LARD3 and their whole charge, and a method of producing a target protein efficiently, by newly investigating a factor which determines secretion of a protein with a bacterial Type 1 Secretion system (T1SS).

[TECHNICAL SOLUTION]

The present inventors have found a method of mass production of a protein efficiently, and a new method of secretion and mass production of protein which can secret insoluble proteins extracellularly through bacterial T1SS (Type 1 Secretion system), as well as a method of mass production of a protein efficiently, and have completed the present invention. In addition, the present inventors have experimented to identify the differences between the proteins being capable of secretion and the proteins which are not secreted, among the proteins in which a lipase ABC transporter recognition domain (LARD3) is bound to a target protein to be secreted extracellularly.

Specifically, *Pseudomonas fluorescens* which mostly lives on the surface of plants has been consumed by humans for a long time, and therefore has been verified for its biological stability, and *Pseudomonas fluorescens* can endure various fermentation conditions under the high concentration of cell culture, and can produce a large amount of recombinant proteins. In addition, *Pseudomonas fluorescens* naturally has a number of secretion systems such as type I secretion system (T1SS) to type 6 secretion system (T6SS), and in particular, *Pseudomonas fluorescens* has the type 1 secretion system which transports heat-resistant lipase (TliA) through TliDEF of an ATP-binding cassette (ABC) carrier. Because of the applicability of *Pseudomonas fluorescens* for recombinant protein secretion, its transportation ability for some recombinant proteins has been proven and the secretion signal has been studied.

As the result of previous researches on protein mass production using *P. fluorescens,* it has been confirmed that for many proteins, when the recombinant proteins were prepared by conjugating lipase-ABC-transporter 3 (LARD3), the extracellular secretion was improved through TliDET transporter. However, most of some proteins were not secreted extracellularly and were present only in the cytoplasm, even though LARD3 was conjugated.

Accordingly, there is a need for researches on the identification of a factor determining whether a protein conjugated with LARD3 can be secreted by the ABC transporter, and a method of allowing extracellular secretion of a protein, which has not been increased even through the conjugation of LARD3, through the ABC transporter.

For the purpose, various protein genes bound to LARD3 were introduced to *P. fluorescens,* and the concentration of each protein was analyzed in supernatant and cell pellet of each culture. As a result, it has been confirmed that the pI of proteins has an important role in secretion using TliDEF that is the T1SS transporter of *P. fluorescens,* and it has been discovered that secretion of proteins with certain pI is promoted also in various T1SS transporters derived from microorganisms other than *P. fluorescens,* thereby completing the present invention.

Specifically, the present inventors used pDART plasmid vector developed in the previous research, to conveniently connect LARD3 to proteins (Ryu, J., Lee, U., Park, J., Yoo, D. H., and Ahn, J. H. (2015), A vector system for ABC transporter-mediated secretion and purification of recombinant proteins in Pseudomonas species. Appl Environ Microbiol 81, 1744-1753). The pDART plasmid has a multiple cloning site (MCS) directly followed by in-frame LARD3 gene, and the gene inserted to the multiple cloning site of pDART is expressed with LARD3 attached to its carboxyl terminus.

The LARD3 sequence is a sequence which is recognized by the ABC transporter of bacterial Type 1 Secretion System (T1SS) such as *Pseudomonas fluorescens* TliDEF, *Pseudomonas aeruginosa* AprDEF(PaAprDEF), *Dickeya dadantii PrtDEF*(DdPrtDEF), and *Escherichia coli* HlyBD+TolC, or the like, and which makes the recombinant proteins be secreted by the ABC transporter of T1SS.

In addition, pDART plasmid vector includes a Kanamycin-resistant gene for clone selection, has an origin of replication in broad host range to function as a shuttle vector between *Escherichia coli* and *P. fluorescens,* and comprises tliD, tliE, tliF genes expressing TliDEF complex.

Subsequently, the present inventors have attached an oligopeptide sequence to these proteins in order to artificially lower the pI value and add negative charge. To perform this work, the present inventors have produced two plasmids which attach aspartate polypeptide (oligo-aspartate) sequence to target proteins. After the experiment, the present inventors have produced a plasmid which attaches arginine polypeptide to target proteins, to investigate the effect when a positively charged amino acid is added to a target protein.

Lastly, the present inventors have experimented the secretion of supercharged mutants of the green fluorescent protein (GFP) developed in the previous research and have confirmed whether the supercharged mutants of the protein show a different secretion pattern from the original protein.

Type I secretion system (T1SS) means a polypeptide secretion system using an ABC transporter of bacteria, and is a chaperone-independent secretion system employing the Hly and TolC gene clusters. The secretion process is initiated by recognition of HlyA secretion signal and binding of HlyB to membrane. This signal sequence is very specific to the ABC transporter. Specifically, the HlyAB complex starts to untie coil by stimulating HlyD and TolC arrives at the outer membrane which recognizes terminal molecules or signals of HlyD. HlyD draws TolC to the inner membrane and HlyA is released outside of the outer membrane through a long-tunnel of protein channel.

The bacterial T1SS transports various molecules such as ions, drugs and proteins with various sizes (20 to 900 kDa). The secreted molecules have various sizes from small peptide colicimV (10kDa) of *Escherichia coli* to cell adhesion protein (520 kDa) of *Pseudomonas fluorescens.* The proteins characterized well are RTX toxins and lipases. Type 1 secretion also involves in secretion of non-protein substrates such as cyclin $\beta$-glucans and polysaccharides.

T1SS is mainly present in Gram negative bacteria. Bacteria having T1SS includes *Pseudomonas sp., Dickeva sp., E. coli,* or the like, and more preferably, *Pseudomonas fluorescens, Dickeya dadantii* (or *Erwinia chrysanthemi*), *Escherichia coli, Pseudomonas aeruginosa,* or the like.

Because *Pseudomonas fluorescens,* Gram-negative bacterium does not accumulate acetic acid, it has resistance to the high cell concentration caused by fermentation conditions, and it is generally non-pathogenic to humans. Thus, it is a candidate appropriate for protein production technology of proteins using secretion. In addition, *Pseudomonas fluorescens* is a Gram-negative psychrotrophic bacterium, and it has various excellent characteristics for recombinant protein production.

The present inventors have researched that a signal sequence recognized by an ABC transporter for polypeptide belonging to T1SS and a target protein are fused to secret the target protein into the culturing solution by ABC transporter of a microorganism, and as a result, have determined that the ABC transporters belonging to T1SS show the secretion efficiency of recombinant proteins proportionate to the isoelectric point (pI) of transport proteins, that is the charge property at pH 7. In other words, it has been confirmed that the negatively supercharged recombinant protein obtained by lowering pI of the target protein can increase the efficiency of secretion using the ABC transporter of T1SS, and pI has experimentally a close relation to the charge quantity of the protein (See FIG. 6).

Furthermore, the present invention has confirmed that the negatively supercharging has effect on the Type I secretion system other than TliDEF transporter of *Pseudomonas fluorescens* (See FIG. 19, FIG. 20, and FIG. 21). The T1SS means a polypeptide secretion system using an ABC transporter, and the TliDEF transporter is also a typical one of T1SS.

The present invention has isolated the genes of various T1SS transporters from microorganisms other than *Pseudomonas fluorescens,* specifically, three T1SS transporters of *Pseudomonas aeruginosa* AprDEF (*PaApr*DEF), *Dickeya dadantii* (also called *Erwinia chrysanthemi*) PrtDEF (*Dd*PrtDEF), *Escherichia coli* HlyBD+TolC (E. coli expresses the original TolC protein). The three T1SS transporters have the nucleotide sequence identity of 60%, 59% and 27% to that of TliDEF transporter of *Pseudomonas fluorescens,* respectively. It has been confirmed that the recombinant proteins to be secreted in which LARD3 signal sequence is attached are secreted through the three transporters, respectively (See FIG. 19). Accordingly, it has been confirmed that the technology for improvement in the protein secretion by employing the negatively-supercharging is not only limited to the TliDEF transporter of *Pseudomonas fluorescens,* but also can be widely applied to T1SS transporters having the nucleotide sequence identity of about 27% to TliDEF (See FIG. 20, FIG. 21, FIG. 22 and FIG. 23).

The present invention provides an expression vector for expression and extracellular secretion of a target protein in bacteria, comprising an expression cassette including a nucleotide sequence encoding a lipase ABC transporter recognition domain (LARD) and a nucleotide sequence encoding a target protein which are operably linked, where the LARD and target protein have acidic pI values and are expressed as fusion proteins secreted extracellularly.

According to an embodiment of the present invention, the expression vector may further comprise a nucleotide sequence encoding an ABC transporter of bacterial T1SS.

The term "target protein" means a target protein which can be produced at a large amount by producing biologically in bacteria and secreting extracellularly. The kind of the target protein is not particularly limited, and it may be cytokines, industrial enzymes, growth hormones, immune-related proteins, adhesive proteins, or the like. For example, it may be any one or more selected from the group consisting of Mannanase, MBP, NKC-TliA, Eg1V, GFP, thioredoxin, phospholipase A1, alkaline phosphatase, EGF, TliA, MAP, Capsid, Hsp40, M37 lipase, Cutinase, Chitinase, and CTP-TliA.

In order that pI of target protein can be adjusted to be an acidic pI of less than 7 by various methods. For example, the methods for adding acidic amino acids to the target protein, removing basic amino acids from the target protein, or substituting with other amino acid may be used, and the methods for supercharging a protein includes the manual supercharging comprising selecting amino acids which are amino acids protruding outside in the three-dimensional structure of the protein and do not affect the structure of protein, and substituting them with acidic amino acids, or the supercharging using Average Number of Neighboring Atoms Per Sidechain Atom (AvNAPSA) algorithm (1. Lawrence MS, Phillips KJ, Liu DR. Supercharging Proteins Can Impart Unusual Resilience. Journal of the American Chemical Society 2007; 129: 10110-10112.). In this case, however, it is preferable to recombine so as not to affect the structure and the function of protein.

The term "fusion protein" is a protein that is expressed in a connecting form of a nucleotide sequence encoding LARD and a nucleotide sequence encoding a target, and that has an acidic pI value and is secreted extracellularly.

According to an embodiment of the present invention, the pI value of the fusion protein may be less than 7, preferably 1 to 6, more preferably 2 to 5.5, or most preferably 3.0 to 5.5, for example 4.0 to 5.5.

When the pI value of the fusion protein is over 7, the amount of the protein secreted extracellularly through the ABC transporter of T1SS is significantly small in spite of the LARD-attached proteins, and most are present inside of cells. The protein having the pI value of the fusion protein less than 1 has very unstable structure, and therefore it is preferable to have a pI value in the range.

According to another embodiment of the present invention, the ABC transporter of T1SS may be a protein complex belonging to the T1SS transporter having the nucleotide sequence identity of 20% or more, when the nucleotide sequence identity is calculated to the parts of *Pseudomonas fluorescens* TliDEF according to the common calculation method for the nucleotide sequence identity (See FIG. 23). According to an embodiment, the T1SS transporter having the nucleotide sequence identity of 20% or more may be HasDEF of *Serratia marcescens,* CyaBDE of *Bordetella pertussis,* CvaBA+TolC of *Escherichia coli,* RsaDEF of *Caulobacter crescentus,* or the like, but not limited thereto. Preferably, it may be *Pseudomonas aeruginosa* AprDEF (PaAprDEF), *Dickeya dadantii PrtDEF* (DdPrtDEF), and *Escherichia coli* HlyBD+TolC, and more preferably, it may be *Pseudomonas fluorescens* TliDEF.

The TliDEF are a multimer of three kinds of ATP binding cassette (ABC), membrane fusion proteins (MFP), and outer membrane proteins of TliD, TliE and TliF. The ABC protein selectively recognizes a secretion domain at the C-terminal part of a target protein and hydrolyzes ATP to secret the target protein. The membrane fusion proteins are embedded in cytoplasmic membrane and connect the ABC protein and outer membrane proteins. The outer membrane proteins are positioned in the outer membrane, and span most of cell periplasm forming channels through which the target protein is secreted.

In *Pseudomonas fluorescens,* the ABC protein, membrane fusion protein and outer membrane protein are encoded by tliD, tliE and tliF, respectively, which are located in the upstream of tliA in the lipase operon. The secretion/chaperone domain at the C-terminus of tliA is defined as a lipase ABC transporter recognition domain (LARD). Until now, five fragments of LARD with different lengths have been compared functionally, and it has been confirmed that LARD3 comprising 4 RTX (repeats-in-toxin) motifs is the most effective C-terminal signal in secretion using the ABC transporter. The *Pseudomonas fluorescens* including fusion protein construct of tliDEF and LARD3 can efficiently secret the LARD3- fused protein and obtain the secreted LARD3- fused protein directly from the culture broth.

According to one embodiment of the present invention, the nucleotide sequence encoding the target protein in the expression vector may further include a nucleotide sequence encoding an acidic peptide.

The number of amino acids in the added acidic peptides is not particularly limited, but according to one embodiment of the present invention, the number of amino acids comprised of the acidic peptide may be 6 to 20, preferably 7 to 15, for example, 10. When the number of amino acids comprised of the peptide is less than 6, the pH of the fusion protein does not show sufficient acidity, and thus may not be secreted efficiently through Type 1 secretion system (T1SS).

The acidic peptide may include one or more amino acids selected from the group consisting of aspartic acid and glutamic acid, and preferably, the nucleotide sequence encoding the acidic peptide may include a nucleotide sequence encoding the amino acid sequence of SEQ ID NO: 33 (10 aspartic acids; D10). The nucleotide sequence encoding the acidic peptide may be located at the 3'-terminus or 5'-terminus of the nucleotide sequence encoding the target protein, and preferably, it may be attached to the 3'-terminus.

In addition, the vector may further comprise a nucleotide sequence encoding a linker. The linker may be one to three peptides linked in which the each peptide consists of an amino acid sequence of Gly-Gly-Gly-Gly-Ser.

According to one embodiment of the present invention, the nucleotide sequence encoding the target protein may be obtained by removing one or more of basic amino acids contained in the target protein. The basic amino acid is lysine or arginine.

According to other one embodiment of the present invention, the target protein may be a supercharged target protein, or preferably negatively supercharged target protein. The present invention can increase the extracellular secretion of the target protein, by negatively supercharging the charge of the target protein which has not been secreted outside of Gram-negative bacterial cells before.

For example, a target protein may be negatively supercharged using a manual supercharging technique, which visualizes the three-dimensional structure of target proteins using software rendering, selects amino acids that are present relatively outside of proteins and have a functional group protruding on the direction of a solvent, so as not to largely affect the structure even though being changed, and then substituting them with aspartic acid and glutamic acid when the amino acids are basic amino acids, or substituting them with lysine and arginine when the amino acids are acidic amino acids. As another example, the negatively supercharged target protein may be prepared by remodeling the protein surface using AvNAPSA (Average Neighbor Atoms per Sidechain Atom) algorithm. The protocol of AvNAPSA has been known well (WO2007/143574 A1). Specifically, the algorithm is algorithm digitizing and showing how much close each amino acid of proteins is to other atoms.

As shown in the examples, the present inventors have obtained a protein (negatively supercharged protein) sequence of which amino acids with 100 or less of AvNAPSA score (namely, amino acids which are present relatively outside of proteins and have a functional group protruding on the direction of a solvent, and therefore do not largely affect the structure even though being changed) are replaced with aspartic acid and glutamic acid according to the known protocol, have been synthesized to DNA sequence corresponding to the protein sequence and added the synthesized DNA sequence to pDART plasmid to express proteins for secretion. As a result, it has been confirmed that the efficiency of extracellular secretion of the negatively supercharged protein is significantly increased, compared to the proteins which are not negatively supercharged.

According to one embodiment of the present invention, the lipase ABC transporter recognition domain may be LARD1, LARD2 or LARD3. The LARD may mean a secretion/chaperon domain at the C-terminus of TliA in the heat-resistant lipase operon of *Pseudomonas fluorescens.* Specifically, the LARD peptide is classified into LARD1 to LARD 5 peptides by its sequence, and the LARD used in the present invention may be LARD 3, or preferably, LARD 3 peptide consisting of the amino acid sequence of SEQ ID NO: 22.

The LARD peptide including a sequence for purification being capable of functionally performing purification using hydrophobic chromatography, and the purification sequence is VLSFGADSVTLVGVLGGLWSEGVLIS (SEQ ID NO: 29) which the present inventor discloses in Korean Patent No. KR10-1677090. The proteins including the purification sequence can be easily purified using hydrophobic interaction chromatography. Accordingly, the LARD3 peptide including the purification sequence may be used for purification of a target protein.

Furthermore, the LARD peptide includes a signal sequence of functionally inducing secretion from inside of cells to outside of cells, and the secretion signal sequence is GSDGNDLIQGGKGADFIEGGKGNDTIRDNSGHNTFLFSGHFGQD (SEQ ID NO: 30) which the present inventor discloses in Korean Patent No. KR10-1677090. The proteins including the secretion signal sequence may be secreted from inside of cells to outside of cells. Among LARD, LARD 1 to LARD 3 peptides including both the secretion signal sequence and the purification sequence, may be used for secretion to outside of cells and purification of the target protein. Preferably, the secretion signal sequence may be LARD 3 peptide (SEQ ID NO: 22).

The nucleotide sequence encoding the LARD is located at the 3'-terminus of the nucleotide sequence encoding a recombinant target protein, and it may encode a protein fused at the C-terminus of the recombinant target protein. When it is fused to the C-terminus of the recombinant target protein, the C-terminal signal sequence is not hydrolyzed advantageously, on the contrary to the signal sequence of the N-terminus hydrolyzed by extracellular secretion.

[Table 1]

| Name | Amino acid sequence | SEQ ID NO |
|---|---|---|
| LARD purification sequence | VLSFGADSVTLVGVGL | 29 |
| LARD secretion signal sequence | GSDGNDLIQG GKGADFIEGG KGNDTIRDNS GHNTFLFSGH FGQD | 30 |
| LADR 1 | SIANLSTWVS HLPSAYGDGM TRVLESGFYE QMTRDSTIIV ANLSDPARAN TWVQDLNRNA EPHTGNTFII GSDGNDLIQG GKGADFIEGG KGNDTIRDNS GHNTFLFSGH FGQDRIIGYQ PTDRLVFQGA DGSTDLRDHA KAVGADTVLS FGADSVTLVG VGLGGLWSEG VLIS | 31 |
| LARD 2 | DSTIIVANLS DPARANTWVQ DLNRNAEPHT GNTFIIGSDG NDLIQGGKGA DFIEGGKGND TIRDNSGHNT FLFSGHFGQD RIIGYQPTDR LVFQGADGST DLRDHAKAVG ADTVLSFGAD SVTLVGVGLG GLWSEGVLIS | 32 |
| LARD 3 | GSDGNDLIQG GKGADFIEGG KGNDTIRDNS GHNTFLFSGH FGQDRIIGYQ PTDRLVFQGA DGSTDLRDHA KAVGADTVLS FGADSVTLVG VGLGGLWSEG VLIS | 22 |

According to an embodiment of the present invention, a cell comprising the aforementioned expression vector is provided.

Specifically, the expression vector included in the cell may be an expression vector for expressing and secreting a target protein in bacteria, characterized by including an expression cassette in which a nucleotide sequence encoding a recombinant target protein and a nucleotide sequence encoding a lipase ABC transporter recognition domain (LARD) are operably linked, and expressing secretary proteins with an acidic pI value.

The cell may further comprise an expression vector including a nucleotide sequence encoding an ABC transporter of bacterial T1SS.

The cell may be a Gram-negative bacterium, and for example, may be *Pseudomonas* sp. strains, *Dickeya* sp., *Escherichia* sp., *Xanthomonas* sp., or *Burkholderia* sp. but not limited thereto. For example, in the present invention, the extracellular secretion of the target protein is achieved by functions of the ABC transporter. However, the ABC transporter functions in a Gram-negative bacterium with double membrane, and therefore, any Gram-negative bacterium may be used in the range of the present invention without limitation.

The *Pseudomonas* sp. may include any strain belonging to *Pseudomonas* sp., but for example, it may be *Pseudomonas fluorescens, Pseudomonas fragi, Pseudomonas putida, Pseudomonas syringae, or Pseudomonas aeruginosa,* and preferably, may be *Pseudomonas fluorescens* or *Pseudomonas aeruginosa.*

When the cell is *Pseudomonas fluorescens,* the target proteins introduced to *Pseudomonas fluorescens* may bind to the C-terminal signaling sequence of TliA and be secreted extracellularly in a form of fusion protein. The intrinsic lipase and protease of *Pseudomonas fluorescens* are also secreted extracellularly by the ABC transporter. Accordingly, when target proteins are expressed using wild type *Pseudomonas fluorescens,* or a strain producing complete lipase or protease, there is a problem that even though the target proteins are secreted extracellularly, the lipase and protease are mixed as impurities so as to make the next purification process become complex, and the protease hydrolyzes the produced target proteins.

Therefore, the *Pseudomonas* sp. may be a mutant of *Pseudomonas fluorescens,* in which some regions of one or more genes selected from the group consisting of lipase gene (*tliA*) and protease gene (*prtA*) of *Pseudomonas fluorescens* are deleted, and the partial deletion of genes are deleting gene regions so as to leave fragments with at least 100bp or more of size at one or both of terminuses of the genes. The mutant strain may not produce one or more kinds of functional proteins selected from the group consisting of functional lipase and functional protease. The example of the mutant strain may be single deletion strain of lipase (mutant strain Δ*tliA*), single deletion strain of protease (mutant strain Δp*rtA*) and double deletion strain of lipase/protease (mutant strain Δ*tliA* Δ*prtA*). The contents of these mutant strains are described in detail in Korean patent publication 10-2004-0041159.

The mutant strains of *Pseudomonas fluorescens* do not produce functional protease protein, and may be achieved by all or partial deletion of the protease gene, or all or partial deletion of protease inhibitor gene (inh).

The mutant strain of *Pseudomonas fluorescens* may be, for example, the mutant strains of *Pseudomonas fluorescens* with accession number KCTC 12276BP, KCTC 12277BP, or KCTC12278BP, but not limited thereto.

According to an embodiment of the present invention, the present invention provides a method for producing target proteins in a cell, including preparing a cell transformed by the aforementioned expression vector, producing proteins to be secreted by culturing the cell, and separating or purifying the produced proteins.

The cell may further include an expression vector comprising a nucleotide sequence encoding an ABC transporter of bacterial T1SS.

The cell may be a gram negative bacterium. In the method for producing target proteins in a cell, the method for preparing a gram negative bacterial cell transformed by the expression vector may use general method of gene introduction, and for example, a recombinant vector in which a gene encoding target proteins is introduced may be introduced into the gram negative bacterium, or a gene encoding target proteins in the introduced vector may be inserted into genome by homologous recombination.

The vector may be all vectors including plasmid vector, cosmid vector, bacteriophage vector, virus vector and the like, but not limited thereto. The introduction of a vector into a gram-negative bacterium may be performed by the known methods such as electroporation, calcium phosphate ($CaPO_4$) precipitation, calcium chloride ($CaCl_2$) precipitation, PEG, dextran sulfate, lipofectamine, and the like.

In the culture of the cell, the culture conditions such as medium components, culturing temperature and culturing time, and the like may be appropriately controlled. Specifically, the culture medium may comprise all nutrients essential for growth and survival of microorganisms such as carbon source, nitrogen source, microelement components, and the like. In addition, the pH of the medium may be appropriately adjusted, and it may comprise components such as antibiotics. Moreover, expression of proteins may be induced by treating an inducer, and the kind of the treated inducer may be decided according to the vector system, and conditions such as inducer administration time and dosage and the like may be suitably controlled.

To effectively express target proteins in the wild type strain and mutant strain Δ*tliA,* 2x LB medium should be used. But, LB medium may be used for mutant strain Δ*prtA* and mutant strain Δ*tliA* Δ*prtA*, and the medium concentration may be decreased. In addition, the mutant strain Δ*tliA* Δ*prtA* has advantages that produce target proteins with the protection from PrtA hydrolysis without interruption of TliA outside of cells, can use LB medium and does not compete with lipase or protease-derived signal sequence, and thus, it makes production, secretion and purification of foreign proteins simple with the higher productivity, and thus it may be usefully used for mass production of target proteins.

The target proteins may be collected and purified by common methods, except for performing hydrophobic interaction chromatography using LARD including a purification sequence. For example, the cells collected by centrifugation may be disrupted using French press, ultrasonicator, and the like. When proteins are secreted to culture, the culture supernatant may be collected. When they are aggregated by overexpression, it may be obtained by dissolving proteins in an appropriate solution and denaturing and refolding. Oxidation and reduction systems of glutathione, dithiothreitol, β-mercaptoethanol, cystine and cystamine may be used, and a refolding agent such as urea, guanidine, arginine, and the like may be used, and some of salts may be used with the refolding agent.

As one embodiment, the method for producing target proteins in a gram negative bacterium may further comprise inserting a protease recognition site such as Factor Xa or Tobacco Etch Virus (TEV) protease, Enterokinase (EK), and the like, to cleave the acidic peptide and LARD bound to the target proteins, after isolating or purifying target proteins.

As one embodiment of the present invention, the purification of target proteins may use hydrophobic interaction. Then, the purification sequence of the present invention may be used as a purification tag. For example, the hydrophobic interaction chromatography is hydrophobic interaction chromatography using alkyl or aryl sepharose, and the alkyl group may be a methyl, ethyl, propyl or butyl group and aryl group may be phenyl group. Preferably, the alkyl group may be a methyl group. When a methyl group is used as the alkyl group, proteins comprising the purification sequence may be excellently purified, compared to using other alkyl groups.

Generally, for purification of proteins using a column, purification using His-tag are performed a lot, but the column for His-tag purification is costly, and also it is not appropriate for performing large capacity purification. In addition, NTA column is used a lot, but there is a problem in that $Ni^{2+}$ or NTA is separated if reused, and there is a limit to repeated use. On the other hand, a hydrophobic column used in hydrophobic interaction chromatography has advantages in that it is a low-cost column and is highly economical and is suitable for using in large capacity separation, and has a high reuse rate.

Other embodiment of the present invention provides a method for increasing extracellular secretion of proteins for secretion in a gram-negative bacterium, comprising inserting the aforementioned vector to a cell.

The cell may further contain an expression vector comprising a nucleotide sequence encoding an ABC transporter of bacterial Type 1 Secretion System (T1SS).

The present inventors have confirmed that the secretion rate of target proteins through the ABC transporter of bacterial Type 1 Secretion System (T1SS) is increased in negatively charged target proteins, and therefore, there is qualitative correlation between the pI of proteins and possibility of secretion by the T1SS ABC transporter.

In other words, proteins with strong acidic pI and strong negative charge are secreted by the ABC transporter of T1SS, but proteins with less negative charge and high pI are little secreted. As one embodiment, when genes are introduced to pFD10, secretion of some proteins is increased, and secretion of pBD10 is increased without reduction of expression.

According to the results of the pFD10 and pBD10 experiments and the comparison of secretion pattern of supercharged proteins performed in the following examples of the present invention, it has been confirmed that the secretion efficiency of proteins engineered for more negative charge is increased. The reason is why the energy required for overcoming membrane potential energy barrier between the cytoplasm and extracellular space is reduced.

Generally, the gram negative bacteria maintain the membrane potential of the inner membrane at about -150 mV and the cytoplasmic side is more negatively charged than the periplasm. This polarized charge distribution is maintained by various cell mechanisms including active proton transport across the membrane. The potential of the outer membrane also has a negative value, and the periplasm is charged more negatively than the extracellular space due to negatively charged membrane-derived oligosaccharide. However, due to many holes in the outer membrane, the magnitude of the outer membrane potential is commonly less than -30 mV.

Considering all these facts, secreting negatively charged proteins is generally advantageous in aspect of energy, and this affects equilibrium of the secretion reaction. The membrane potential is very powerful at the biochemical level, and has a significant effect on the change of free energy during transport through the ABC transporter. Transporting polypeptide across the inner membrane with -150 mV potential requires about 3.5 kcal/mol energy per charge which the polypeptide carries. The calculation at certain pressure, temperature and concentration is as follows.

$$\mathrm{w} = -nFV = 14.47\ n\ \mathrm{kJ/mol} = 3.5\ n\ \mathrm{kcal/mol}$$

Herein, n is the total charge of polypeptide, and F is Faraday constant. In case of secreting proteins with ten positive net charge (N=+10), w = 35 kcal/mol, and the secretion becomes that much worse. The typical value of the free energy change ($\Delta G$) of ATP hydrolysis under the concentration of living body is 11.4 kcal/mol. The model suggested for the mechanism of the ABC transporter indicates that the ABC protein acts through continuous conversion between "inward" form and "outward" form associated with ATP hydrolysis. According to this model, one of the major power sources of the ABC transporter is power of "power stroke" that occurs in this process. The negatively charged membrane potential exerts electrostatic force on the charged polypeptide, promoting (for negative charges) or even decreasing (for the positive charges) for the force exerted by this power stroke, ultimately affecting secretion equilibrium.

The term, "membrane potential" hypothesis is supported by many previous researches across various secretion types. It has been reported that a positive-charge inducing mutation on E. *coli* lipoprotein interrupts protein folding near the membrane in both prokaryotic and eukaryotic organisms, thereby reducing secretion, and in addition, it has been discovered that the process of passing through the outer membrane was stopped, when the net negative charge of the passenger domain of *E. coli* autotransporter (type Va transport system) is neutralized or reversed.

In case of TliDEF, other factor to be considered is the state of charge of TliD. TliD is an ABC protein which is a component of the inner membrane of the TliDEF transporter. This protein has a nucleotide binding domain (NBD) and a transmembrane domain (TMD) which are linked by short sequence between domains. In particular, TliD ABC protein has a very high theoretical pI particularly, around the TMD (pI 9.43) and the sequence between domains (pI 8.14).

In homology-based structure of TliD, it has been shown that the dimer of this protein has positive charge distribution inside of the channel (FIG. 9A and FIG. 9B). This predicted model was prepared using Aquifex aeolicus PrtD (PDB ID 5122) with sequence homology of 40.98% as a template. In addition, the ConSurf homologue analysis on TliD has shown that this positive charge distribution in the central part of the channel is actually evolutionally conserved (FIG. 9C and FIG. 9D, yellow circle). Moreover, there is a positively charged residue which protrudes toward the opening of the window and blocks the substrate entry window in the ADP binding state of TliD, and the ConSurf results also verify that arginine or lysine is present at this position in all homologues (FIG. 9C, black arrow). The present inventors estimate that the inner surface of the positively charged channel promotes secretion by interaction with the negative-charged residue of the cargo protein during the protein transport (FIG. 9E).

In addition, the positive charge on the inner surface of the channel and substrate entry window may push the positive-charged section of polypeptide to prevent entry into the channel and ultimately block secretion (as could be seen from the results of the following experiments of attaching arginine polypeptide). Herein, the present inventors have hypothesized that proteins unfold (at least partially) during the transport process, and this is because the hole of TliF which is expected to have a very similar structure to *E. coli* TolC (1tqq of PDB ID) has a mean inner diameter of 19.8 Å. This is clearly smaller than 20-30Å, the mean diameter of most of sphere proteins including GFP barrel of 24Å. TliF has a relatively rigid β-barrel form of transmembrane structure, and therefore is impossible to enlarge the hole during transport.

ABC transporters of other T1SS likewise have TMD whose ABC proteins are positively charged. HlyB-HlyD-TolC that is an *E. coli* haemolysin transport complex has significant positive charge distribution in TMD of ABC protein, HlyB, which is a homologue of TliD. *Dickeya dadantii* PrtD is also same. This fact intensively supports charge-dependence of T1SS ABC transporter secretion mechanism.

In conclusion, the present inventors have newly discovered that only highly acidic proteins can be transported through ABC transporters and basic or weak acidic proteins cannot be secreted through ABC transporters, and provides a method for improving secretion of target proteins extracellularly, by artificially lowering pI by attaching aspartic acid polypeptide to target proteins or negatively supercharging. In addition, a method is provided to confirm that an ABC transporter can secret the target protein through simple pI inspection, and ultimately, the range of proteins which can be efficiently produced through ABC transporter-dependent secretion can be extended by supercharging the proteins.

[ADVANTAGEOUS EFFECTS]

The present invention produces a protein for secretion with an acidic pI value, thereby providing a method of effectively secreting a target protein extracellularly through an ABC transporter of bacterial Type 1 Secretion System (T1SS). The method allows simple and efficient mass production of proteins without further purification processes.

[BRIEF DESCRIPTION OF THE DRAWINGS]

FIG. 1a and FIG. 1b confirm the secretion of selected proteins according to Example 6, and represent the western blotting images showing expression and secretion of target proteins.

FIG. 2a and FIG. 2b show the correlation between the secretion ratio of the target proteins and their isoelectric points according to Example 6. The pI values of the target proteins are calculated for the sequence including attached LARD3.

FIG. 3a and FIG. 3b are results of expressing Lunasin and derivatives of Lunasin with different length of oligo-aspartic acid tail through LARD3 attachment and confirming secretion, in order to determine the optimal length of the oligo-aspartic acid sequence in P.*fluorescens* expression and secretion system according to Example 7.

FIG. 4 shows the structure of the plasmid used in the present invention according to Example 8, and shows the structure of pDART plasmid comprising MCS. Proteins fused with tliD, tliE, tliF and LARD3 are controlled by single operon. In case of (A), as there is the LARD3 gene right behind MCS, the inserted target gene is expressed with LARD3 attached to the C-terminus. (B) shows the structure of pFD10 which is a plasmid in which D10 sequence is attached to the N-terminus. The D10 gene directly follows start codon and is located right before the MCS and LARD3. (C) shows the structure of pBD10 plasmid, which attaches D10 sequence at the C-terminus, but before LARD3. The D10 gene is located between the MCS and LARD3.

FIG. 5 shows the result of detecting by western blotting and the lipase activity with a measurement medium, after adding 10 aspartic acids (D10) to the N-terminus (FD10) and C-terminus (BD10) of two kinds of TliA lipases (NKC-TliA, CTP-TliA) in which NKC and CTP sequences are attached, respectively, and expressing through pDART plasmid, according to Example 9.

FIG. 6 shows the result of western blotting detection, after adding 10 aspartic acids (D10) to the N-terminus (FD10) and C-terminus (BD10) of green fluorescent protein (GFP), mannanase, maltose binding protein (MBP) and Thioredoxin, and expressing through pDART plasmid, according to Example 10.

FIG. 7 shows the result of detecting with western blotting and lipase activity medium, after adding 10 aspartic acids (D10) or 10 arginines (R10), respectively, to the C-terminus of TliA lipase and green fluorescent protein (GFP) according to Example 11.

FIG. 8 shows the result of adding green fluorescent protein (GFP) supercharged by AvNAPSA method to pDART and expressing it, and detecting it by western blotting, according to Example 12.

FIG. 9 shows the charge distribution of TliD structure which is ABC protein of TliDEF complex according to Example 5. The parts indicated by circles in A, B, C of FIG. 9 show positively charged parts, and the parts indicated by the circle in D of FIG. 9 show pores inside of the transporter, and the white arrow inside of the circle represents the relatively negative atom and the black arrow represents the relatively positive atom.

FIG. 10 shows the comparison of secretion of TliA, CTP-TliA and NKC-TliA according to Example 9, and FIG. 10a is the result of enzyme plate analysis of TliA, CTP-TliA and NKC-TliA, and FIG. 10b shows the western blotting result of TliA, CTP-TliA and NKC-TliA.

FIG. 11 shows the relation between the protein pI and charge at pH 7.0 according to Example 5.

FIG. 12 shows the result of predicting the structure of TliD according to Example 5.

FIG. 13 shows the result of prediction of transmembrane helices of modeled TliD according to Example 5. The rectangular box part corresponds to the transmembrane part predicted by the server.

FIG. 14 shows the result of ConSurf homologue conservation analysis of modeled TliD according to Example 5. The dark black parts are well conserved parts, and the lighter the color is, the less conserved it is.

FIG. 15 shows the protein secretion in pDAR-TliA, -NKC (-), NKC-L1, -NKC-L2, NKC-L3, -NKC-TliA according to Example 13. (A) Western blotting of TliA. (B) shows the result of enzyme plate analysis of TliA in different plasmids.

FIG. 16 shows the result of analysis of secretion of -10SAV, wtSAV, + 13SAV and 2-10GST, wtGST, + 19GST according to Example 14 (SAV: streptavidin / GST: glutathione S-transferase).

FIG. 17 shows the result of inserting and expressing glutathione S-transferase (GST) supercharged by replacing protruding amino acids with aspartic acid or arginine and streptavidin (SAv) to pDART, and detecting with western blotting, while looking at the structure without using AvNAPSA (Average Number of Neighboring Atoms Per Sidechain Atom) method according to Example 14.

FIG. 18 shows the result of highly negatively charging $MelC_2$ tyrosinase, cutinase (Cuti), chitinase (Chi) and M37 lipase by AvNAPSA method, and then adding highly negatively charged protein (red) and non-supercharged natural protein corresponding thereto (black) to pDART plasmid, respectively, and expressing them, and detecting by western blotting, according to Example 15.

FIG. 19 shows the experimental result of measuring the degree of protein secretion in the enzyme activity measurement medium through the color change of the colony peripheral medium, after simultaneously expressing TliA protein (original substrate of TliDEF transporter) and T1SS transporters isolated from different 3 kinds of bacteria, according to Example 16.

FIG. 20 shows the experimental result of measuring the degree of protein secretion in the enzyme activity measurement medium through the color change of the colony peripheral medium, by suspending the LARD3 signal sequence to cutinase protein (Cuti) and highly negatively charged cutinase protein (Cuti(-)) and then expressing them together with different 3 kinds of TISS transporter proteins in E. Coli according to Example 17.

FIG. 21 shows the experimental result of detecting the protein concentration inside and outside the cell by western blotting, after attaching the LARD3 signal sequence to cutinase protein (Cuti) and highly negatively charged cutinase protein (Cuti(-)) and then expressing them together with different 3 kinds of T1SS transporter proteins in E. Coli and liquid culturing them, according to Example 18.

FIG. 22 shows the experimental result of detecting the protein concentration inside and outside of the cell by western blotting, after attaching LARD3 signal sequence to M37 lipase protein (M37) and highly negatively charged M37 lipase protein (M37(-)), and then expressing them with different 3 kinds of T1SS transporter proteins in *E. Coli* by performing liquid culture according to Example 19.

FIG. 23 shows the sequence identity between TliDET transporter and various T1SS transporters and the proportion of the portion of similar sequence in the full sequence.

[DETAILED DESCRIPTION OF THE EMBODIMENTS]

Hereinafter, the present invention will be described in detail by examples. However, the following examples are intended to illustrate the present invention only, but the present invention is not limited by the following examples.

## [Example 1] Bacterial strains and growth media

Plasmid construction and gene cloning were performed in E. *coli* XL1-BLUE. Protein expression and secretion were observed in the *P.fluorescens* Δ*dliA* Δ*prtA* strain, which is a double-deletion derivative of P. *fluorescens* SIK-W1 (Son, M., Moon, Y., Oh, M. J., Han, S. B., Park, K. H., Kim, J. G., and Ahn, J. H. (2012) Lipase and protease double-deletion mutant of Pseudomonas fluorescens suitable for extracellular protein production. Appl Environ Microbiol 78, 8454-8462). Microorganisms were cultured in lysogeny broth (LB) with 30 μg/ml kanamycin. An enzyme plate assay for the target genes with lipase activity (TliA, NKC-TliA, and CTP-TliA) was prepared with LB agar media containing blender-mixed 0.5% colloidal glyceryl tributyrate. E. *coli* and *P. fluorescens* were incubated at 37°C and 25 °C, respectively. *E. coli* transformation was performed following the standard heat-shock method, and *P. fluorescens* transformation was performed via electroporation at 2.5 kV, 125 Ω, and 50 μF, with electrocompetent cells prepared using a standard electroporation protocol (Ausubel, M. F. (2014) Escherichia coli, Plasmids, and Bacteriophages. in Current Protocols in Molecular Biology, John Wiley & Sons, Inc. pp). The transformed *P. fluorescens* were cultured in test tubes with 5 ml of liquid LB media, including 60 μg/ml kanamycin, and were incubated at 25 °C in a 180 rpm shaking incubator until the stationary phase was reached. The proteins were analyzed for both expression and secretion by seeding the transformed cells in liquid LB or streaking them on the solid-plate activity assay.

**[Example 2] Plasmid vector constructions**

Plasmid pDART was used for the secretory production of different proteins of the present inventors (Ryu, J., Lee, U., Park, J., Yoo, D. H., and Ahn, J. H. (2015) A vector system for ABC transporter-mediated secretion and purification of recombinant proteins in Pseudomonas species. Appl Environ Microbiol 81, 1744-1753). Plasmid vectors pFD10 and pBD10 were derivatives of pDART, constructed by adding codons for 10 aspartic acid residues to the target proteins in either the upstream or downstream position of MCS. The DNA sequence for 10 aspartic acids was amplified via PCR using synthesized Glycine max lunasin gene (Galvez, A. F., Chen, N., Macasieb, J., and de Lumen, B. O. (2001) Chemopreventive Property of a Soybean Peptide (Lunasin) That Binds to Deacetylated Histones and Inhibits Acetylation. Cancer Research 61, 7473-7478) as a template. Two different PCR products were obtained, each for pFD10 and pBD10. One or two arbitrary bases are inserted upstream or downstream of the primers to keep the translation in-frame, causing a slight size and pI difference between the pFD10- and pBD10-inserted proteins.

Then, recombining the PCR product with pDART to construct pFD10 and pBD10 was accomplished with an In-Fusion cloning kit (Clontech In-Fusion HD cloning plus CE). To linearize pDART, it was digested with either XbaI (pFD10 construction) or SacI (pBD10). Then, the linearized pDART and the corresponding PCR products were digested with In-Fusion 3'-to-5'-exodeoxyribonuclease and re-ligated following the standard protocol of the In-Fusion kit. Ligation of these DNA fragments with complementary ~15-base 5'-overhangs resulted in pFD10 and pBD10 plasmid, ready for target gene insertions. pDART, pFD10, and pBD10 sequences near their MCSs are provided in Table 2.

The amino acid sequences underlined in the following Table 2 represent LARD3 signal sequences, and the bold "IEGR" is a residue that connects the target protein and LARD3 signal sequence, and is a part that Factor Xa recognizes and cleaves.

The target protein may be further purified from Factor Xa and LARD3 by purification tag such as His-tag.

The description of each part of the sequence of the following Table 2 was disclosed in FIG. 19a to FIG. 19g. FIG. 19a to FIG. 19f represent the total sequence of target proteins in FASTA format, and FIG. 19g represents color codes for indicating enzyme sites and polypeptide characteristics.

[Table 2]

| Full sequences of the target proteins, in FASTA formats | | SEQ ID NO |
|---|---|---|
| TliA, wild type (as a reference) | MGVFDYKNLGTEASKTLFADATAITLYTYHNLDNGF AVGYQQHGLGLGLPATLVGALLGSTDSQGVIPGIPWN PDSEKAALDAVHAAGWTPISASALGYGGKVDARGTF FGEKAGYTTAQAEVLGKYDDAGKLLEIGIGFRGTSGP RESLITDSIGDLVSDLLAALGPKDYAKNYAGEAFGGL LKTVADYAGAHGLSGKDVLVSGHSLGGLAVNSMAD LSTSKWAGFYKDANYLAYASPTQSAGDKVLNIGYEN DPVFRALDGSTFNLSSLGVHDKAHESTTDNIVSFNDH YASTLWNVLPFSIANLSTWVSHLPSAYGDGMTRVLES GFYEQMTRDSTIIVANLSDPARANTWVQDLNRNAEP HTGNTFIIGSDGNDLIQGGKGADFIEGGKGNDTIRDNS GHNTFLFSGHFGQDRIIGYQPTDRLVFQGADGSTDLR DHAKAVGADTVLSFGADSVTLVGVGLGGLWSEGVLI S | 1 |

(continued)

| Full sequences of the target proteins, in FASTA formats | | SEQ ID NO |
|---|---|---|
| TliA, expressed in pDART plasmid (this is used for computational analysis) | MSRMGVFDYKNLGTEASKTLFADATAITLYTYHNLD<br>NGFAVGYQQHGLGLGLPATLVGALLGSTDSQGVIPGI<br>PWNPDSEKAALDAVHAAGWTPISASALGYGGKVDA<br>RGTFFGEKAGYTTAQAEVLGKYDDAGKLLEIGIGFRG<br>TSGPRESLITDSIGDLVSDLLAALGPKDYAKNYAGEAF<br>GGLLKTVADYAGAHGLSGKDVLVSGHSLGGLAVNS<br>MADLSTSKWAGFYKDANYLAYASPTQSAGDKVLNIG<br>YENDPVFRALDGSTFNLSSLGVHDKAHESTTDNIVSF<br>NDHYASTLWNVLPFSIANLSTWVSHLPSAYGDGMTR<br>VLESGFYEQMTRDSTIIVANLSDPARANTWVQDLNRN<br>AEPHTGNTFIIGSDGNDLIQGGKGADFIEGGKGNDTIR<br>DNSGHNTFLFSGHFGQDRIIGYQPTDRLVFQGADGST<br>DLRDHAKAVGADTVLSFGADSVTLVGVGLGGLWSE<br>GVLISEL**IEGR**GSDGNDLIQGGKGADFIEGGKGNDTIR<br>DNSGHNTFLFSGHFGQDRIIGYQPTDRLVFQGADGST<br>DLRDHAKAVGADTVLSFGADSVTLVGVGLGGLWSE<br>GVLIS | 2 |
| NKC-TliA: NKC is marked cyan | MSRHMGTAPKAMKLLKKLLKLQKKGIGSMGVFDYK<br>NLGTEASKTLFADATAITLYTYHNLDNGFAVGYQQH<br>GLGLGLPATLVGALLGSTDSQGVIPGIPWNPDSEKAA<br>LDAVHAAGWTPISASALGYGGKVDARGTFFGEKAGY<br>TTAQAEVLGKYDDAGKLLEIGIGFRGTSGPRESLITDSI<br>GDLVSDLLAALGPKDYAKNYAGEAFGGLLKTVADY<br>AGAHGLSGKDVLVSGHSLGGLAVNSMADLSTSKWA<br>GFYKDANYLAYASPTQSAGDKVLNIGYENDPVFRAL<br>DGSTFNLSSLGVHDKAHESTTDNIVSFNDHYASTLWN<br>VLPFSIANLSTWVSHLPSAYGDGMTRVLESGFYEQMT<br>RDSTIIVANLSDPARANTWVQDLNRNAEPHTGNTFIIG<br>SDGNDLIQGGKGADFIEGGKGNDTIRDNSGHNTFLFS<br>GHFGQDRIIGYQPTDRLVFQGADGSTDLRDHAKAVG<br>ADTVLSFGADSVTLVGVGLGGLWSEGVLISEL**IEGR**G<br>SDGNDLIQGGKGADFIEGGKGNDTIRDNSGHNTFLFS<br>GHFGQDRIIGYQPTDRLVFQGADGSTDLRDHAKAVG<br>ADTVLSFGADSVTLVGVGLGGLWSEGVLIS | 3 |
| CTP-TliA: CTP is marked cyan | MSRMRGSHHHHHHGMASMTGGQQMGRDLYDDDD<br>KDRWGSMYGRRARRRRRRSMAGTGGMGVFDYKNL<br>GTEASKTLFADATAITLYTYHNLDNGFAVGYQQHGL<br>GLGLPATLVGALLGSTDSQGVIPGIPWNPDSEKAALD<br>AVHAAGWTPISASALGYGGKVDARGTFFGEKAGYTT<br>AQAEVLGKYDDAGKLLEIGIGFRGTSGPRESLITDSIG<br>DLVSDLLAALGPKDYAKNYAGEAFGGLLKTVADYA<br>GAHGLSGKDVLVSGHSLGGLAVNSMADLSTSKWAG | 4 |

(continued)

| Full sequences of the target proteins, in FASTA formats | | SEQ ID NO |
|---|---|---|
| | FYKDANYLAYASPTQSAGDKVLNIGYENDPVFRALD<br>GSTFNLSSLGVHDKAHESTTDNIVSFNDHYASTLWNV<br>LPFSIANLSTWVSHLPSAYGDGMTRVLESGFYEQMTR<br>DSTIIVANLSDPARANTWVQDLNRNAEPHTGNTFIIGS<br>DGNDLIQGGKGADFIEGGKGNDTIRDNSGHNTFLFSG<br>HFGQDRIIGYQPTDRLVFQGADGSTDLRDHAKAVGA<br>DTVLSFGADSVTLVGVGLGGLWSEGVLISEL**IEGR**GS<br>DGNDLIQGGKGADFIEGGKGNDTIRDNSGHNTFLFSG<br>HFGQDRIIGYQPTDRLVFQGADGSTDLRDHAKAVGA<br>DTVLSFGADSVTLVGVGLGGLWSEGVLIS | |
| Mannanase (Mann) | MSRHHHHHHTVSPVNPNAQQTTKAVMNWLAHLPNR<br>TENRVLSGAFGGYSHDTFSMAEADRIRSATGQSPAIY<br>GCDYARGWLETANIEDSIDVSCNSDLMSYWKNDGIP<br>QISLHLANPAFQSGHFKTPITNDQYKKILDSSTAEGKR<br>LNTMLSKIADGLQELENQGVPVLFRPLHEMNGERFW<br>WGLTSYNQKDNERISLYKQLYKKIYHYMTDTRGLDH<br>LIWVYSPDANRDFKTDFYPGASYVDIVGLDAYFQDA<br>YSINGYDQLTALNKPFAFTEVGPQTANGSFDYSLFINA<br>IKHRYPKTIYFLAWNDEWSPAVNKGASALYHDSWTL<br>NKGEIWNGDSLTPIVEEL**IEGR**GSDGNDLIQGGKGAD<br>FIEGGKGNDTIRDNSGHNTFLFSGHFGQDRIIGYQPTD<br>RLVFQGADGSTDLRDHAKAVGADTVLSFGADSVTLV<br>GVGLGGLWSEGVLIS | 5 |
| Mussel adhesion protein (MAP): used SpeI - SacI insertion | M**SS**MRGSHHHHHHGSASAKPSYPPTYKAKPSYPPTY<br>KAKPSYPPTYKGCSSEEYKGGYYPGNSNHYHSGGSY<br>HGSGYHGGYKGKYYGKAKKYYYKYKNSGKYKYLK<br>KARKYHRKGYKKYYGGSSEFAKPSYPPTYKAKPSYP<br>PTYKAKPSYPPTYKEL**IEGR**GSDGNDLIQGGKGADFI<br>EGGKGNDTIRDNSGHNTFLFSGHFGQDRIIGYQPTDRL<br>VFQGADGSTDLRDHAKAVGADTVLSFGADSVTLVGV<br>GLGGLWSEGVLIS | 6 |

(continued)

| Full sequences of the target proteins, in FASTA formats | | SEQ ID NO |
|---|---|---|
| Maltose binding protein (MBP) | MSRKIEEGKLVIWINGDKGYNGLAEVGKKFEKDTGIK<br>VTVEHPDKLEEKFPQVAATGDGPDIIFWAHDRFGGYA<br>QSGLLAEITPDKAFQDKLYPFTWDAVRYNGKLIAYPI<br>AVEALSLIYNKDLLPNPPKTWEEIPALDKELKAKGKS<br>ALMFNLQEPYFTWPLIAADGGYAFKYENGKYDIKDV<br>GVDNAGAKAGLTFLVDLIKNKHMNADTDYSIAEAAF<br>NKGETAMTINGPWAWSNIDTSKVNYGVTVLPTFKGQ<br>PSKPFVGVLSAGINAASPNKELAKEFLENYLLTDEGLE<br>AVNKDKPLGAVALKSYEEELAKDPRIAATMENAQKG<br>EIMPNIPQMSAFWYAVRTAVINAASGRQTVDEALKD<br>AQTRITKEL**IEGR**GSDGNDLIQGGKGADFIEGGKGND<br>TIRDNSGHNTFLFSGHFGQDRIIGYQPTDRLVFQGADG<br>STDLRDHAKAVGADTVLSFGADSVTLVGVGLGGLWS<br>EGVLIS | 7 |
| Thioredoxin (Trx) | MSRMLHQQRNQHARLIPVELYMSDKIIHLTDDSFDTD<br>VLKADGAILVDFWAEWCGPCKMIAPILDEIADEYQG<br>KLTVAKLNIDQNPGTAPKYGIRGIPTLLLFKNGEVAAT<br>KVGALSKGQLKEFLDANLAEL**IEGR**GSDGNDLIQGG<br>KGADFIEGGKGNDTIRDNSGHNTFLFSGHFGQDRIIGY<br>QPTDRLVFQGADGSTDLRDHAKAVGADTVLSFGADS<br>VTLVGVGLGGLWSEGVLIS | 8 |
| Cutinase (Cuti) | MSRHHHHHHAPTSNPAQELEARQLGRTTRDDLINGN<br>SASCADVIFIYARGSTETGNLGTLGPSIASNLESAFGK<br>DGVWIQGVGGAYRATLGDNALPRGTSSAAIREMLGL<br>FQQANTKCPDATLIAGGYSQGAALAAASIEDLDSAIR<br>DKIAGTVLFGYTKNLQNRGRIPNYPADRTKVFCNTGD<br>LVCTGSLIVAAPHLAYGPDARGPAPEFLIEKVRAVRG<br>SALEEL**IEGR**GSDGNDLIQGGKGADFIEGGKGNDTIR<br>DNSGHNTFLFSGHFGQDRIIGYQPTDRLVFQGADGST<br>DLRDHAKAVGADTVLSFGADSVTLVGVGLGGLWSE<br>GVLIS | 9 |

(continued)

| Full sequences of the target proteins, in FASTA formats | | SEQ ID NO |
|---|---|---|
| Chitinase (Chi) | MSRHHHHHHANSPKQSQKIVGYFPSWGVYGRNYQV<br>ADIDASKLTHLNYAFADICWNGKHGNPSTHPDNPNK<br>QTWNCKESGVPLQNKEVPNGTLVLGEPWADVTKSYP<br>GSGTTWEDCDKYARCGNFGELKRLKAKYPHLKTIISV<br>GGWTWSNRFSDMAADEKTRKVFAESTVAFLRAYGF<br>DGVDLDWEYPGVETIPGGSYRPEDKQNFTLLLQDVR<br>NALNKAGAEDGKQYLLTIASGASRRYADHTELKKISQ<br>ILDWINIMTYDFHGGWEATSNHNAALYKDPNDPAAN<br>TNFYVDGAINVYTNEGVPVDKLVLGVPFYGRGWKSC<br>GKENNGQYQPCKPGSDGKLASKGTWDDYSTGDTGV<br>YDYGDLAANYVNKNGFVRYWNDTAKVPYLYNATT<br>GTFISYDDNESMKYKTDSIKTKGLSGAMFWELSGDCR<br>TSPKYSCSGPKLLDTLVKELLGGPINQKDTEPPTNVKN<br>IVVTNKNSNSVQLNWTASTDNVGVTEYEITAGEEKW<br>STTTNSITIKNLKPNTEYKFSIIAKDAAGNKSQPTALTV<br>KTDEANMTPPDGNGTATFSVTSNWGSGYNFSIIIKNN<br>GTNPIKNWKLEFDYSGNLTQVWDSKISSKTNNHYVIT<br>NAGWNGEIPPGGSITIGGAGTGNPAELLNAVISENEL**I**<br>**EGR**GSDGNDLIQGGKGADFIEGGKGNDTIRDNSGHN<br>TFLFSGHFGQDRIIGYQPTDRLVFQGADGSTDLRDHA<br>KAVGADTVLSFGADSVTLVGVGLGGLWSEGVLIS | 10 |
| M37 lipase **(M37)** | MSRHMSYTKEQLMLAFSYMSYYGITHTGSAKKNAEL<br>ILKKMKEALKTWKPFQEDDWEVVWGPAVYTMPFTIF<br>NDAMMYVIQKKGAEGEYVIAIRGTNPVSISDWLFNDF<br>MVSAMKKWPYASVEGRILKISESTSYGLKTLQKLKPK<br>SHIPGENKTILQFLNEKIGPEGKAKICVTGHSKGGALS<br>STLALWLKDIQGVKLSQNIDISTIPFAGPTAGNADFAD<br>YFDDCLGDQCTRIANSLDIVPYAWNTNSLKKLKSIYIS<br>EQASVKPLLYQRALIRAMIAETKGKKYKQIKAETPPL<br>EGNINPILIEYLVQAAYQHVVGYPELMGMMDDIPLTD<br>IFEDAIAGLLLEHHHHHHGTASEL**IEGR**GSDGNDLIQG<br>GKGADFIEGGKGNDTIRDNSGHNTFLFSGHFGQDRIIG<br>YQPTDRLVFQGADGSTDLRDHAKAVGADTVLSFGAD<br>SVTLVGVGLGGLWSEGVLIS | 11 |

(continued)

| Full sequences of the target proteins, in FASTA formats | | SEQ ID NO |
|---|---|---|
| **Capsid (Cap),** *Chaetoceros salsugineum* nulear inclusion virus (CsNIV | MSRMARKKSTPRRRKAVKRRRTVRRRQSPKARVRST<br>TTKAKRRISPSGSGSQHLTVRKQPFSNATSQPKILDGA<br>LTSSLSRRLQNVIGLTNGNGGLGTEIMHIFFAPTLGIPL<br>IAMNSAEGVALRPSSSADPFFIGFPGQTIKFDYVSSGTT<br>PPATGNLVTFSNECGFSKWRIVSQGLRMELANSDEEN<br>DGWFEAVRFNWRNNPADICFTPIDGTLGGAKTTDFA<br>VAPSPVGMYALKDMAMVEQPGYTTGLLKDLKNHEF<br>MLHPQSTTHDPIILEQSYEGTIGTTAADDMYYSVTSEV<br>FELGNTVRGNTMKNSLVDNNMDWIYLRLHCRTNNG<br>TTSNGSKLIVNAIQNLEVSFNPSSDFAAFQTINKMHPQ<br>QKKVDDQLNNSAEASNKRQKTGGGEL**IEGR**GSDGN<br>DLIQGGKGADFIEGGKGNDTIRDNSGHNTFLFSGHFG<br>QDRIIGYQPTDRLVFQGADGSTDLRDHAKAVGADTV<br>LSFGADSVTLVGVGLGGLWSEGVLIS | 12 |
| DnaJ (Hsp40) | MSRMAKQDYYEILGVSKTAEEREIRKAYKRLAMKYH<br>PDRNQGDKEAEAKFKEIKEAYEVLTDSQKRAAYDQY<br>GHAAFEQGGMGGGGFGGGADFSDIFGDVFGDIFGGG<br>RGRQRAARGADLRYNMELTLEEAVRGVTKEIRIPTLE<br>ECDVCHGSGAKPGTQPQTCPTCHGSGQVQMRQGFFA<br>VQQTCPHCQGRGTLIKDPCNKCHGHGRVERSKTLSV<br>KIPAGVDTGDRIRLAGEGEAGEHGAPAGDLYVQVQV<br>KQHPIFEREGNNLYCEVPINFAMAALGGEIEVPTLDGR<br>VKLKVPGETQTGKLFRMRGKGVKSVRGGAQGDLLC<br>RVVVETPVGLNERQKQLLQELQESFGGPTGEHNSPRS<br>KSFFDGVKKFFDDLTRGTASEL**IEGR**GSDGNDLIQGG<br>KGADFIEGGKGNDTIRDNSGHNTFLFSGHFGQDRIIGY<br>QPTDRLVFQGADGSTDLRDHAKAVGADTVLSFGADS<br>VTLVGVGLGGLWSEGVLIS | 13 |
| Endo-1,4-β-glucanase V (Eg15) | MSRHHHHHHYKATTTRYYDGQEGACGCGSSSGAFP<br>WQLGIGNGVYTAAGSQALFDTAGASWCGAGCGKCY<br>QLTSTGQAPCSSCGTGGAAGQSIIVMVTNLCPNNGNA<br>QWCPVVGGTNQYGYSYHFDIMAQNEIFGDNVVVDFE<br>PIACPGQAASDWGTCLCVGQQETDPTPVLGNDTGSTP<br>PGSSPPATSSSPPSGGGQQTLYGQCGGAGWTGPTTCQ<br>APGTCKVQNQWYSQCLPGTASEL**IEGR**GSDGNDLIQ<br>GGKGADFIEGGKGNDTIRDNSGHNTFLFSGHFGQDRII<br>GYQPTDRLVFQGADGSTDLRDHAKAVGADTVLSFGA<br>DSVTLVGVGLGGLWSEGVLIS | 14 |

(continued)

| Full sequences of the target proteins, in FASTA formats | | SEQ ID NO |
|---|---|---|
| Green fluorescent protein (GFP) | MSRMSKGEELFTGVVPILVELDGDVNGHKFSVSGEGE<br>GDATYGKLTLKFICTTGKLPVPWPTLVTTFSYGVQCF<br>SRYPDHMKRHDFFKSAMPEGYVQERTISFKDDGNYK<br>TRAEVKFEGDTLVNRIELKGIDFKEDGNILGHKLEYN<br>YNSHNVYITADKQKNGIKANFKIRHNIEDGSVQLADH<br>YQQNTPIGDGPVLLPDNHYLSTQSALSKDPNEKRDH<br>MVLLEFVTAAGITHGMDEL**IEGR**GSDGNDLIQGGKG<br>ADFIEGGKGNDTIRDNSGHNTFLFSGHFGQDRIIGYQP<br>TDRLVFQGADGSTDLRDHAKAVGADTVLSFGADSVT<br>LVGVGLGGLWSEGVLIS | 15 |
| -30 Negatively supercharged GFP (GFP-(-30)) | MSRMGHHHHHHGGASKGEELFDGVVPILVELDGDV<br>NGHEFSVRGEGEGDATEGELTLKFICTTGELPVPWPT<br>LVTTLTYGVQCFSDYPDHMDQHDFFKSAMPEGYVQE<br>RTISFKDDGTYKTRAEVKFEGDTLVNRIELKGIDFKED<br>GNILGHKLEYNFNSHDVYITADKQENGIKAEFEIRHN<br>VEDGSVQLADHYQQNTPIGDGPVLLPDDHYLSTESAL<br>SKDPNEDRDHMVLLEFVTAAGIDHGMDELYKEL**IEG**<br>**R**GSDGNDLIQGGKGADFIEGGKGNDTIRDNSGHNTFL<br>FSGHFGQDRIIGYQPTDRLVFQGADGSTDLRDHAKAV<br>GADTVLSFGADSVTLVGVGLGGLWSEGVLIS | 16 |
| +36 Positively supercharged GFP (GFP-(+36)) | MSRMGHHHHHHGGASKGERLFRGKVPILVELKGDV<br>NGHKFSVRGKGKGDATRGKLTLKFICTTGKLPVPWP<br>TLVTTLTYGVQCFSRYPKHMKRHDFFKSAMPKGYVQ<br>ERTISFKKDGKYKTRAEVKFEGRTLVNRIKLKGRDFK<br>EKGNILGHKLRYNFNSHKVYITADKRKNGIKAKFKIR<br>HNVKDGSVQLADHYQQNTPIGRGPVLLPRNHYLSTR<br>SKLSKDPKEKRDHMVLLEFVTAAGIKHGRDERYKEL**I**<br>**EGR**GSDGNDLIQGGKGADFIEGGKGNDTIRDNSGHN<br>TFLFSGHFGQDRIIGYQPTDRLVFQGADGSTDLRDHA<br>KAVGADTVLSFGADSVTLVGVGLGGLWSEGVLIS | 17 |
| Epidermal growth factor (EGF) | MNSDSECPLSHDGYCLHDGVCMYIEALDKYACNCVV<br>GYIGERCQYRDLKWWELRSR**IEGR**GSDGNDLIQGGK<br>GADFIEGGKGNDTIRDNSGHNTFLFSGHFGQDRIIGYQ<br>PTDRLVFQGADGSTDLRDHAKAVGADTVLSFGADSV<br>TLVGVGLGGLWSEGVLIS | 18 |

(continued)

| Full sequences of the target proteins, in FASTA formats | | SEQ ID NO |
|---|---|---|
| Alkaline phosphatase (AP) | MSSMPVLENRAAQGDITAPGGARRLTGDQTAALRDSLSDKPAKNIILLIGDGMGDSEITAARNYAEGAGGFFKGIDALPLTGQYTHYALNKKTGKPDYVTDSAASATAWSTGVKTYNGALGVDIHEKDHPTILEMAKAAGLATGNVSTAELQDATPAALVAHVTSRKCYGPSATSEKCPGNALEKGGKGSITEQLLNARADVTLGGGAKTFAETATAGEWQGKTLREQAQARGYQLVSDAASLNSVTEANQQKPLLGLFADGNMPVRWLGPKATYHGNIDKPAVTCTPNPQRNDSVPTLAQMTDKAIELLSKNEKGFFLQVEGASIDKQDHAANPCGQIGETVDLDEAVQRALEFAKKEGNTLVIVTADHAHASQIVAPDTKAPGLTQALNTKDGAVMVMSYGNSEEDSQEHTGSQLRIAAYGPHAANVVGLTDQTDLFYTMKAALGLKEL**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 19 |
| Phospholipase A1 (PLA1) | MSMSLSFTSAIAPAAIQPPMVRTQPEPLSSSQPVEASATKAPVATLSQNSLNAQSLLNTLVSEISAAAPAAANQGVTRGQQPQKGDYTLALLAKDVYSTGSQGVEGFNRLSADALLGAGIDPASLQDAASGFQAGIYTDNQQYVLAFAGTNDMRDWLSNVRQATGYDDVQYNQAVSLAKSAKAAFGDALVIAGHSLGGGLAATAALATGTVAVTFNAAGVSDYTLNRMGIDPAAAKQDAQAGGIRRYSEQYD | 20 |
| | MLTGTQESTSLIPDAIGHKITLANNDTLSGIDDWRPSKHLDRSLTAHGIDKVISSMAEQKPWEAMANAHHHHHHGTASEL**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | |

[Table 3]

| Color code for enzyme sites and polypeptide features >Multiple doing site (MCS): XbaI: tctaga, SR NdeI: catatg, HM KpnI: ggtacc, GT NheI: gctagc, AS SacI: gagctc, EL | | |
|---|---|---|
| **Factor Xa** | **IEGR** | 21 |
| **LARD3 signal peptide** | GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 22 |
| **pDART Translation Structure** | MSRHMGTASEL**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 23 |

(continued)

| Color code for enzyme sites and polypeptide features >Multiple doing site (MCS): XbaI: tctaga, SR NdeI: catatg, HM KpnI: ggtacc, GT NheI: gctagc, AS SacI: gagctc, EL | | |
|---|---|---|
| **pFD10 Translation Structure** | MSSDDDDDDDDDDSRHMGTASEL**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 24 |
| **pBD10 Translation Structure** | MSRHMGTASELDDDDDDDDDD**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 25 |
| **pBE10 Translation Structure** | MSRHMGTASELEEEEEEEEEEG**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 26 |
| **pBH10 Translation Structure** | MSRHMGTASELHHHHHHHHHHG**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 27 |
| **pBR10 Translation Structure** | MSRHMGTASELRRRRRRRRRRG**IEGR**GSDGNDLIQGGKGADFIEGGKGNDTIRDNSGHNTFLFSGHFGQDRIIGYQPTDRLVFQGADGSTDLRDHAKAVGADTVLSFGADSVTLVGVGLGGLWSEGVLIS | 28 |

**[Example 3] Construction of plasmids with inserted target genes**

Thirteen target genes were selected for pDART insertion. The genes were amplified with PCR from extracted genomic DNA samples (TliA, MBP, Trx, and Hsp40), total cDNA (EglV), synthesized DNA products (NKC-TliA, CTP-TliA, MAP, lunasin, lunasin derivatives, GFP, and supercharged GFPs), or plasmids (other proteins), or the like.

Their N-terminal signal peptides were detected with the SignalP 4.1 web-based prediction algorithm (http://www.cbs.dtu.dk/services/SignalP/) (Petersen, T. N., Brunak, S., von Heijne, G., and Nielsen, H. (2011) SignalP 4.0: discriminating signal peptides from transmembrane regions. Nature methods 8, 785-786) and were excluded from cloning and expression processes. For synthetic genes, the codons were optimized for either E. coli expression (supercharged GFPs) or *P. fluorescens* expression (TliA derivatives).

The lunasin gene was synthesized and amplified with PCR for pDART insertion. With various primers, we also synthesized its variations with differing lengths of Asp polypeptide tail at their C terminus such as lunasin-D0, lunasin-D5, lunasin-D15, and lunasin-D20 (FIG. 3B). NKC-TliA and CTP-TliA are derivatives of TliA. NKC is an antibiotic polypeptide developed previously, and CTP is a cytoplasmic transduction peptide that was developed as a cellular import tag previously. We have synthesized genes for these two, with codons optimized for *P. fluorescens* expression.

The supercharged variations of GFP, including negatively supercharged GFP (-30) and positively supercharged GFP (+36), were previously developed by replacing solvent-exposed residues of GFP with negatively or positively charged amino acids. We have completely synthesized genes that code for these two supercharged proteins, with codons optimized for E. *coli* expression.

The primers we used for PCR had restriction enzyme sites that were utilized to insert the target genes to the MCSs of the plasmids (pDART, pFD10, and pBD10). The PCR products and plasmid vectors were double-digested with two restriction enzymes for XbaI, KpnI, SacI, or SpeI (which is compatible with XbaI). The specific pair of enzymes used on each gene can be directly identified from the full sequences provided in Table 2.

Then, the plasmid treated by restriction enzyme and the gene were ligated with T4 ligase. The constructed plasmids were then introduced into *E. coli* for cloning, and the cloned plasmids were first obtained using a standard plasmid purification method. The purified plasmids were then introduced to *P. fluoresceins,* for which expression and secretion were analyzed.

**[Example 4] Western blotting conditions**

After 48 h of cell growth (secretion occurs during the entire growth), the liquid culture reached stationary growth phase, and the cell density reached about 1.5 $\times$ $10^9$ cells/ml (OD600 = ~3). Then, 400 $\mu$l of the liquid cultures were taken and centrifuged at 18,000 rcf for 10 min to separate the supernatant and the cell pellet. 16 $\mu$l of culture (~0.048 OD) equivalents of the cell pellet extract and supernatant were each loaded onto 10% polyacrylamide gels. SDS-PAGE was used to separate the proteins according to their sizes.

Then, the proteins were transferred to a nitrocellulose membrane (Amersham) for Western blotting. Polyclonal anti-LARD3 rabbit immunoglobulin G (IgG) and anti-neomycin phosphotransferase 2 (Abcam, ab33595) were utilized as the primary antibody with 1:3000 and 1:500 dilution each, and anti-rabbit recombinant goat IgG-peroxidase (anti-rIgG goat IgG-peroxidase) was used as the secondary antibody with 1:1000 dilution. The bands were then detected using a chemiluminescence agent (Advansta WesternBright Pico). Western blotting images were acquired using an Azure C600 automatic detecting system. All included Western blotting images are representative results from at least three different repeated experiments, starting over again from cell culturing with independent *P. fluorescens* colonies.

After the images were obtained, the results of experiment 1 (FIG. 1) was quantified with ImageJ software. Then, % secretion of the target proteins of this experiment was calculated. The % secretion was calculated as follows.

$$\%\ \text{secretion} = S_{\text{supernatant}}/(S_{\text{supernatant}} + S_{\text{cell}})\ \text{X}\ 100\ \%$$

where S is the normalized signal strength of each bands in the Western blotting image, and the subscripts denote the sample type of the lanes.

**[Example 5] Analysis of polypeptide properties and protein structure**

The theoretical pI values of the target proteins were calculated using the ExPASy Compute pI/Mw tool (Wilkins, M. R., Gasteiger, E., Bairoch, A., Sanchez, J. C., Williams, K. L., Appel, R. D., and Hochstrasser, D. F. (1999) Protein identification and analysis tools in the ExPASy server. Methods Mol Biol 112, 531-552). The entire sequences were used, and LARD3 and any additional sequences from the enzyme sites were included in the sequences for this purpose. The protein pI values are highly correlated with their charge per residue, and the correlation analysis of the protein pI values and their charge per residue is included in FIG. 11.

FIG. 11 shows relationship between protein pI and their charges at pH 7.0. Isoelectric points and charge per 100 residues of the LARD3-attached recombinant proteins show highly linear correlation. Wild-type TliA is marked in blue. Proteins that were observed not to be secreted to the extracellular culture were marked in red. As a result, a clear linear correlation is observed. The estimated unfolded protein charge at pH 7.0 is calculated by Protein Calculator v3.4 (http: //protcalc. sourceforge.net/cgi-bin/protcalc).

Then, SWISS-MODEL structural homology modeling (https://swissmodel.expasy.org/) was used to study the ABC transporter protein structures (Arnold, K., Bordoli, L., Kopp, J., and Schwede, T. (2006) The SWISS-MODEL workspace: a web-based environment for protein structure homology modelling. Bioinformatics 22, 195-201).

The present inventors used *A. aeolicus* PrtD (PDB code 5122) (Morgan, J. L. W., Acheson, J. F., and Zimmer, J. (2017) Structure of a Type-1 Secretion System ABC Transporter. Structure 25, 522-529) as a template, with sequence identity of 40.98%. The result of prediction of the structure of TliD was shown in FIG. 12.

FIG. 12 shows the structure prediction result of TliD and alignment with template, colored according to QMEAN4 score. Residues with low prediction degree (light color part of FIG. 12) are mainly located on the external surface, typically on random coils and protrusion parts. QMEAN4 score and coloring were obtained by SWISS-MODEL.

The model's transmembrane helices were verified by DAS-TMfilter (http://mendel.imp.ac.at/sat/DAS/) (Cserzo, M., Eisenhaber, F., Eisenhaber, B., and Simon, I. (2002) On filtering false positive transmembrane protein predictions. Protein Engineering, Design and Selection 15, 745-752), and the results are provided in FIG. 13.

FIG. 13 shows transmembrane helices prediction result of modeled TliD. The prediction was obtained by DAS-TMfilter webserver. (A) Predicted structure of TliD dimer, with transmembrane helices marked with different colors. (B) Sequence of TliD, highlighted with the identical color-codes with (A).

The surface of the obtained 3D model was calculated with Swiss PdbViewer (spdbv) (http://spdbv.vital-it.ch/) and colored according to the charge. The present inventors used the ConSurf web server (http://consurf.tau.ac.il/2016/) to compare TliD with its homologs and to verify the structure prediction of TliD (Ashkenazy, H., Abadi, S., Martz, E., Chay, O., Mayrose, I., Pupko, T., and Ben-Tal, N. (2016) ConSurf 2016: an improved methodology to estimate and visualize evolutionary conservation in macromolecules. Nucleic Acids Research 44, W344-W350). FIG. 14 includes information of conserved residues of TliD.

FIG. 14 shows the Consurf homologue conservation analysis result of modeled TliD. The present inventors ran a ConSurf homolog conservation analysis on TliD. Multiple Sequence Alignment were built using MAFFT, the homologues were collected from UniProt, homologue search algorithm BLAST, PSI-BLAST E-value 0.001, number of PSI-BLAST Iterations 5, %ID Between Sequences 25-95%. 197 unique proteins were scanned, and among them, 50 sequences closest to the query were used. Phylogenic neighbors were scanned with ML distance, and the conservation score was calculated with Bayesian algorithm. (A) TliD dimer, colored according to the Bayesian conservation score. The transmembrane helices of TliD were conserved among the homologues. Specifically, the residues facing inside of the TliD's central channel were highly conserved, while residues facing outside of the central channel (facing the phospholipids or the cytoplasm) were highly variable.

The ConSurf homology analysis also approved our structural prediction in a sense that most of the transmembrane helices were highly conserved in the inner surface-facing residues, and this makes our structural prediction even more persuasive. Finally, the present inventors checked side-chain *pKa* values of the highly conserved arginine and lysine residues at the potentially important positions (C, D and F of FIG. 9) with the web-based PDB 2PQR server (http://nbcr-222.ucsd.edu/pdb2pqr_2.0.0/).

In the subsequently progressed homology-based structure prediction model, it was shown that the dimer of this protein has positive charge distribution at the inner surface of the channel (FIG. 9A and FIG. 9B). This prediction model was prepared using *Aquifex aeolicus* PrtD (PDB code 5122) with sequence identity of 40.98% as a template. Moreover, a ConSurf homolog conservation analysis on TliD showed that these charges were indeed conserved, forming a positively charged sub-region at the midpoint of the channel (FIG. 9C and FIG. 9D). In addition, on the kinked helix on the substrate entry window, there is a positively-charged residue that sticks out toward the pore of the window and blocks the window in ADP-bound state of TliD. The ConSurf results also verified that this residue was charge-conserved, as all of the 50 homologs had either arginine or lysine at this residue (FIG. 9C, black arrow). The present inventors expect that this positively-charged inner surface interacts with negatively-charged residues during protein transport, facilitating secretion (FIG. 9E).

FIG. 9 shows the charge distribution in the structure of TliD, the ABC protein of the TliDEF complex. (A) electron repulsion surface of the TliD monomer. Colored according to its surface electric potential, from *blue* (+7 *kBT*/*e*) to *red*(-7*kBT*/*e*). The inner surface of the central channel with highly positive charge is *circled* on top. (B) TliD homodimer, with one of the monomers presented in the ribbon model. The inner surface of the central channel with positive charge is circled on top and substrate entry window is *circled* on bottom. (C) TliD. The conserved positive charge cluster at the midpoint of the channel's inner surface are *circled.* The two $\alpha$-helices that form substrate entry window are ovaled. Among the two conserved positively-charged residues, Arg-316 (*black arrow*) sticks out to the pore. (D) TliD dimer, seen from the periplasmic face. Positive charges are located in the middle of the channel (*circled yellow*), whereas negative charges are outside of the channel. (E) schematic model of the TliD dimer, transporting a highly negatively charged recombinant polypeptide with the attached LARD3. The NBD (nucleotide-binding domain) and transmembrane domain (TMD) of TliD are labeled accordingly. The electric potential across the inner membrane (*IM*) is -150 mV, where the cytoplasm (*CP*) is more negative than the periplasm (PP). This potential difference also makes it more favorable to outward-transport negatively-charged proteins than positively-charged proteins.

The present inventors visualized the results with the PyMOL software. All sequences that were used for the analysis are provided in Table 2 and Table 3.

**[Example 6] Cross-analyzing the secretion of recombinant proteins and their pI**

Thirteen genes (Table 4) of target proteins of different sizes, flexibility, volume, weight, etc. were introduced to *P. fluorescens* $\Delta$*tliA* $\Delta$*prtA* via pDART, where they are attached to a C-terminal LARD3 signal sequence. Table 4 represents the list of genes and their references, and genes indicated by * represent genes which were not used in the present experiment, but were secreted in the previous research. Then, after liquid culturing the cells, the supernatant and cell pellet were analyzed via Western blotting (FIG. 1a and FIG. 1b).

[Table 4]

| Code | Protein name | Source | DNA type |
|---|---|---|---|
| TliA | Thermostable lipase A | *Pseudomonas fluorescens* SIK-W1 | Genomic DNA |
| NKC-TliA | NKC-TliA | Yang, K. S., Sung, B. H., Park, M. K., Lee, J. H., Lim, K. J., Park, S. C., Kim, S. J., Kim, H. K., Sohn, J.-H., Kim, H. M., and Kim, S. C. (2015) Recombinant Lipase Engineered with | Synthesi zed |
| | | Amphipathic and Coiled-Coil Peptides. ACS Catalysis 5, 5016-5025 | |
| CTP-TliA | CTP-TliA | Kim, D., Jeon, C., Kim, J.-H., Kim, M.-S., Yoon, C.-H., Choi, I.-S., Kim, S.-H., and Bae, Y.-S. (2006) Cytoplasmic transduction peptide (CTP): New approach for the delivery of biomolecules into cytoplasm in vitro and in vivo. Experimental Cell Research 312, 1277-1288 | Synthesi zed |
| Mann | Mannanase | *Bacillus subtilis* | Plasmid |
| MAP | Mussel adhesion protein | MAP fp-151 | Synthesi zed |
| MBP | Maltose binding protein | *Escherichia coli* XL1-Blue | Genomic DNA |
| Trx | Thioredoxin | *Escherichia coli* XL1-Blue | Genomic DNA |
| Cuti | Cutinase | *Nectria haematococca* | Plasmid |
| Chi | Chitinase | *Bacillus thuringenesis* | Plasmid |
| M37 | M37 lipase | *Photobacterium lipolyticum* | Plasmid |
| Cap | Capsid protein | *Chaetoceros salsugineum* DNA inclusion virus | Plasmid |
| Hsp40 | DnaJ charperone | *Escherichia coli XL1-Blue* | Genomic DNA |
| EglV | Endo-1,4-β-glucanase V | *Trichoderma reesei* QM6a | Total cDNA |
| GFP | Green fluorescent protein | pGFPuv (Clontech) | Plasmid |
| GFP(-30) | Negatively supercharged GFP | Lawrence, M. S., Phillips, K. J., and Liu, D. R. (2007) Supercharging proteins can impart unusual resilience. Journal of the American Chemical Society 129, 10110-10112 | Synthesi zed |
| GFP (+36 ) | Positively supercharged GFP | Lawrence, M. S., Phillips, K. J., and Liu, D. R. (2007) Supercharging proteins can impart unusual resilience. Journal of the American Chemical Society 129, 10110-10112 | Synthesi zed |
| EGF | Epidermal growth factor | *Homo sapiens* | Plasmid |
| AP | Alkaline phosphatase | *Escherichia coli* XL1-Blue | Genomic DNA |
| PLA1 | Phospholipase $A_1$ | *Serracia marescens* | Plasmid |

As shown in FIG. 1a and FIG. 1b, Mannanase, MBP, NKC-TliA, EglV, GFP, and thioredoxin were both detectable in the cell pellet and the supernatant, showing successful expression and secretion out to the extracellular media. However, MAP, cutinase, chitinase, capsid, Hsp40, and CTP-TliA were not detected in the supernatant despite being detected in the cell pellet, signifying that they were not secreted.

FIG. 1a and FIG. 1b confirm secretion of selected proteins, and represent western blotting image showing the expression and secretion of the target proteins. The cell samples show the amount of the protein that remains in the cytoplasm, and the supernatant samples represent the amount of protein that is localized to the extracellular space. For comparison, equivalent amounts of cell extract and culture supernatant (16 μl) were loaded onto the gel and were analyzed via Western blotting. 50 ng of TliA was loaded in the middle of the gel as a reference. Two other Western blottings were obtained from different culture samples. All of the unpresented results exhibit similar patterns. Below the images, there are Western blottings of the same samples but with primary antibody against cytosolic Neo, the neomycin/kanamycin phosphotransferase 2 protein. The nonspecific lysis or leakage is minimal in all samples except capsid.

These non-secreted proteins have a relatively high theoretical pI. All of them (with one exception, CTP-TliA) were above ~5.5, being either positively or less negatively charged. In contrast, the secreted proteins were relatively acidic and highly negatively charged with a pI that does not exceed 5.5 (FIG. 2a and FIG. 2b).

FIG. 2a and FIG. 2b shows correlation between % secretion of the target proteins and their pI values. The pI value of the target proteins is calculated from the sequence. The proteins that have not been secreted have their *bars colored red.* AP, EGF, and PLA1 are proven to be secreted in previous studies and are added in this figure. FIG. 2b added secretion percentage to pI. Three different biological replicates (independent culture samples) of the experiment in FIG. 1 were used for the quantitative analysis. Two highly basic outlier proteins that were not secreted, MAP (pI = 9.61) and capsid (pI = 9.25), were excluded from the plot. There was a negative correlation between the protein pI and their % secretion.

As could be seen in FIG. 1b and FIG. 2b, the secretion of NKC-TliA and CTP-TliA decreased dramatically from that of original TliA. These are derivatives of TliA with an N-terminally attached short, extremely positively-charged sequence (Table 2). CTP-TliA was not secreted at all. Note that CTP has nine consecutive residues composed solely of arginine with only one exception, alanine (RRARRRRRR), as described in Table 2.

Then, after quantification of western signal strengths of proteins, the percentage of secreted protein *versus* the total amount of expressed protein was plotted. The result was shown in FIG. 2b.

As shown in FIG. 2b, there seemed to be a weak negative correlation between protein pI and their secretion efficiency, but there were also a few exceptions.

**[Example 7] Analysis of lunasin and its derivatives**

Lunasin is an anticancer polypeptide from soybean *Glycine max.* It has a unique feature of nine consecutive aspartate (aspartic acid, Asp) sequences at its C terminus. The present inventors have constructed multiple derivatives of lunasin with different lengths of the aspartate polypeptide tails.

Then, lunasin and its derivatives were introduced to *P. fluoresceins* via pDART, and their expression and secretion were observed via Western blotting, and the result was shown in FIG. 3a and FIG. 3b.

FIG. 3a and FIG. 3b is the result of confirming expression and secretion of lunasin and its derivatives with different lengths of the oligo-aspartic acid tails, to determine the optimal length of the oligo-aspartic acid sequence in P. *fluorescens* expression and secretion system.

FIG. 3a detected expression of lunasin and its derivatives in the cell and supernatant via Western blotting, and specifically, 36-μl eq of cell extract and supernatant were loaded onto the gel and were analyzed via Western blotting. FIG. 3b represents protein sequence and domain structure of lunasin and its derivatives whose length of the aspartic acid tail is modified, and they were named as lunasin-D0, lunasin-D5, original lunasin (D9), lunasin-D15, and lunasin-D20, respectively.

As shown in FIG. 3a, the original lunasin showed that the highest secretion and relative amount of secreted proteins declined as the length of the oligo-aspartate tail decreased. The present inventors have also observed decreased secretion and expression levels in lunnasin-D15. Lunasin-D20 was not expressed in either the cell or supernatant. The exact sequence of the lunasin polypeptide and its derivatives is given in FIG. 3b.

Based on this experiment, the present inventors determined that the optimal length of the aspartate polypeptide sequence would be approximately nine, and we set up the experiments below.

**[Example 8] Construction of pFD10 and pBD10 with added aspartate polypeptide**

Among the 20 most common amino acids, aspartic acid has the lowest side chain *pKa* value (Mathews, C. K. (2013) Biochemistry, 4th ed., Pearson, Toronto). Inspired by the lunasin protein sequence in the Example 7, the present inventors developed two plasmids that add the aspartate polypeptide sequence to the inserted proteins as well as the LARD3 signal sequence.

The present inventors have synthesized an aspartate-decamer-coding DNA sequence based on the DNA sequence of the lunasin gene's aspartate polypeptide tail, and have named D10 (DDDDDDDDDD : SEQ ID NO: 33). Then, the present inventors conjugated D10 to the pDART plasmid, creating two types of plasmid that either add *D10* to the N terminus or to the C-terminus of the gene inserted to MCS, respectively, by inserting into pDART plasmid (named pGD10 and pBD10, respectively), and this was shown in FIG. 4.

FIG. 4 shows the structures of plasmids used, and represents the structure of pDART plasmid comprising MCS.

tliD, tliE, tliF, and the LARD3-attached fusion protein are controlled in a single operon. (A) MCS is directly followed by the LARD3 gene, and thus the inserted target gene is expressed with LARD3 attached on its C terminus. (B) represents structure of pFD10 plasmid that attaches D10 sequence at the N terminus. The D10 gene directly follows the start codon and is located right before the MCS and LARD3. (C) represents structure of the pBD10 plasmid, which attaches D10 sequence at the C-terminal side, but before LARD3. The D10 gene is located between the MCS and LARD3.

Then, selected proteins were inserted into both of the newly created plasmids, pFD10 and pBD10. These pFD10 or pBD10-cloned recombinant proteins were introduced to *P. fluorescens* alongside their pDART-cloned counterparts, and the secretion efficiency was analyzed via Western blot analysis.

**[Example 9] Insertion of TliA-derived recombinant proteins into pFD10 and pBD10**

NKC-TliA and CTP-TliA are both derivatives of TliA, each with an N-terminal basic-peptide attachment. Their secretion efficiency through TliDEF is significantly smaller than wild-type TliA (FIG. 1b and FIG. 10a, b).

FIG. 10a shows the result of enzyme plate assay of TliA, CTP-TliA and NKC-TliA, and TliA was secreted as expected (TliA is the natural substrate for the TliDEF transporter). However, secretion of CTP-TliA is blocked, and secretion of NKC-TliA is somewhat weaker than TliA.

FIG. 10b represents the result of western blotting of TliA, CTP-TliA, and NKC-TliA, and as could be seen in the enzyme plate assay, the secretion of TliA is strong, NKC-TliA is only weakly secreted, and CTP-TliA is not secreted. NKC is highly positively charged, and CTP has even more positively charged. CTP carries a consecutive nine residues composed solely of arginine with one exception in the middle, alanine.

However, the oligo-aspartate attachment on them by pFD10 or pBD10 greatly re-increases their secretion. The experimental result was shown in FIG. 5.

FIG. 5 shows the result of adding 10 aspartic acids to the N-terminus (FD10) and C-terminus (BD10) of two kinds of TliA lipases in which NKC and CTP sequences are attached, respectively (NKC-TliA, CTP-TliA) and expressing through pDART plasmid, then detecting by western blotting and lipase activity measuring medium.

In (A) of FIG. 5, secretion strongly improved in both pFD10 and pBD10 when compared with pDART. (B) represents the result of enzyme plate assay of NKC-TliA in different plasmids. (C) is the result of western blotting of CTP-TliA in pFD10 and pBD10, and it is confirmed that secretion strongly increased in pBD10. (D) represent the result of enzyme plate assay of CTP-TliA in different plasmids. pBD10 exhibits a major increase in secretion. Two other Western blot results were obtained from different culture samples, and both of them exhibit similar patterns. Two other enzyme plate assays were obtained from different colonies, and both of them exhibit similar patterns.

In terms of the secretion ratio (secreted protein *versus* intracellular protein), NKC-TliA shows a dramatic increase in secretion after the addition of either an upstream or downstream D10 sequence, as shown in (A) and (B) of FIG. 5.

CTP-TliA also shows a drastic increase in secretion in both the Western blotting and activity plate assays when a downstream D10 sequence was added by pBD10, as shown in (C) and (D) of FIG. 5. In enzyme plate activity assays, the halo sizes of NKC-TliA and CTP-TliA in pDART or pBD10 are generally consistent with the band strength of the supernatant samples in their respective Western blotting results. However, pFD10 has a slightly smaller halo than expected from their band strength, indicating the possibility of a reduced enzymatic activity.

**[Example 10] Insertion of negatively-charged proteins to pFD10 and pBD10**

A recombinant plasmid obtained by introducing genes for GFP, mannanase, maltose binding protein (MBP), and thioredoxin to pDART, pFD10, and pBD10 was introduced to *P. fluorescens* to prepare transformed proteins. The produced proteins were secreted by the TliDEF transporter, and the experimental result was shown in FIG. 6.

FIG. 6 shows secretion of negatively-charged proteins in pFD10 and pBD10. (A) represents the result of western blotting of GFP, and both pFD10 and pBD10 exhibit an increase in protein secretion in the supernatant. (B) represents the result of western blotting of Mannanase, and both pFD10 and pBD10 exhibit slight increases in mannase secretion. (C) represents the result of western blotting of MBP, and the increased secretion ratio was observed in both pFD10 and pBD10. (D) represents the result of western blotting of thioredoxin, and the signals were weak overall, but there was an increase in the secretion for both pFD10 and pBD10. Overall, the bands of more negatively-charged proteins in pBD10 appeared in slightly upward-shifted positions. Three other Western blot results for pDART and pBD10 were from different culture samples were obtained, whereas there were two other Western blot results for pDART and pFD10. All of them exhibit similar patterns.

As shown in FIG. 6, GFP showed the most dramatic increase in the increase of secretion. A comparison of the band strength of pDART and pBD10-inserted GFP showed a remarkable change in the supernatant versus cell expression ratio. pFD10-GFP also exhibited some improvement in terms of the ratio between the supernatant and the cell pellet ((A) of FIG. 6).

The case of mannanase was somewhat vague, but it could be concluded that pBD10-mannanase exhibits a better secretion than pDART-mannanase. In addition, although the absolute expression itself decreased, there was a small improvement in the ratio when an upstream D10 sequence was added by pFD10 ((B) of FIG. 6).

The secretion of MBP improved in both pFD10 and pBD10 in terms of the supernatant/cell ratio, compared with pDART ((C) of FIG. 6).

In the case of Trx (thioredoxin), the supernatant/cell ratio improved in pFD10 and pBD10 ((D) of FIG. 6).

Consequently, as the result of western blotting, it was confirmed that proteins in which aspartic acid was added to the N-terminus or C-terminus had increased (Supernatant) to intracellular (Cell) protein concentration ratio, and FD10 showed a significantly reduced pattern of expression.

### [Example 11] Addition of positively charged amino acid oligomers - construction and analysis of pBR10

The present inventors constructed an additional plasmid that closely resembles pBD10, but with one difference. In this plasmid, the D10 sequence, the DNA sequence that codes for aspartate oligomer, was replaced with R10 that codes for arginine oligomer.

The present inventors inserted the TliA and GFP gene to pDART, pBD10, and pBR10 plasmids and examined their secretion by enzyme activity media (TliA only) and Western blotting, and the results were shown in FIG. 7.

FIG. 7 represents the result of adding 10 aspartic acids (D) or 10 arginines, respectively, at the C-terminus of TliA lipase and green fluorescent protein (GFP) and then detecting by western blotting and lipase activity media. negatively-charged proteins, TliA and GFP, were inserted in the plasmids that attach nothing except the signal sequence (pDART), oligo-aspartate (pBD10), and oligo-arginine (pBR10). A of FIG. 7 represents the result of western blot of TliA in these plasmids. TliA in pDART and pBD10 shows good secretion. However, the secretion was blocked when R10 was attached. B of FIG. 7 represents the result of enzyme plate assay of TliA in these plasmids. Secretion of TliA was blocked when it was inserted to pBR10.

In Western blotting of TliA, pDART and pBD10 exhibited good secretion efficiency. pBR10, however, blocked the secretion (A of FIG. 7). Similar patterns were observed in enzyme plate assay, pBR10 did not exhibit halo, but the others did (B of FIG. 7). In Western blotting of GFP, both pDART and pBD10 exhibited secretion. Yet again, pBR10 blocked secretion of GFP as it did to TliA (C of FIG. 7).

### [Example 12] Western blot analysis of supercharged proteins

Green fluorescent protein (GFP) and its two supercharged derivatives, GFP (-30) and GFP (+36) by Average Number of Neighboring Atoms Per Sidechain Atom (AvNAPSA) method (Lawrence MS, Phillips KJ, Liu DR. Supercharging Proteins Can Impart Unusual Resilience. Journal of the American Chemical Society 2007; 129: 10110-10112.), were recombined with LARD3 through pDART and introduced to *P. fluorescens ΔtliA ΔprtA,* to express proteins, and then the samples were analyzed via western blotting, and the result was shown in FIG. 8.

FIG. 8 represents secretion of GFP and supercharged GFPs. GFP (-30) exhibited a much higher extracellular (Supernatant) to intracellular (Cell) protein concentration ratio, and a significantly higher secretion than the original GFP.

On the other hand, positively supercharged GFP (+36) was detected in cells at a high concentration, but was not secreted at all outside of the cell (Supernatant). Although the bands of the supercharged GFPs are also slightly shifted upwards, but two other Western blot results from different culture samples were obtained, and both of them exhibit similar patterns.

As could be seen in FIG. 8, both GFP and GFP (-30) were detected in the cell pellet and the supernatant, indicating that they were effectively expressed and secreted to the extracellular space. Herein, it could be confirmed that GFP (-30) was more strongly localized to the supernatant than the original GFP.

In contrast, GFP (+36) was heavily expressed but localized in the cell pellet and was not secreted to the extracellular space. The pI values for these recombinant proteins were 4.64 for GFP (-30), 5.36 for unmodified GFP, and 10.42 for GFP (+36).

### [Example 13] Confirmation of the optimal linker length for increasing the protein secretion efficiency

NKC-TliA was selected as a model protein. NKC consists of 21 amino acids, and is a peptide forming amphiphilic $\alpha$-helix. 21 amino acids include lysine a lot, and as pI=10.78, when NKC-TliA is prepared by fusion to TliA lipase in which pI=5.01, pI=5.34 and the protein secretion is reduced.

The present inventors have confirmed that the secretion by replacing all the lysine of NKC protein to aspartate (NKC(-)), and in addition, have compared the secretion efficiency of NKC(-) through various linker lengths by linking linkers with various lengths to NKC(-) and TliA through western blotting and activity analysis plate, and the results were shown in FIG. 15. The lengths of linker were represented by L1 with one GGGGS from NKC (-) where noting is present, L2 with 2, and L3 with 3.

FIG. 15 represents the protein secretion in TliA, NKC(-), NKC-L1, NKC-L2, NKC-L3, and NKC-TliA. (A) of FIG. 15 is the western blotting result of TliA, and (B) of FIG. 15 shows the result of enzyme plate analysis of TliA in other plasmids.

As the result of the western blotting of (A) of FIG. 15, it could be confirmed that the far right NKC-TliA was not secreted at all, but secretion of NKC(-) in which all the lysine was replaced with aspartate and the protein in which a linker was attached to negatively charged NKC was significantly increased.

According to the result of the activity analysis plate of (B) of FIG. 15, it could be confirmed that the protein secretion was increased in NKC(-) than the conventional NKC, and the secretion was increased overall when a linker was introduced, and in particular, when 3 linkers were attached, the secretion was significantly increased. Through this result, it could be seen that the negatively charged NKC increased the protein secretion.

**[Example 14] Confirmation of protein secretion increase of negative supercharge**

The present inventors have observed the tendency of protein secretion by replacing amino acids of proteins to negatively charged amino acids, to confirm secretion efficiency changes by changing the protein charge.

For this, negative charge supercharge -10 and positive charge supercharge +13 were produced from Streptavidin (SAV) wild type protein, and similar to this, supercharge proteins with the negative charger supercharge -20 and positive charge supercharge +19 were produced from glutathione S-transferase (GST), to analyze the protein secretion, and the result was shown in the following Table 16.

FIG. 16 represents the analysis result of secretion of -10SAV, wtSAV, +13SAV and - 20GST, wtGST, and +19GST (SAV: streptavidin / GST: glutathione S-transferase). SAV (135aa) produces a tetramer and GST (215aa) produces a dimer. In gene synthesis, the charge of monomers was calculated (-10SAV: pI4.96/ wtSAV:pI6.76 / +13SAV: pI10.29 / -20GST: pI4.73 / wtGST: pI7.86 /+19GST: pI9.87).

As shown in FIG. 16, it could be confirmed that negatively charged supercharger proteins were present in cells a lot and they were secreted well, but it could be seen that the wild type protein and positively charged supercharge proteins were not expressed and secreted, and it could be confirmed that the negative charge supercharge increased the protein secretion.

Similarly, while looking at the structure randomly without using AvNAPSA method, supercharged glutathione S-transferase (GST) and streptavidin (Sav), in which protruding amino acids were replaced with aspartic acid or arginine, were added to pDART and were expressed, to perform western blotting, and the result was shown in FIG. 17.

As FIG. 17, it could be seen that the extracellular (Supernatant) to intracellular (Cell) protein concentration ratio of negatively supercharged proteins (indicated by red) was remarkably increased. On the other hand, positively supercharged proteins were detected in cells at a significantly high concentration, but they were not detected or were detected at a low concentration outside of cells (Supernatant).

**[Example 15] Confirmation of extracellular secretion increase of negatively supercharged protein using AvNAPSA method**

MelC$_2$ tyrosinase, cutinase (Cuti), Chitinase (Chi), and M37 lipase were negatively sugercharged by AvNAPSA method, and then negatively supercharged proteins (red) and non-supercharged natural proteins corresponding thereto (black) were added to pDART plasmids, repsectively, and were expressed to detect them by western blotting, and the result was shown in FIG. 18.

Specifically, the negatively supercharging method using AvNAPSA is as follows. At first, arpartic acid and glutamic acid are replacted and enter to a suitable position to obtain the negatively supercharged protein sequence by AvNAPSA algorithm (1. Lawrence MS, Phillips KJ, Liu DR. Supercharging Proteins Can Impart Unusual Resilience. Journal of the American Chemical Society 2007; 129: 10110-10112.). Then, the DNA sequence corresponding to the protein sequence was synthesized, and the synthesized DNA sequence was added to pDART plasmid and then negatively supercharged proteins were prepared.

It could be seen the negatively supercharged proteins were observed not only inside of cells (C) but also outside of cells (S) at a very high concentration, different from natural proteins which were not detected at all outside of cells, and their secretion were remarkably increased. In case of MelC2 tyrosinase protein, small sequence differences inclduing His-tag result in samll size differences between supercharged proteins and natural proteins.

In other words, through the expeirment, the present inventors have confirmed that proteins which were not secreted in the past could be extracellularly secreted with considerable efficiency, by supercharging proteins such as tyrosinase, cutinase, and the like, to which the secretion production method was not applicable by conventional techniques, using AvNAPSA algorithm.

**[Example 16] Confirmation of TliA protein secretion in cells in which T1SS transporters isolated from three different kinds of bacteria are expressed**

16-1. *Escherichia coli* HlyBD+TolC, *Dickeya dadantii* PrtDEF, *Pseudomonas aeruginosa* AprDEF isolation

The present inventors amplified certain part of operon comprising HlyB, HlyD genes from isolated genome of *Escherichia coli* CFT073 strain (Genbank AE014075) through PCR using two primers of hlyBD-s (SEQ ID NO: 34: GGGGAGCTCGGATTCTTGTCATAAAATTGATT), hlyBD-a (SEQ ID NO: 35: GGGGGATCCTTAACGCTCATGTAAACTTTCT), and plasmid pSTV-HlyBD was prepared in which this was inserted in order together with start codon and kozak sequence to pSTV plasmid (one of derivatives of pACYC plasmid) by amplifying transporter genes from genome of each strain through PCR, respectively. TolC consisting of transporters together with HlyB and HlyC was not comprised separately, since it is produced by E. coli itself.

In addition, the present inventors prepared the plasmid expressing three genes of PrtD, PrtE and PrtF of *Dickeya dadantii,* pEcPrtDEF (Delepelaire P, Wandersman C. Protein secretion in gram-negative bacteria. The extracellular metalloprotease B from Erwinia chrysanthemi contains a C-terminal secretion signal analogous to that of Escherichia coli alpha-hemolysin. J Biol Chem. 1990;265:17118-17125) and the plasmid expressing three genes of AprD, AprE and AprF of *Pseudomonas aeruginosa,* pAGS8 (Duong F, Soscia C, Lazdunski A, Murgier M. The Pseudomonas fluorescens lipase has a C-terminal secretion signal and is secreted by a three-component bacterial ABC-exporter system. Mol Microbiol. 1994;11:1117-1126).

16-2. Confirmation of protein secretion in cells in which T1SS transporters isolated from three different kinds of bacteria are expressed

The present inventors introduced one plasmid which the gene of TliA protein (original substrate of TliDEF transporter) was inserted to pQE184 plasmid, and one of plasmids expressing one kind of T1SS transporters isolated from three different kinds of bacteria prepared above (namely, pSTV-HlyBD expressing *Escherichia coli* HlyBD, pEcPrt-DEF expressing *Dickeya dadantii* PrtDEF, pAGS8 expressing *Pseudomonas aeruginosa* AprDEF) to E. coli by heat shock method simultaneously and expressed TliA and one of three transporters simultaneously, and then measured secretion of the recombinant target proteins from lipase enzyme activity measuring media to outside of cells through color changes of colony peripheral media, and the result was shown in FIG. 19.

As shown in FIG. 19, it could be confirmed that all the three transporters of *Escherichia coli* HlyBD+TolC (E. coli expresses the original TolC protein), *Dickeya dadantii* PrtDEF, and *Pseudomonas aeruginosa* AprDEF secreted TliA protein successfully. This can be inferred from the fact that halo is not observed in the strain in which only TliA protein is expressed (TliA only) without further expression of transporter proteins in E. coli. The result means that T1SS proteins of *Escherichia coli, Dickeya dadantii,* and *Pseudomonas aeruginosa* other than *Pseudomonas fluorescens* can recognize the LARD3 signal sequence of TliA.

**[Example 17] Confirmation of cutinase protein secretion in cells in which T1SS transporters isolated from three different kinds of bacteria are expressed**

17-1. Preparation of Negatively supercharged cutinase protein

Negatively supercharged cutinase protein (Cuti(-)) was prepared using AvNAPSA method to cutinase protein (Cuti).

17-2. Confirmation of cutinase protein secretion in cells in which T1SS transporters isolated from three different kinds of bacteria are expressed

After attaching the LARD3 signal sequence by the method of inserting cutinase genes to pLARD3 plasmid in which the gene of LARD3 signal sequence was inserted right behind of the multiple cloning site, based on pUC19 plasmid, to cutinase protein and negatively supercharged cutinase protein,
with the plasmid expressing three different kinds of T1SS transporter proteins (Escherichia coli HlyBD+TolC, Dickeya dadantii PrtDEF, Pseudomonas aeruginosa AprDEF) obtained by the method of Example 16, similar to the method of Example 16, two plasmids were introduced to E. coli cells simultaneously and were expressed simultaneously, and they were culture in cutinase enzyme activity measuring media at 37 °C for 3 days, and then the protein secretion to outside of E. coli was measured through color changes of colony peripheral media, and the result was shown in FIG. 20.

As shown in FIG. 20, it could be observed that the secretion of negatively supercharged cutinase was remarkably high than non-negatively-supercharged cutinase, in all the three kinds of T1SS transporter proteins. In the same manner, it could be inferred through comparison to control groups in which an empty plasmid was added instead of the transporter plasmid (Cuti(-) only, Cuti only).

**[Example 18] Confirmation of extracellular secretion of cutinase protein using western blotting**

After attaching the LARD3 signal sequence to cutinase protein (Cuti) and negatively supercharged cutinase protein (Cuti(-)), they were expressed in E. coli with three different kinds of T1SS transporter proteins obtained by the method of Example 16 and were liquid cultured, and then the intracellular and extracellular protein concentration was detected by western blotting, and the result was shown in FIG. 21.

As shown in FIG. 21, it could be observed that the secretion of negatively supercharged cutinase was remarkably high than non-negatively-supercharged cutinase, in all the three kinds of T1SS transporter proteins. In the same manner, the secretion fact could be inferred by comparison to control groups in which an empty plasmid was added instead of the transporter plasmid (Cuti(-) only, Cuti only).

**[Example 19] Confirmation of extracellular secretion of M37 lipase protein using western blotting**

After attaching the LARD3 signal sequence to M37 lipase protein and negatively supercharged M37 lipase protein (M37(-)), they were expressed in E. coli with three different kinds of T1SS transporter proteins obtained by the method of Example 16 and were liquid cultured, and then the intracellular and extracellular protein concentration was detected by western blotting, and the result was shown in FIG. 22.

As shown in FIG. 22, it could be observed that the secretion of negatively supercharged M37 was remarkably high than non-negatively-supercharged M37, in all the three kinds of T1SS transporter proteins. In the same manner, the secretion fact could be inferred by comparison to control groups in which an empty plasmid was added instead of the transporter plasmid (M37(-) only, M37 only).

**[Example 20] Evaluation of sequence identity of T1SS ABC transporters**

The sequence identity of TliD of TliDET transporter *of Pseudomonas fluorescens* and T1SS ABC transporters of *Escherichia coli* HlyBD+TolC, *Dickeya dadantii* PrtDEF, and *Pseudomonas aeruginosa* AprDEF with ABC proteins of other kinds of T1SS transporters was measured, and the result was shown in FIG. 23. FIG. 23 represents the sequence identity between TliDEF transporter and various T1SS transporters, and the proportion occupied by the sequence-like portion in the entire sequence.

Specifically, the sequence identity between transporter proteins was calculated using NCBI BLASTp algorithm, and the indicated sequence identity was calculated by omitting some sequences that greatly differ from each other according to the normal calculation method of the algorithm, and limiting within the query coverage. As a result, the omitted sequence portion was less than 10% in any case, suggesting that the sequence identity was very reliable.

The sequence identity of TilD of TliDEF transporter with various T1SS ABC transporters varied from relatively high to relatively low. Among them, the sequence identity of three T1SS transporters of AprD, PrtD, and HlyB which were examples in Examples 16, 17, 18 and 19 were exhibited as 60%, 59%, and 27%, respectively.

Accordingly, the present inventors confirmed that the protein secretion enhancement technology of negatively supercharging was not limited to *Pseudomonas fluorescens* microorganism TliDEF transporters, and could be widely applied to various T1SS transporters having about 27% nucleotide sequence identity (homology).

## Claims

1. An expression vector for extracellular secretion of a target protein in bacteria, comprising an expression cassette including a nucleotide sequence encoding Lipase ABC transporter recognition domain (LARD) and a nucleotide sequence encoding a target protein which are operably linked, wherein the LARD and the target protein have acidic pI and is expressed as a fusion protein.

2. The expression vector of Claim 1, wherein the expression vector comprises a nucleotide sequence comprising ABC transporter of bacterial Type 1 Secretion System (T1SS).

3. The expression vector of Claim 1, wherein the bacteria comprises an ABC transporter of Type 1 Secretion System (T1SS).

4. The expression vector of Claim 1, wherein the bacteria is Gram-negative bacteria.

5. The expression vector of Claim 1, wherein the bacteria is at least one selected from the group consisting of *Pseudomonas* sp., *Dickeya* sp., and *Escherichia* sp.

6. The expression vector of Claim 2, wherein the ABC transporter of T1SS is a transporter having at least 20% of nucleotide sequence identify with TliDEF transporter of *Pseudomonas fluorescence.*

7. The expression vector of Claim 2, wherein the ABC transporter of T1SS is TliDEF of *Pseudomonas fluorescens,* AprDEF of *Pseudomonas aeruginosa* (PaAprDEF), *PrtDEF of Dickeya dadantii* (DdPrtDEF), or HlyBD+TolC of *Escherichia coli.*

8. The expression vector of Claim 1, wherein the nucleotide sequence encoding a target protein further comprises a nucleotide sequence encoding an acidic peptide consisting of 6 to 20 amino acids.

9. The expression vector of Claim 8, wherein the acidic peptide consists of at least an amino acid selected from the group consisting of aspartic acid and glutamic acid.

10. The expression vector of Claim 8, wherein the acidic peptide comprises an amino acid sequence as shown in SEQ ID NO: 33(10 Aspartic acids; D10).

11. The expression vector of Claim 8, wherein the nucleotide sequence encoding an acidic peptide is located at 3'-terminus or 5'-terminus of the nucleotide sequence encoding a target protein.

12. The expression vector of Claim 1, wherein the nucleotide sequence encoding a target protein is obtained by deleting at least one of the basic amino acids in the target protein.

13. The expression vector of Claim 12, wherein the basic amino acids are selected from the group consisting of Lysine and Arginine.

14. The expression vector of Claim 1, wherein the target protein is negatively supercharged protein.

**15.** The expression vector of Claim 14, wherein the target protein is negatively supercharged protein obtained by performing the following steps:

a step of selecting an amino acid not affecting the structure of target protein by having a functional group protruding in three dimensional structure of the target protein and,
a step of negatively supercharging according to the Manual Supercharging method by substituting the selected amino acid with at least one selected from the group consisting of aspartic acid and glutamic acid when the selected amino acid is basic, or negatively supercharging the selected amino acid with at least one selected from the group consisting of lysine and arginine when the selected amino acid is acidic.

**16.** The expression vector of Claim 1, wherein the Lipase ABC transporter recognition domain (LARD) is LARD 1, LARD 2 or LARD 3.

**17.** A cell comprising an expression vector according to any one of Claim 1 to Claim 16.

**18.** The cell of Claim 17, wherein the cell further comprises an expression vector comprise a nucleotide sequence comprising ABC transporter of bacterial Type 1 Secretion System (T1SS).

**19.** The cell of Claim 17, wherein the cell is a bacteria comprising an ABC transporter of Type 1 Secretion System (T1SS).

**20.** The cell of Claim 19, wherein the cell is at least one selected from the group consisting of *Pseudomonas* sp., *Dickeya* sp., and *Escherichia* sp.

**21.** A method of producing a target protein in a cell, comprising
preparing a cell transformed by an expression vector according to any one of Claim 1 to Claim 16.
culturing the cell and producing a secretion protein, and
separating and purifying the produced secretion protein.

**22.** The method of producing a target protein in a cell according to Claim 21, wherein the cell further comprises an expression vector comprise a nucleotide sequence comprising ABC transporter of bacterial Type 1 Secretion System (T1SS).

**23.** A method of increasing extracellular secretion of a target protein in a cell, comprising a step of introducing an expression vector of any one of Claims 1 to 16.

**24.** The method of increasing extracellular secretion of a target protein in a cell according to Claim 23, wherein the cell further comprises an expression vector comprise a nucleotide sequence comprising ABC transporter of bacterial Type 1 Secretion System (T1SS).

【Fig. 1a】

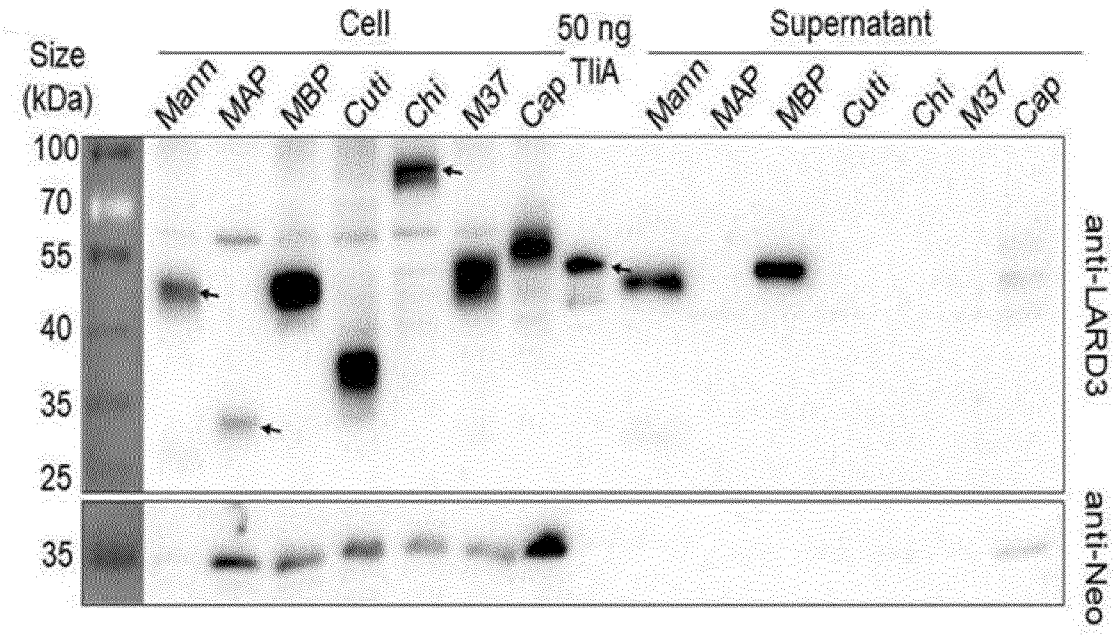
Cell
50 ng TliA
Supernatant
Size (kDa)
Mann
MAP
MBP
Cuti
Chi
M37
Cap
Mann
MAP
MBP
Cuti
Chi
M37
Cap
100
70
55
40
35
25
35
anti-LARD3
anti-Neo

【Fig. 1b】

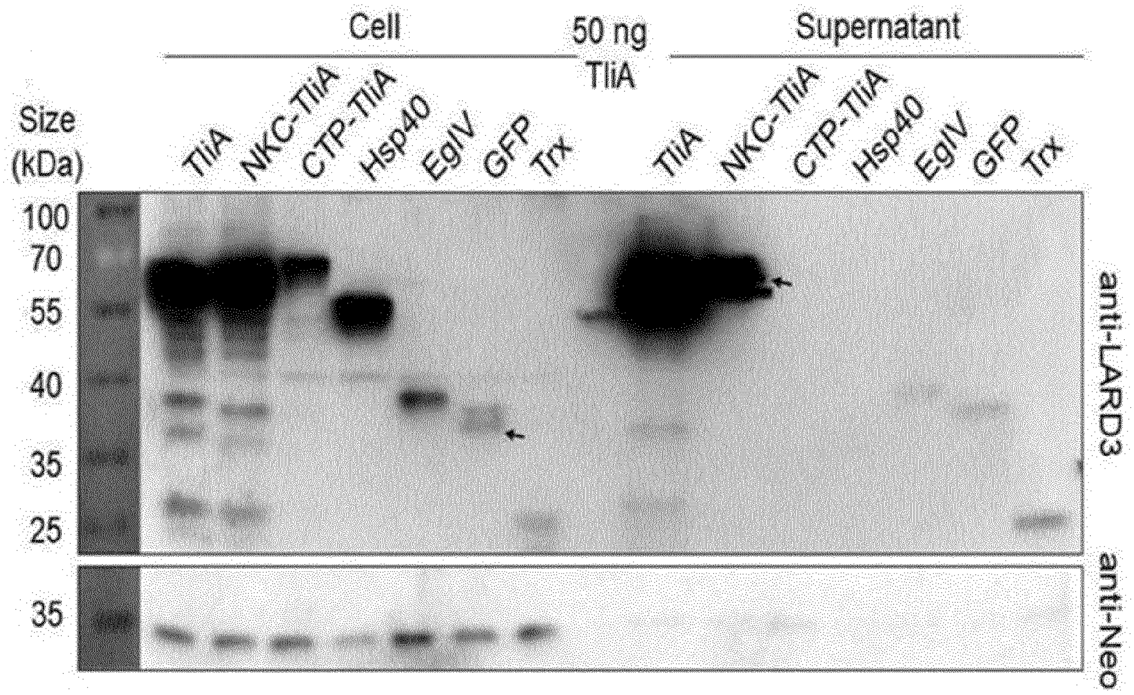
Cell
50 ng TliA
Supernatant
Size (kDa)
TliA
NKC-TliA
CTP-TliA
Hsp40
EglV
GFP
Trx
TliA
NKC-TliA
CTP-TliA
Hsp40
EglV
GFP
Trx
100
70
55
40
35
25
35
anti-LARD3
anti-Neo

【Fig. 2a】

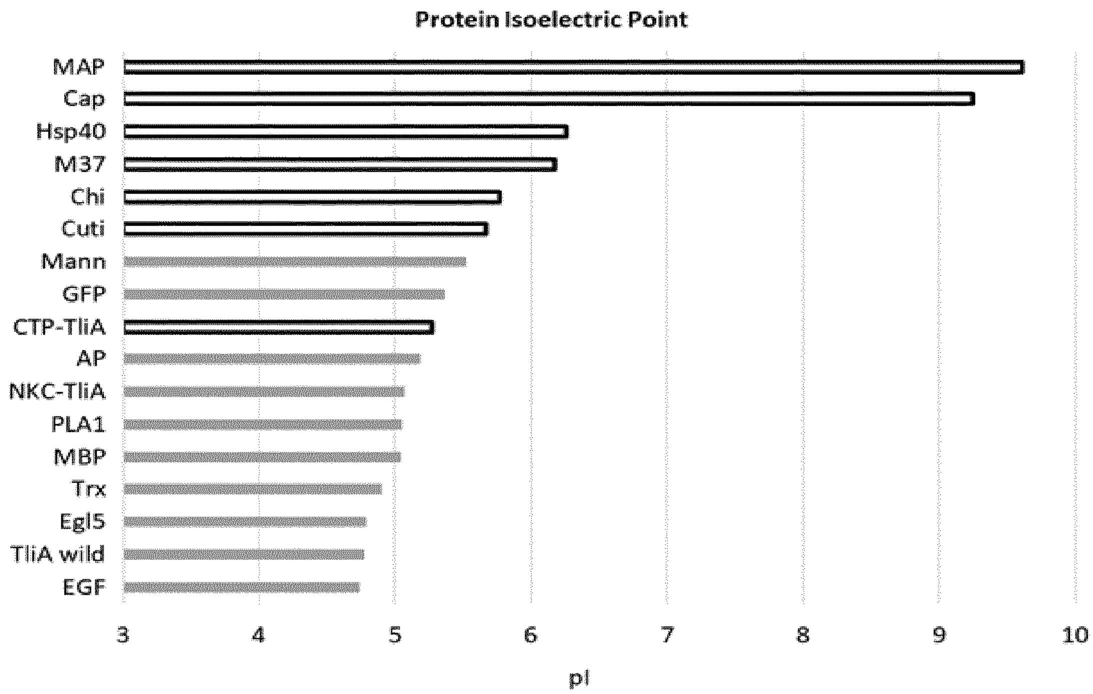
Protein Isoelectric Point
MAP
Cap
Hsp40
M37
Chi
Cuti
Mann
GFP
CTP-TliA
AP
NKC-TliA
PLA1
MBP
Trx
Egl5
TliA wild
EGF
3
4
5
6
7
8
9
10
pI

【Fig. 2b】

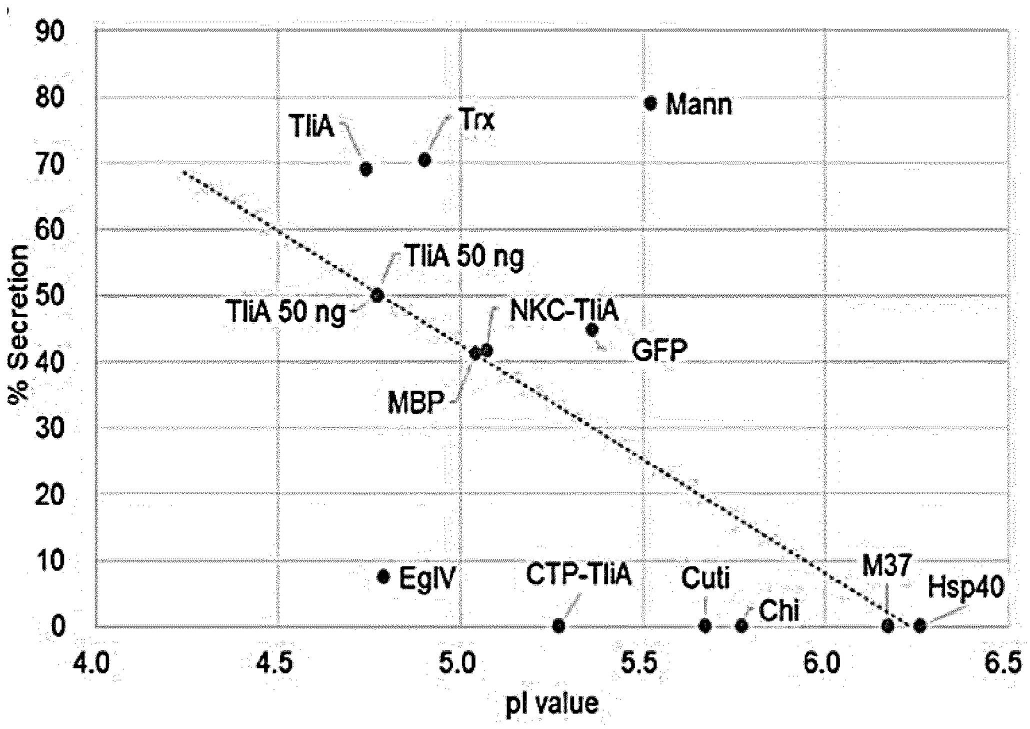
90
80
70
60
50
40
30
20
10
0
% Secretion
4.0
4.5
5.0
5.5
6.0
6.5
pI value
TliA
Trx
Mann
TliA 50 ng
TliA 50 ng
NKC-TliA
GFP
MBP
EglV
CTP-TliA
Cuti
Chi
M37
Hsp40

【Fig. 3a】

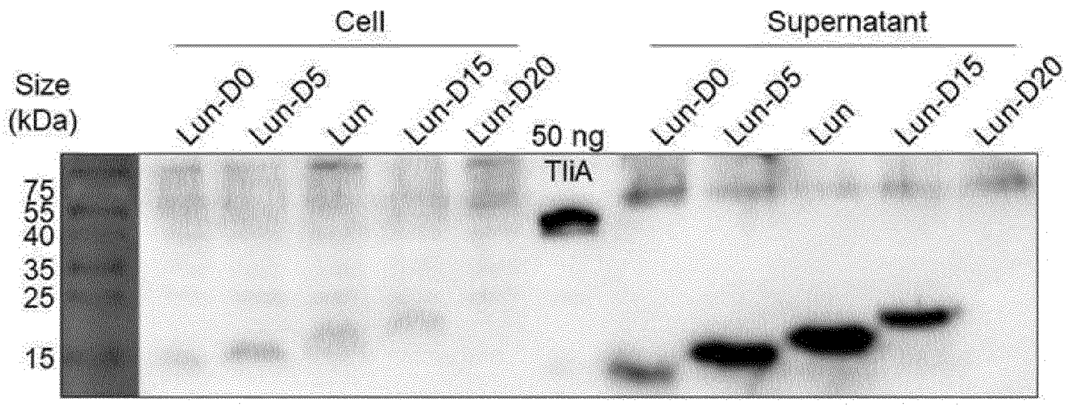
Cell
Supernatant
Size
(kDa)
Lun-D0
Lun-D5
Lun
Lun-D15
Lun-D20
50 ng
TliA
Lun-D0
Lun-D5
Lun
Lun-D15
Lun-D20
75
55
40
35
25
15

【Fig. 3b】

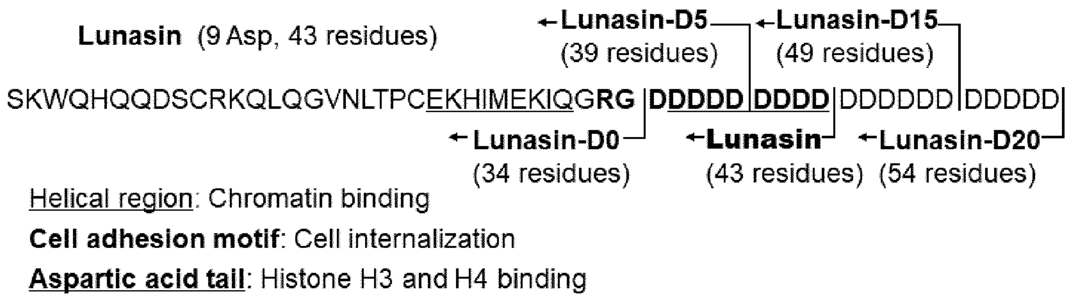
Lunasin (9 Asp, 43 residues)
←Lunasin-D5
(39 residues)
←Lunasin-D15
(49 residues)
SKWQHQQDSCRKQLQGVNLTPCEKHIMEKIQGRG DDDDD DDDD DDDDDD DDDDD
←Lunasin-D0
(34 residues)
←Lunasin
(43 residues)
←Lunasin-D20
(54 residues)
Helical region: Chromatin binding
Cell adhesion motif: Cell internalization
Aspartic acid tail: Histone H3 and H4 binding

【Fig. 4】

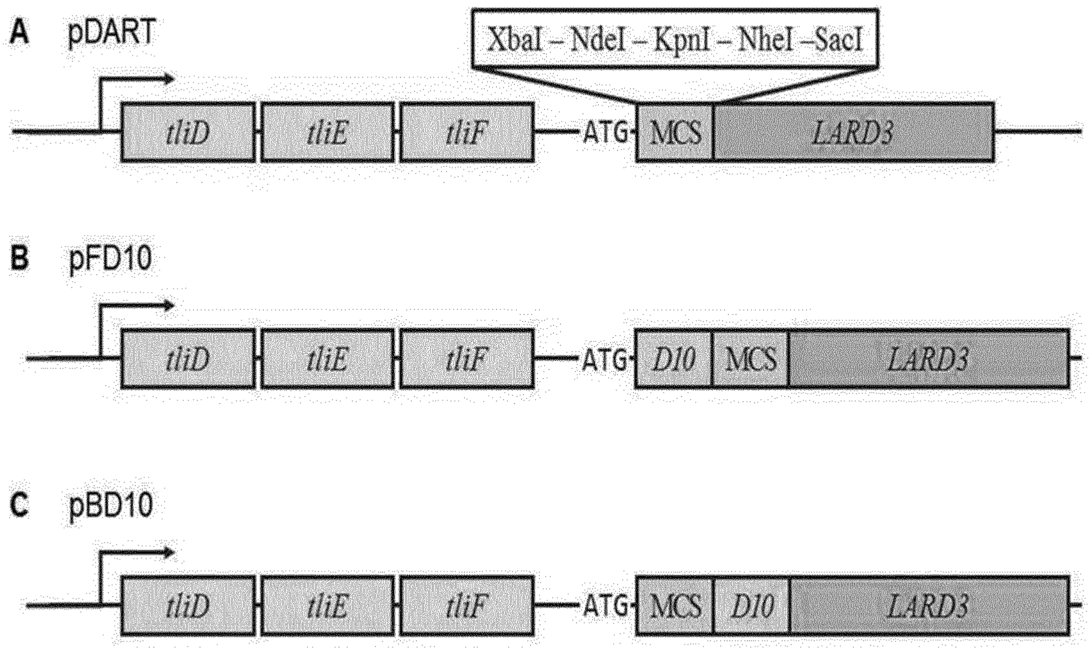
A pDART
XbaI – NdeI – KpnI – NheI –SacI
tliD
tliE
tliF
ATG
MCS
LARD3
B pFD10
tliD
tliE
tliF
ATG
D10
MCS
LARD3
C pBD10
tliD
tliE
tliF
ATG
MCS
D10
LARD3

【Fig. 5】

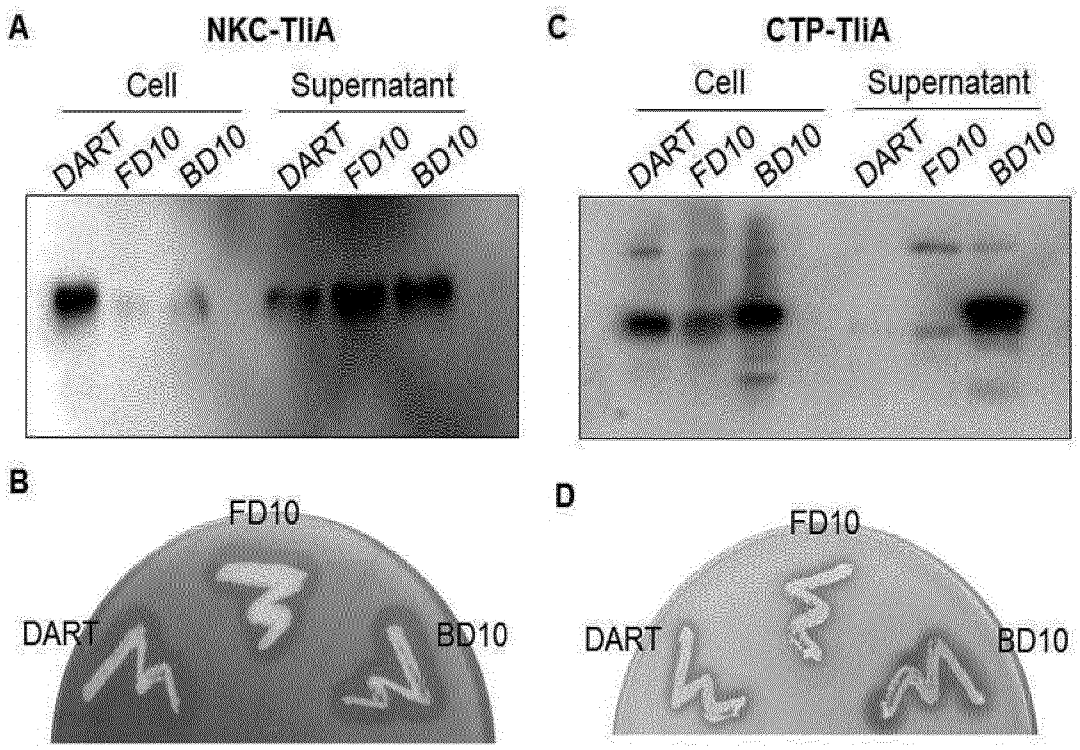
A
NKC-TliA
Cell
Supernatant
DART
FD10
BD10
DART
FD10
BD10
C
CTP-TliA
Cell
Supernatant
DART
FD10
BD10
DART
FD10
BD10
B
FD10
DART
BD10
D
FD10
DART
BD10

【Fig. 6】

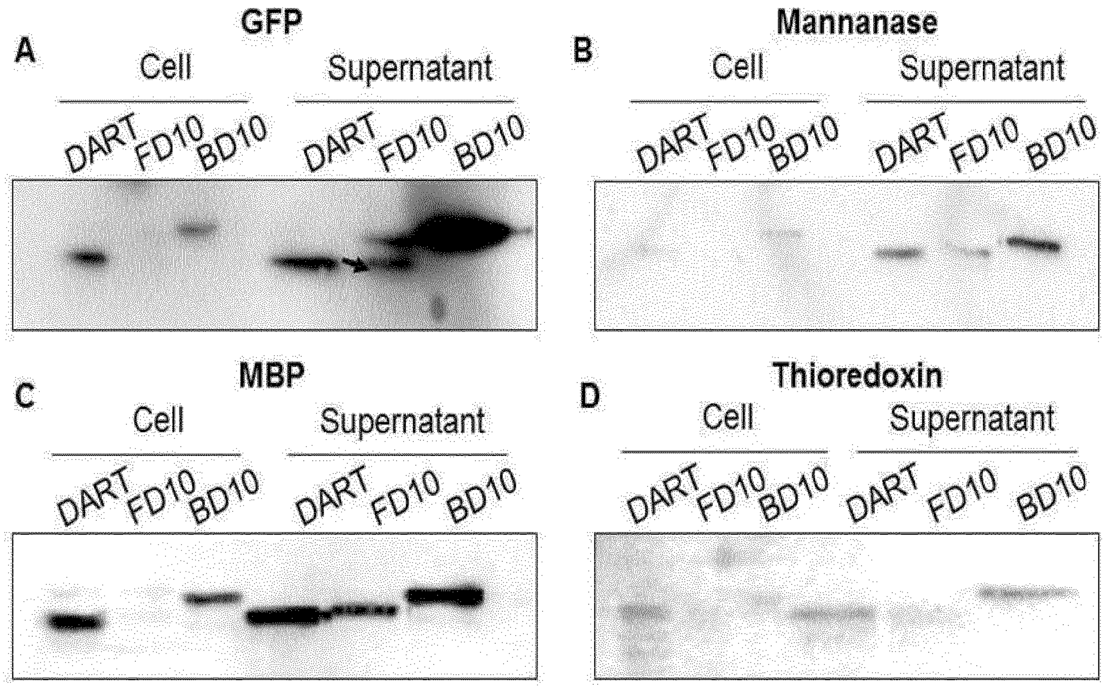
A
GFP
Cell
Supernatant
DART FD10 BD10 DART FD10 BD10
B
Mannanase
Cell
Supernatant
DART FD10 BD10 DART FD10 BD10
C
MBP
Cell
Supernatant
DART FD10 BD10 DART FD10 BD10
D
Thioredoxin
Cell
Supernatant
DART FD10 BD10 DART FD10 BD10

【Fig. 7】

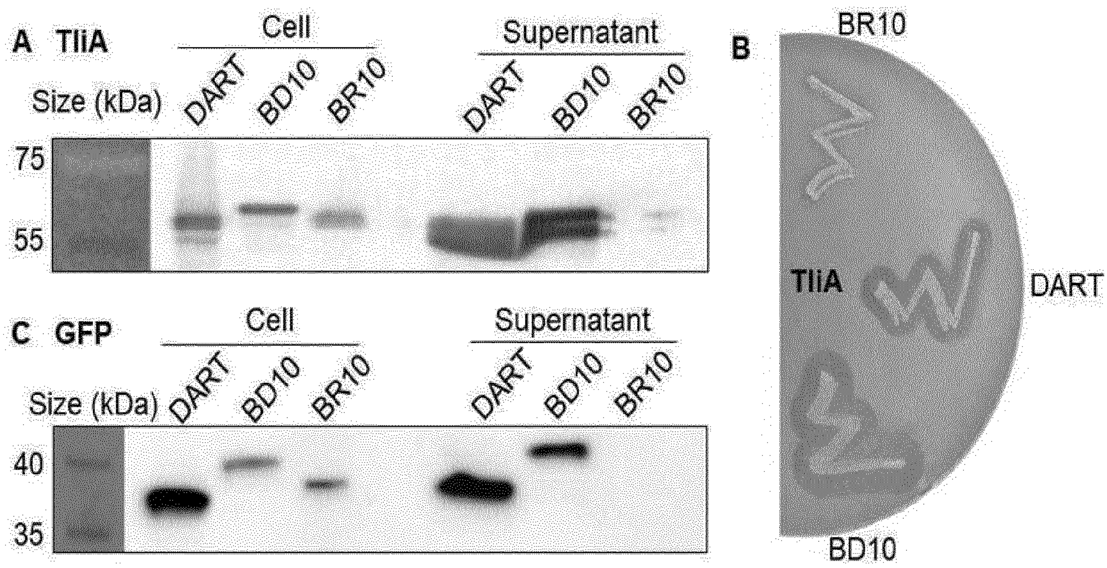
A TliA
Cell
Supernatant
Size (kDa)
DART BD10 BR10 DART BD10 BR10
75
55
C GFP
Cell
Supernatant
Size (kDa)
DART BD10 BR10 DART BD10 BR10
40
35
B
BR10
TliA
DART
BD10

【Fig. 8】

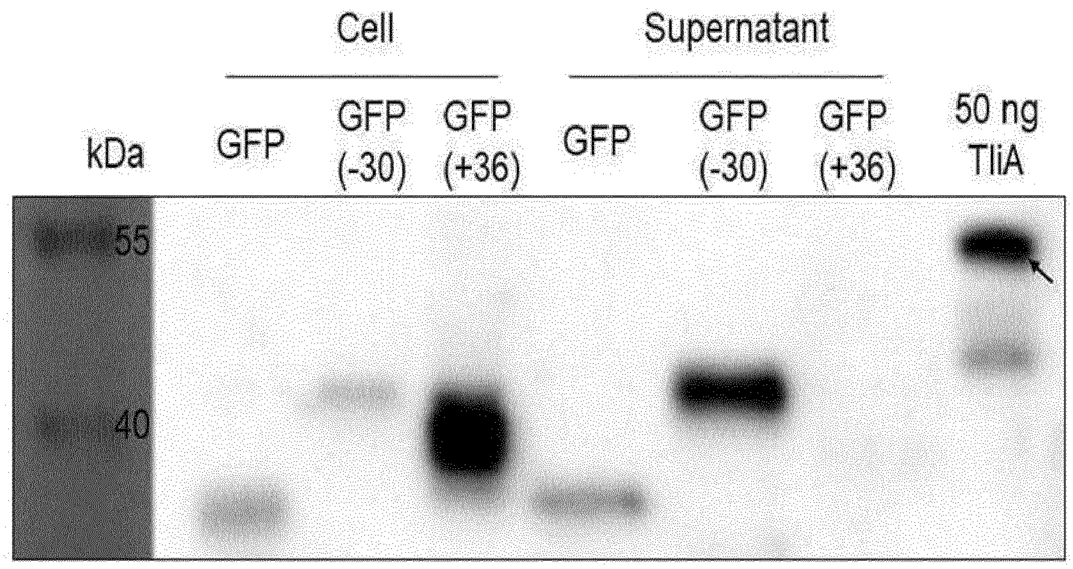
Cell
Supernatant
kDa
GFP
GFP (-30)
GFP (+36)
GFP
GFP (-30)
GFP (+36)
50 ng TliA
55
40

【Fig. 9】

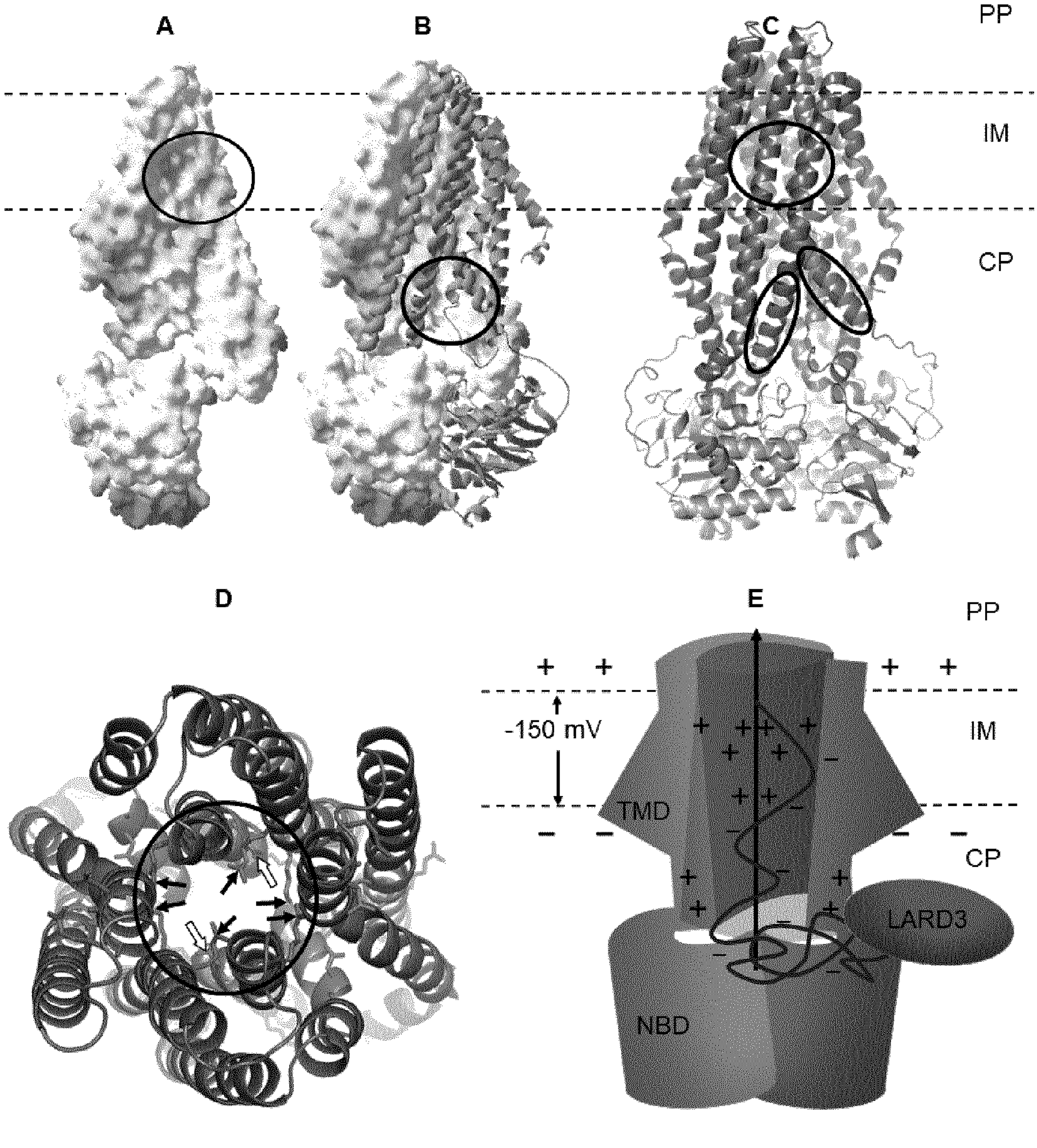

【Fig. 10a】

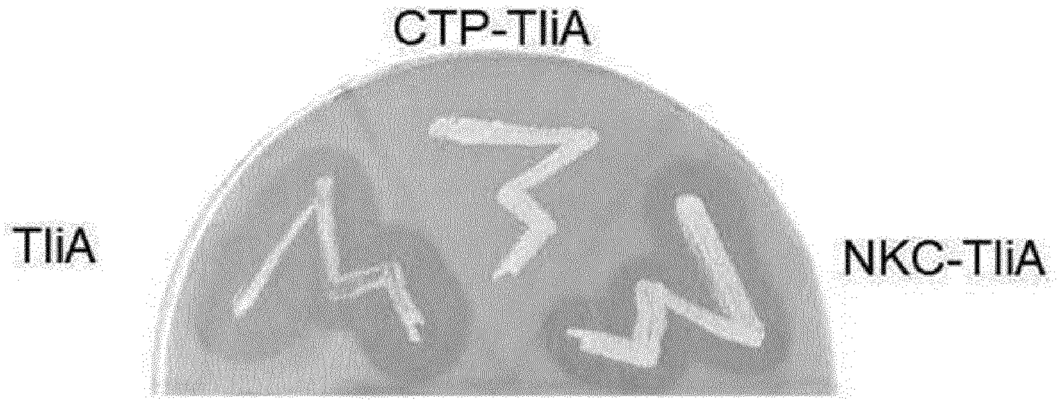


【Fig. 10b】

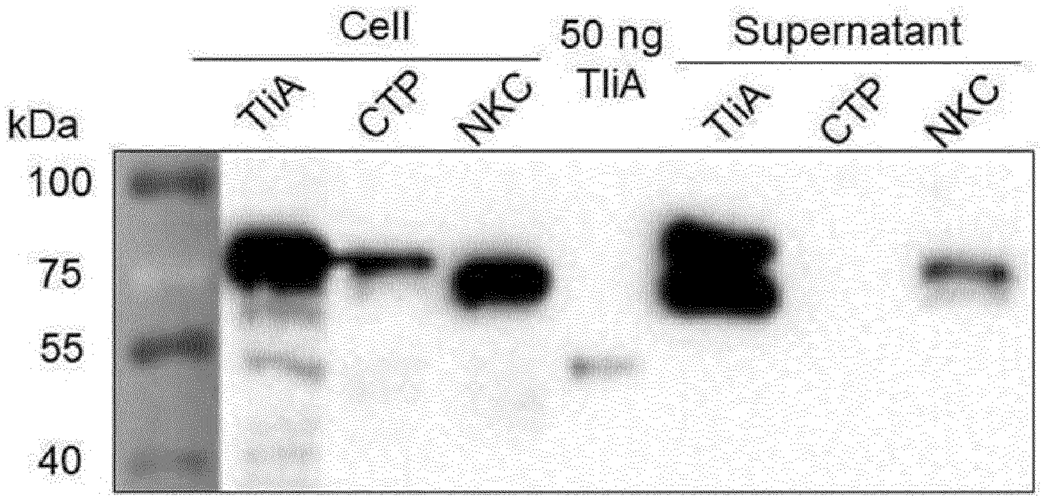

【Fig. 11】

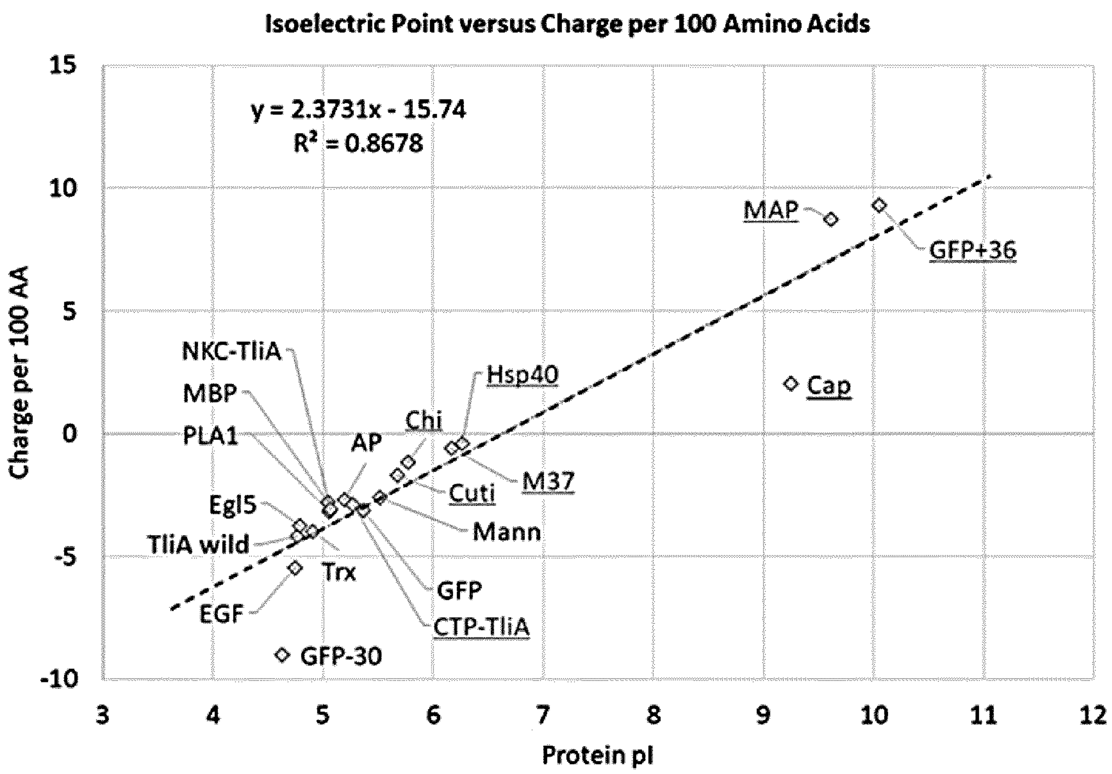
Isoelectric Point versus Charge per 100 Amino Acids
y = 2.3731x - 15.74
R² = 0.8678
Charge per 100 AA
Protein pI
15
10
5
0
-5
-10
3
4
5
6
7
8
9
10
11
12
MAP
GFP+36
Cap
NKC-TliA
MBP
PLA1
Hsp40
Chi
AP
M37
Cuti
Mann
Egl5
TliA wild
Trx
GFP
CTP-TliA
EGF
GFP-30

【Fig. 12】

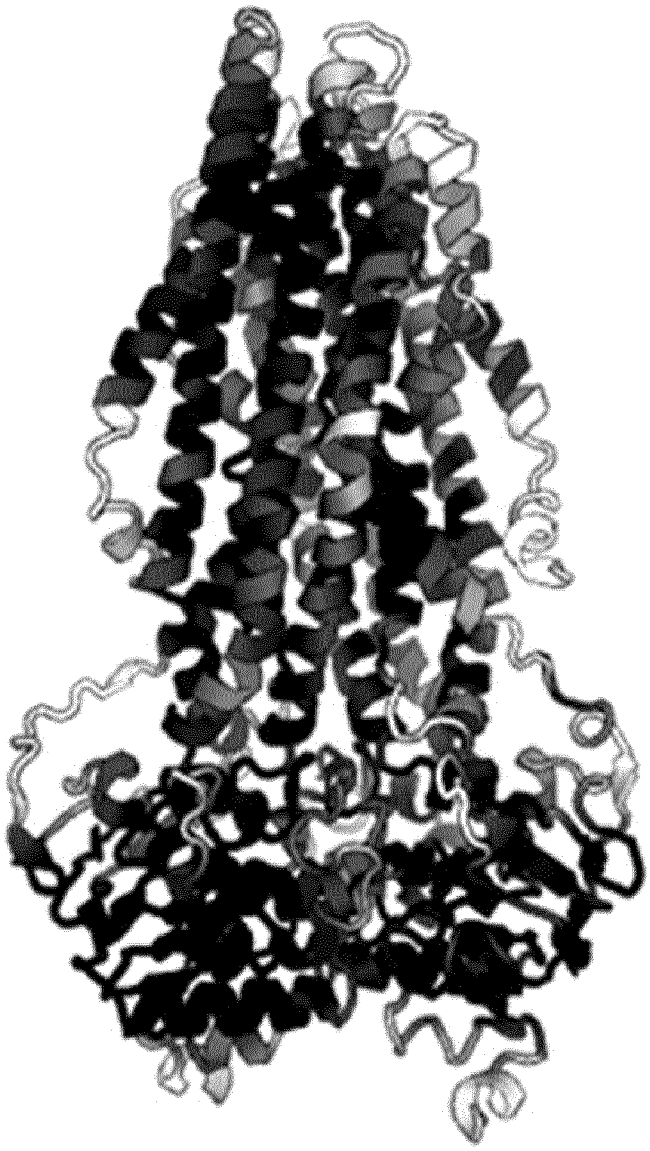

【Fig. 13】

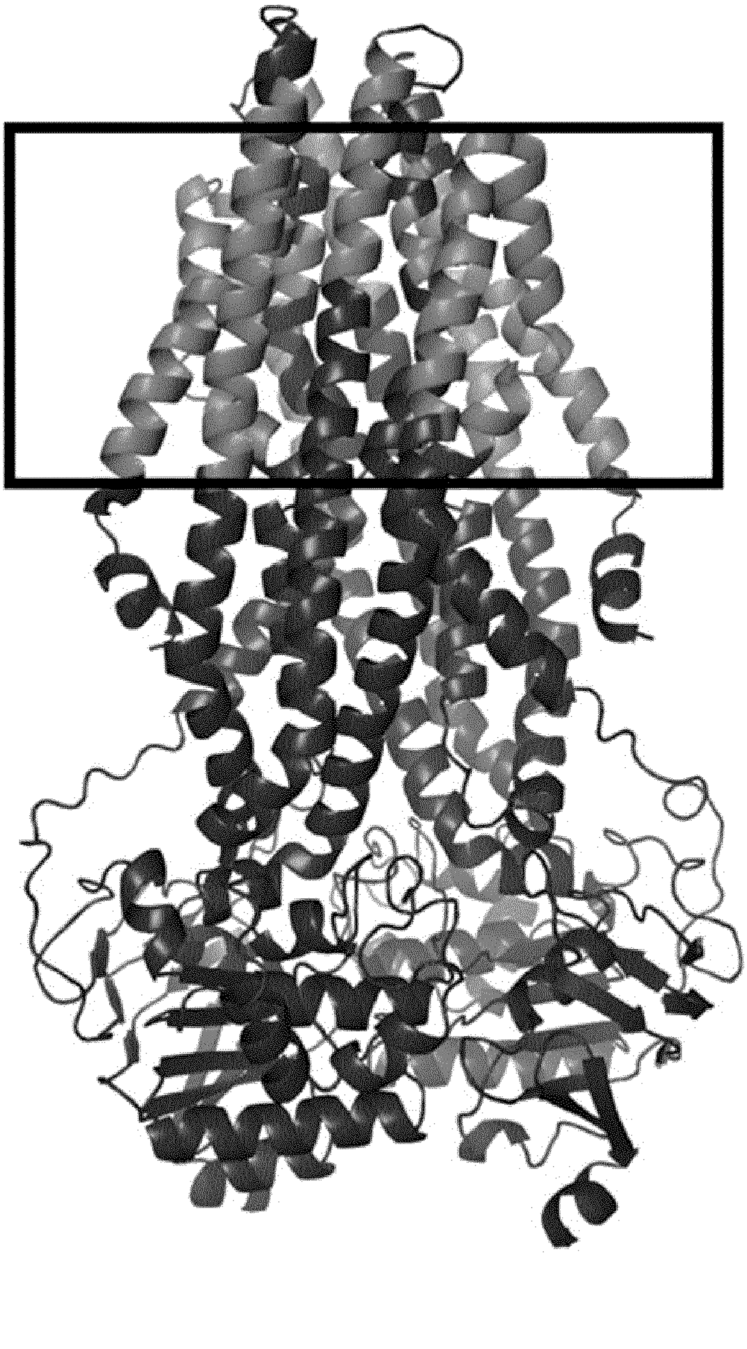

【Fig. 14】

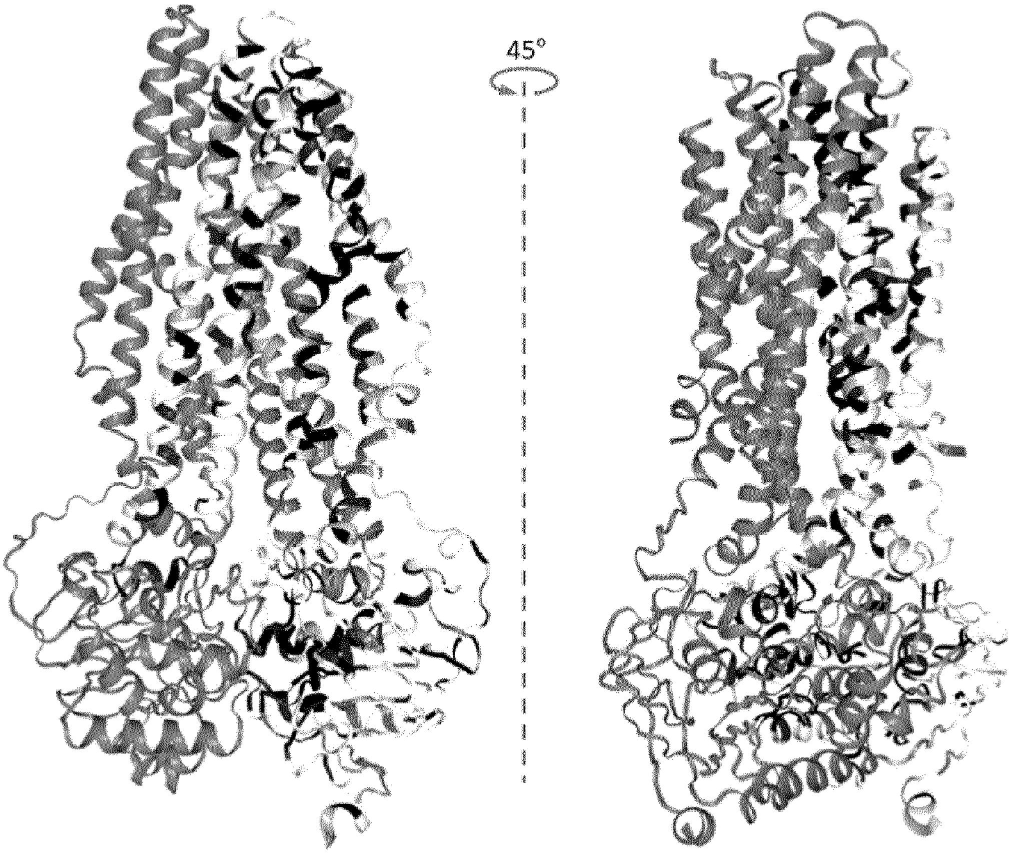

【Fig. 15】

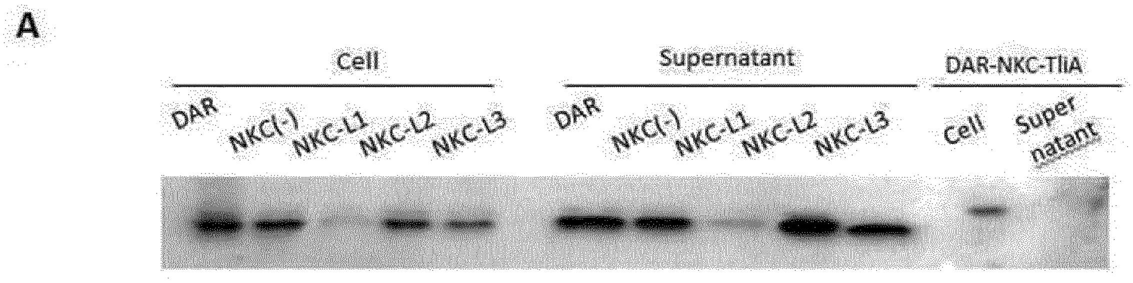
A
Cell
Supernatant
DAR-NKC-TliA
DAR
NKC(-)
NKC-L1
NKC-L2
NKC-L3
DAR
NKC(-)
NKC-L1
NKC-L2
NKC-L3
Cell
Super natant

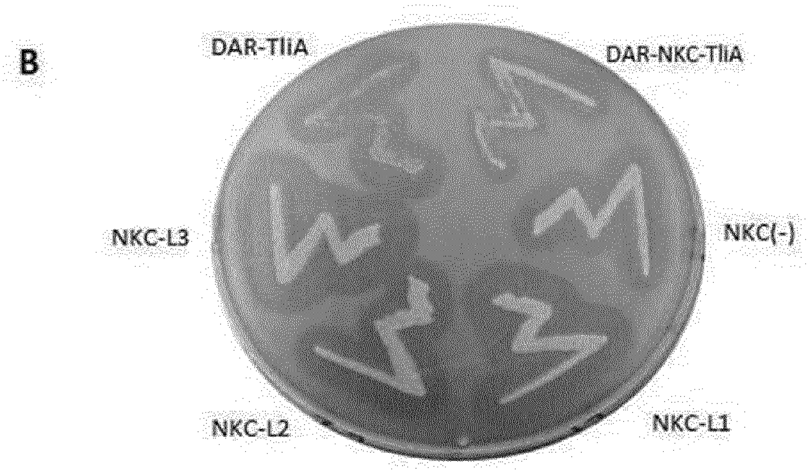
B
DAR-TliA
DAR-NKC-TliA
NKC-L3
NKC(-)
NKC-L2
NKC-L1

【Fig. 16】

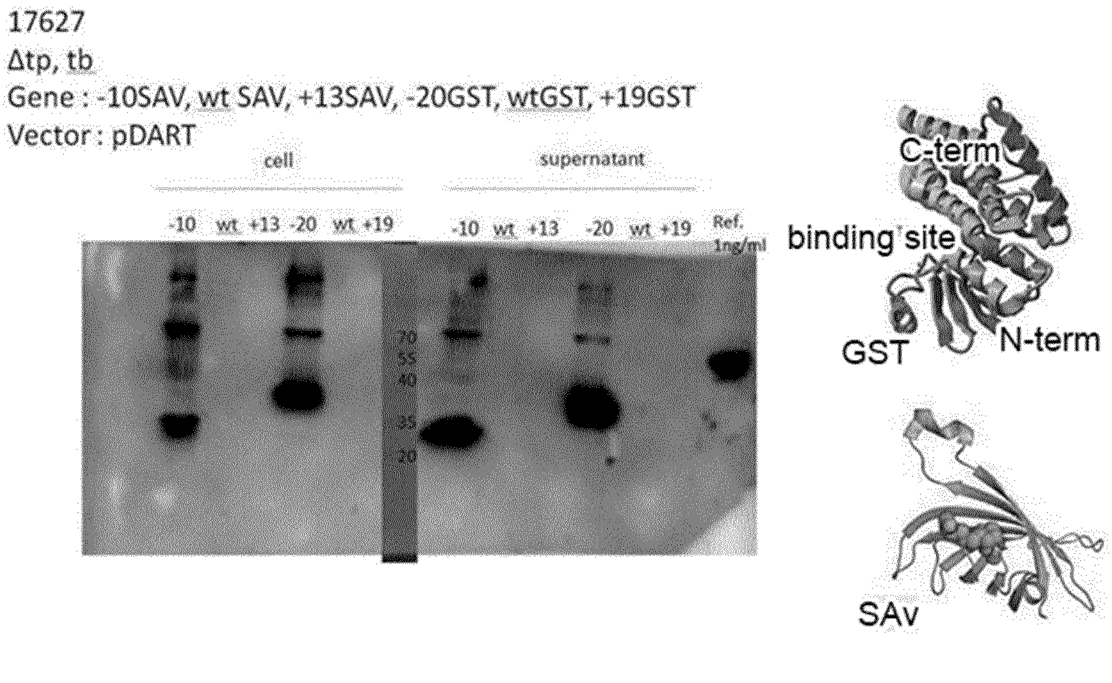
17627
Δtp, tb
Gene : -10SAV, wt SAV, +13SAV, -20GST, wtGST, +19GST
Vector : pDART
cell
supernatant
-10 wt +13 -20 wt +19
-10 wt +13 -20 wt +19
Ref. 1ng/ml
70
55
40
35
20
C-term
binding site
GST
N-term
SAv

【Fig. 17】

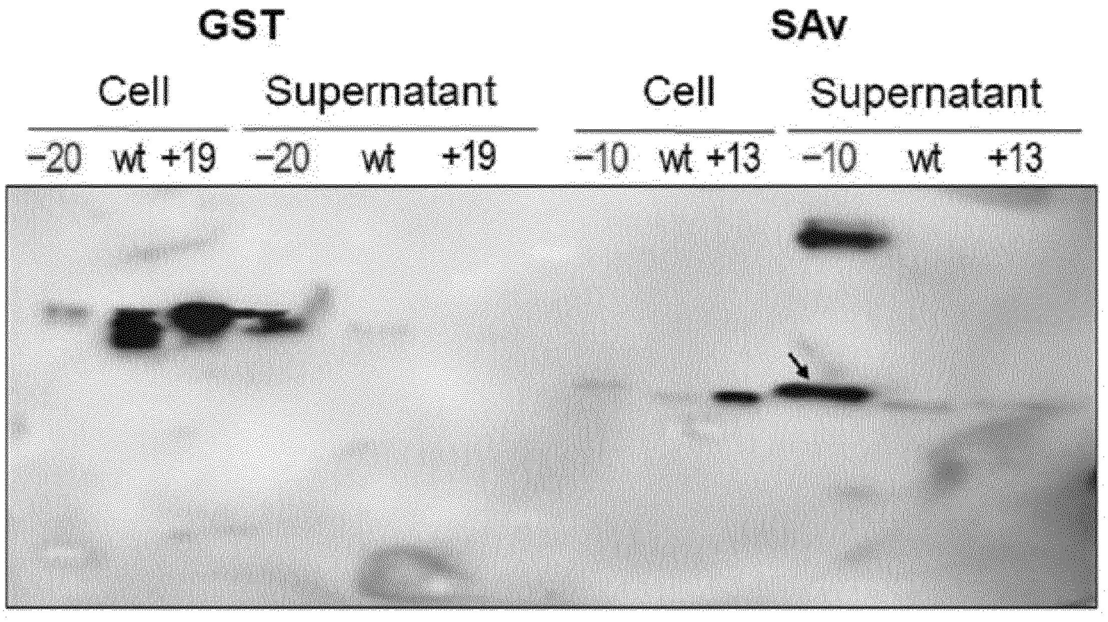
GST
SAv
Cell
Supernatant
Cell
Supernatant
−20 wt +19 −20 wt +19
−10 wt +13 −10 wt +13

【Fig. 18】

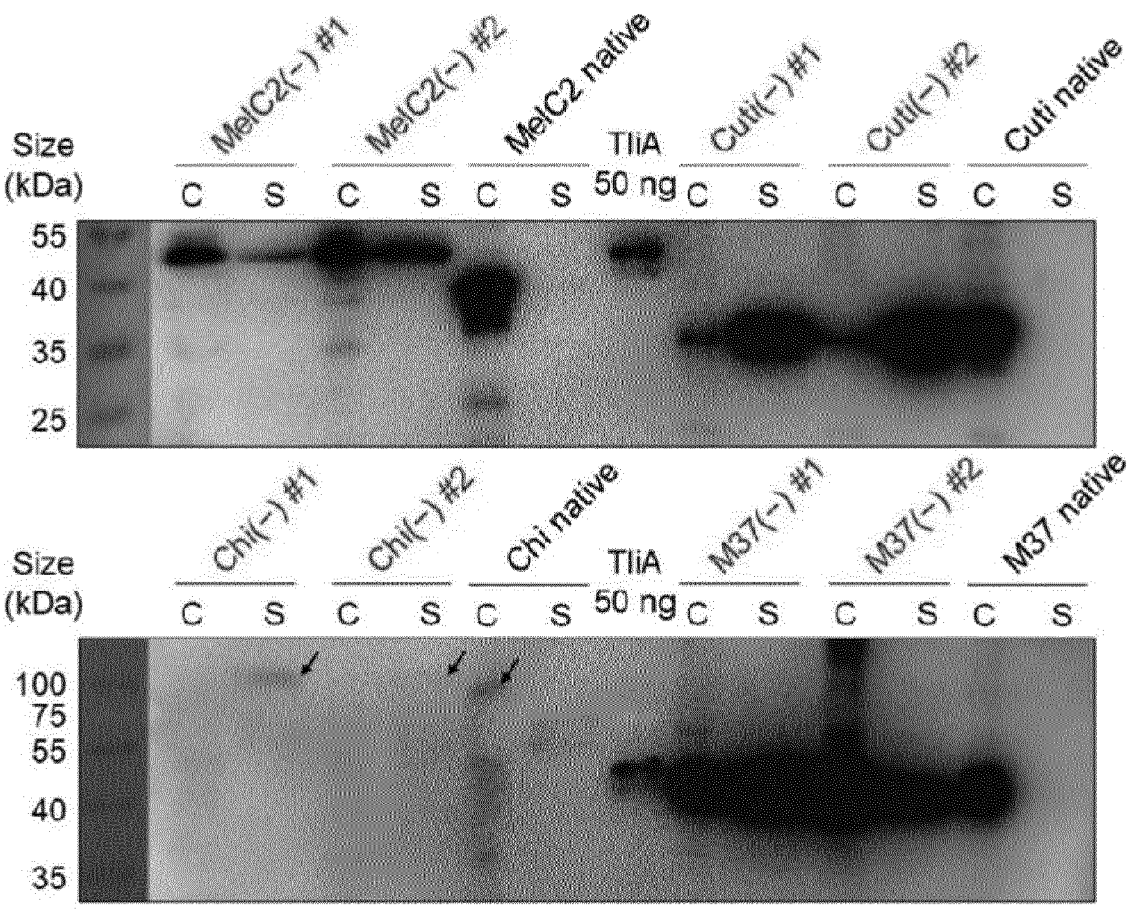
Size
(kDa)
MelC2(−) #1
MelC2(−) #2
MelC2 native
TliA
50 ng
Cuti(−) #1
Cuti(−) #2
Cuti native
C
S
55
40
35
25
Chi(−) #1
Chi(−) #2
Chi native
M37(−) #1
M37(−) #2
M37 native
100
75
55
40
35

【Fig. 19】

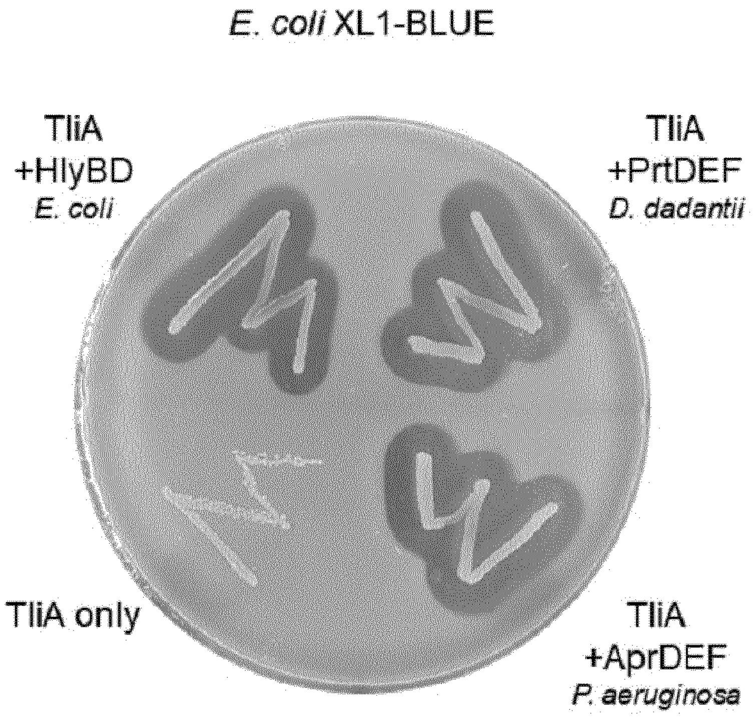
E. coli XL1-BLUE
TliA
+HlyBD
E. coli
TliA
+PrtDEF
D. dadantii
TliA only
TliA
+AprDEF
P. aeruginosa

【Fig. 20】

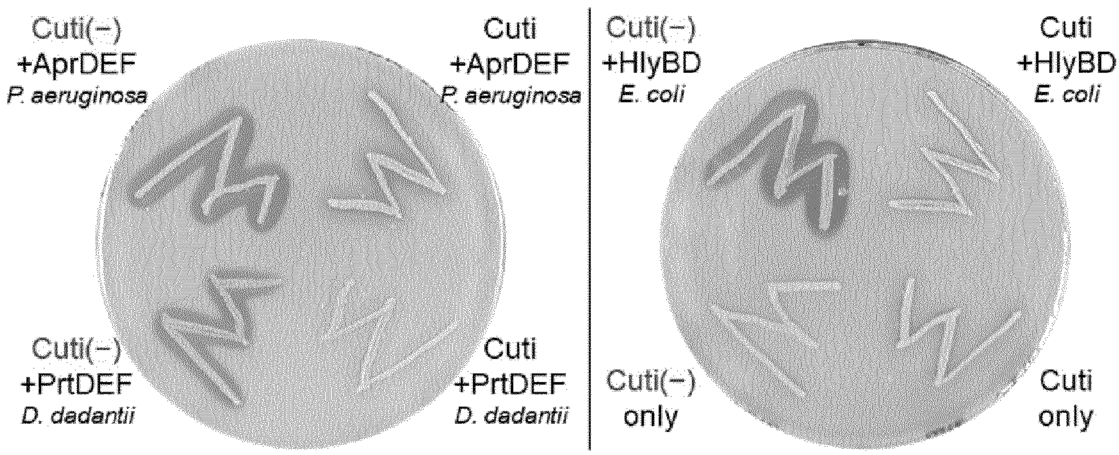
Cuti(−)
+AprDEF
P. aeruginosa
Cuti
+AprDEF
P. aeruginosa
Cuti(−)
+PrtDEF
D. dadantii
Cuti
+PrtDEF
D. dadantii
Cuti(−)
+HlyBD
E. coli
Cuti
+HlyBD
E. coli
Cuti(−)
only
Cuti
only

【Fig. 21】

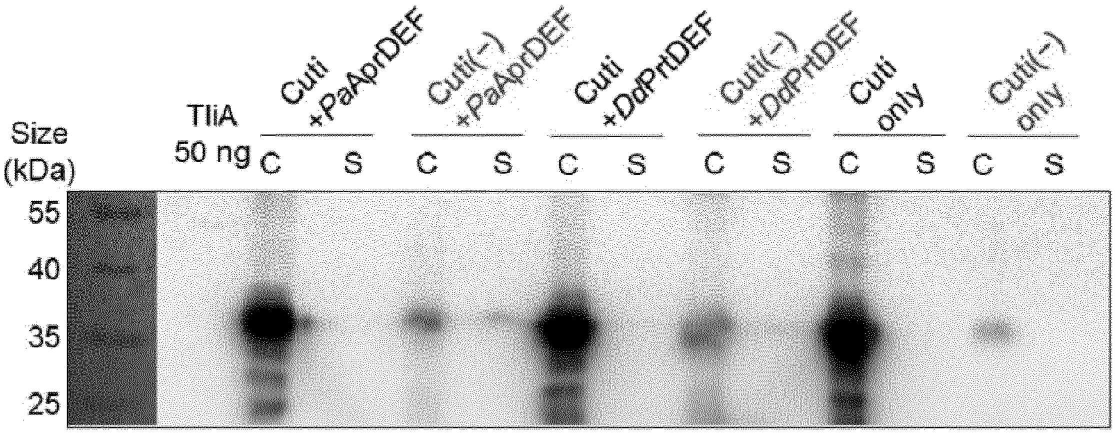
Size
(kDa)
TliA
50 ng
Cuti
+PaAprDEF
Cuti(-)
+PaAprDEF
Cuti
+DdPrtDEF
Cuti(-)
+DdPrtDEF
Cuti
only
Cuti(-)
only
C
S
55
40
35
25

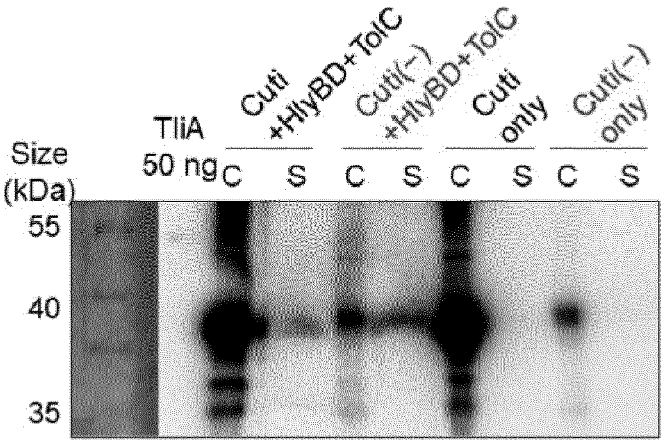
Size
(kDa)
TliA
50 ng
Cuti
+HlyBD+TolC
Cuti(-)
+HlyBD+TolC
Cuti
only
Cuti(-)
only
C
S
55
40
35

【Fig. 22】

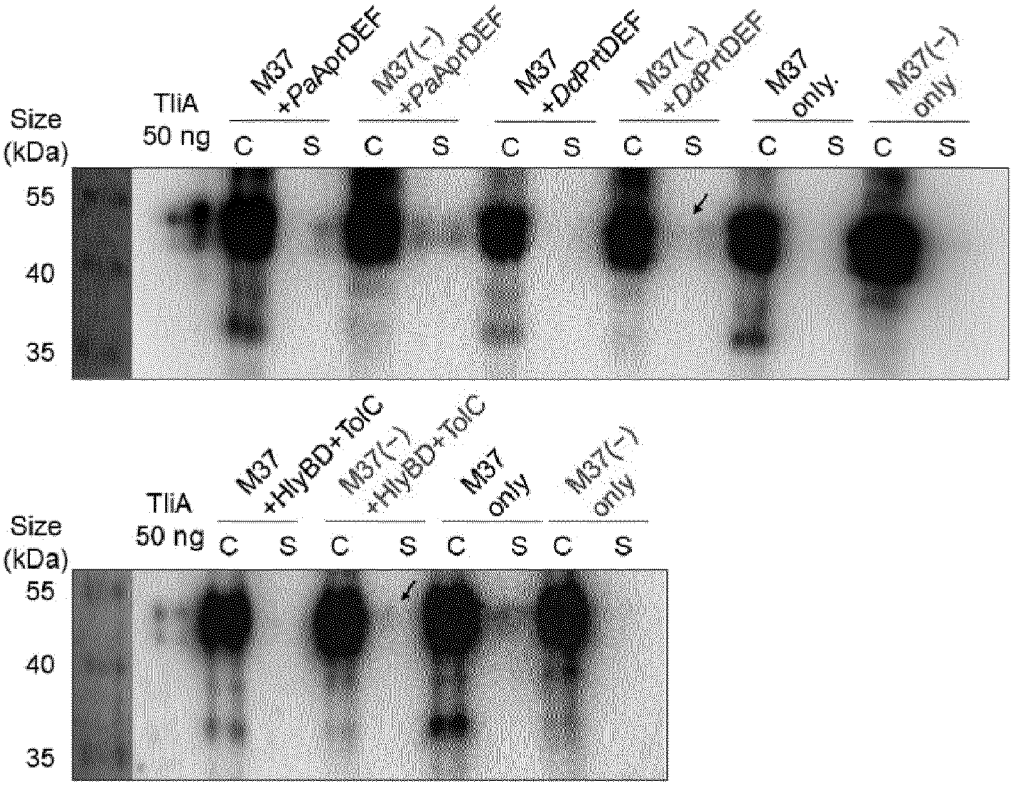
Size
(kDa)
TliA
50 ng
M37
+PaAprDEF
M37(−)
+PaAprDEF
M37
+DdPrtDEF
M37(−)
+DdPrtDEF
M37
only.
M37(−)
only
C
S
55
40
35
M37
+HlyBD+TolC
M37(−)
+HlyBD+TolC
M37
only
M37(−)
only

【Fig. 23】

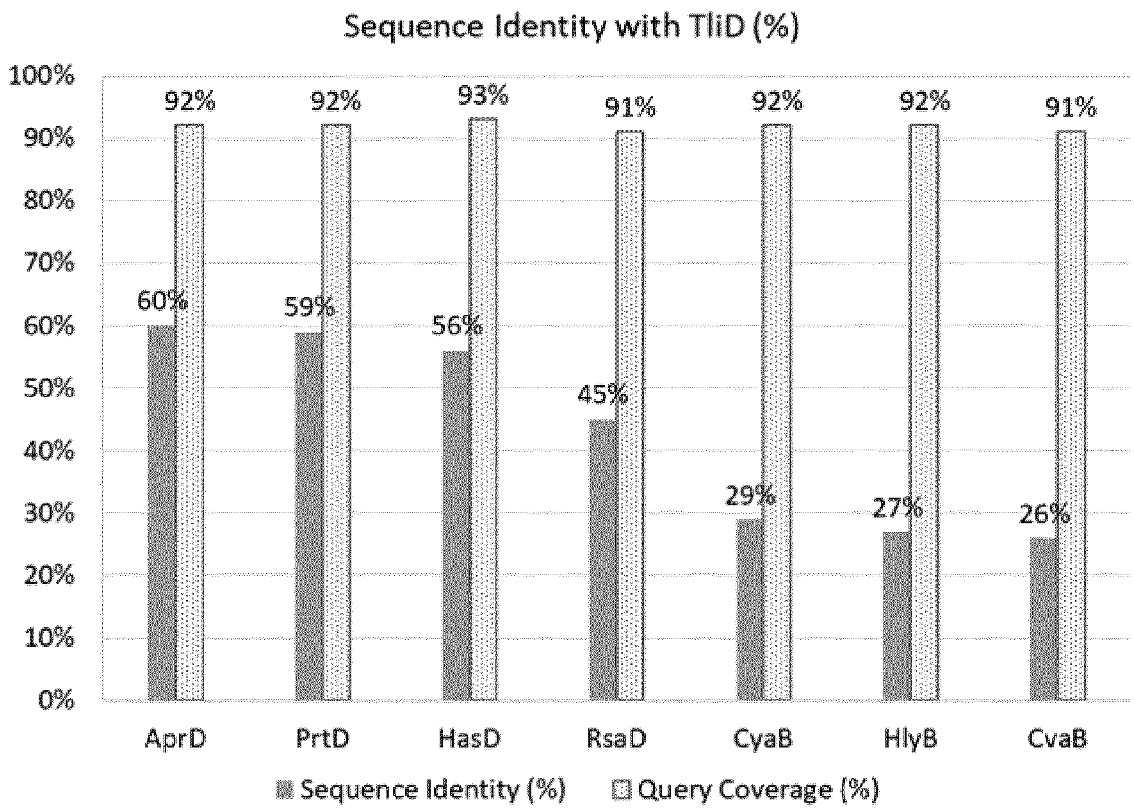
Sequence Identity with TliD (%)
100%
90%
80%
70%
60%
50%
40%
30%
20%
10%
0%
92%
92%
93%
91%
92%
92%
91%
60%
59%
56%
45%
29%
27%
26%
AprD
PrtD
HasD
RsaD
CyaB
HlyB
CvaB
Sequence Identity (%)
Query Coverage (%)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2007143574 A1
- KR 101677090
- KR 1020040041159


### Non-patent literature cited in the description


- **RYU, J. ; LEE, U. ; PARK, J. ; YOO, D. H. ; AHN, J. H.** A vector system for ABC transporter-mediated secretion and purification of recombinant proteins in Pseudomonas species. *Appl Environ Microbiol,* 2015, vol. 81, 1744-1753
- **LAWRENCE MS ; PHILLIPS KJ ; LIU DR.** Supercharging Proteins Can Impart Unusual Resilience. *Journal of the American Chemical Society,* 2007, vol. 129, 10110-10112
- **SON, M. ; MOON, Y. ; OH, M. J. ; HAN, S. B. ; PARK, K. H. ; KIM, J. G. ; AHN, J. H.** Lipase and protease double-deletion mutant of Pseudomonas fluorescens suitable for extracellular protein production. *Appl Environ Microbiol,* 2012, vol. 78, 8454-8462
- Escherichia coli, Plasmids, and Bacteriphages. **AUSUBEL, M. F.** Current Protocols in Molecular Biology. John Wiley & Sons, Inc, 2014
- **GALVEZ, A. F. ; CHEN, N. ; MACASIEB, J. ; DE LUMEN, B. O.** Chemopreventive Property of a Soybean Peptide (Lunasin) That Binds to Deacetylated Histones and Inhibits Acetylation. *Cancer Research,* 2001, vol. 61, 7473-7478
- **PETERSEN, T. N. ; BRUNAK, S. ; HEIJNE, G. ; NIELSEN, H.** SignalP 4.0: discriminating signal peptides from transmembrane regions. *Nature methods,* 2011, vol. 8, 785-786
- **WILKINS, M. R. ; GASTEIGER, E. ; BAIROCH, A. ; SANCHEZ, J. C. ; WILLIAMS, K. L. ; APPEL, R. D. ; HOCHSTRASSER, D. F.** Protein identification and analysis tools in the ExPASy server. *Methods Mol Biol,* 1999, vol. 112, 531-552
- **ARNOLD, K. ; BORDOLI, L. ; KOPP, J. ; SCHWEDE, T.** The SWISS-MODEL workspace: a web-based environment for protein structure homology modelling. *Bioinformatics,* 2006, vol. 22, 195-201
- **MORGAN, J. L. W. ; ACHESON, J. F. ; ZIMMER, J.** Structure of a Type-1 Secretion System ABC Transporter. *Structure,* 2017, vol. 25, 522-529
- **CSERZO, M. ; EISENHABER, F. ; EISENHABER, B. ; SIMON, I.** On filtering false positive transmembrane protein predictions. *Protein Engineering, Design and Selection,* 2002, vol. 15, 745-752
- **ASHKENAZY, H. ; ABADI, S. ; MARTZ, E. ; CHAY, O. ; MAYROSE, I. ; PUPKO, T. ; BEN-TAL, N.** ConSurf 2016: an improved methodology to estimate and visualize evolutionary conservation in macromolecules. *Nucleic Acids Research,* 2016, vol. 44, W344-W350
- **YANG, K. S. ; SUNG, B. H. ; PARK, M. K. ; LEE, J. H. ; LIM, K. J. ; PARK, S. C. ; KIM, S. J. ; KIM, H. K. ; SOHN, J.-H. ; KIM, H. M.** RECOMBINANT LIPASE ENGINEERED WITH SYNTHESIZED AMPHIPATHIC AND COILED-COIL PEPTIDES. *ACS CATALYSIS,* 2015, vol. 5, 5016-5025
- **KIM, D. ; JEON, C. ; KIM, J.-H. ; KIM, M.-S. ; YOON, C.-H. ; CHOI, I.-S. ; KIM, S.-H. ; BAE, Y.-S.** Cytoplasmic transduction peptide (CTP): New approach for the delivery of biomolecules into cytoplasm in vitro and in vivo. *Experimental Cell Research,* 2006, vol. 312, 1277-1288
- **LAWRENCE, M. S. ; PHILLIPS, K. J. ; LIU, D. R.** Supercharging proteins can impart unusual resilience. *Journal of the American Chemical Society,* 2007, vol. 129, 10110-10112
- **MATHEWS, C. K.** Biochemistry. 2013
- **DELEPELAIRE P ; WANDERSMAN C.** Protein secretion in gram-negative bacteria. The extracellular metalloprotease B from Erwinia chrysanthemi contains a C-terminal secretion signal analogous to that of Escherichia coli alpha-hemolysin. *J Biol Chem.,* 1990, vol. 265, 17118-17125
- **DUONG F ; SOSCIA C ; LAZDUNSKI A ; MURGIER M.** The Pseudomonas fluorescens lipase has a C-terminal secretion signal and is secreted by a three-component bacterial ABC-exporter system. *Mol Microbiol.,* 1994, vol. 11, 1117-1126